(19) **Europäisches Patentamt / European Patent Office / Office européen des brevets**

(11) **EP 4 775 687 A2**

(12) **EUROPEAN PATENT APPLICATION**

(43) Date of publication:
15.07.2026 Bulletin 2026/29

(21) Application number: 26170574.3

(22) Date of filing: 18.11.2021

(51) International Patent Classification (IPC):
*C12Q 1/6855* (2018.01)

(52) Cooperative Patent Classification (CPC):
(C-Sets available)
**C12Q 1/6806; C12Q 1/6855; C12Q 1/6869** (Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 19.11.2020 IL 27885620

(62) Document number(s) of the earlier application(s) in
accordance with Art. 76 EPC:
**21894201.9 / 4 247 973**

(71) Applicant: **Nucleix Ltd.**
7670203 Rehovot (IL)

(72) Inventors:
- **FRUMKIN, Danny**
  7630501 Rehovot (IL)
- **WASSERSTROM, Adam**
  7408516 Ness Ziona (IL)

- **AXELRAD, Nimrod**
  7630234 Rehovot (IL)
- **KNIRSH, Revital**
  4854145 Rosh HaAyin (IL)

(74) Representative: **Carpmaels & Ransford LLP**
**One Southampton Row**
**London WC1B 5HA (GB)**

Remarks:
•The complete document including Reference
Table(s) and the Sequence Listing(s) can be
downloaded from the EPO website
•This application was filed on 02-04-2026 as a
divisional application to the application mentioned
under INID code 62.
•Claims filed after the date of filing of the application /
after the date of receipt of the divisional application
(Rule 68(4) EPC).

(54) **DETECTING METHYLATION CHANGES IN DNA SAMPLES USING RESTRICTION ENZYMES AND HIGH THROUGHPUT SEQUENCING**

(57) Methods and systems for genetic and epigenetic profiling of DNA samples and detecting genetic and epigenetic changes in DNA samples are provided, which involve digestion of DNA with methylation-sensitive restriction enzymes, followed by high-throughput sequencing and analysis of sequence reads. Advantageously, the methods and systems of the present invention are sensitive yet accurate, and enable working with very low amounts of DNA and receive vast amount of information, including methylation data, mutation data and more, based on sequencing data from a single run.

**EP 4 775 687 A2**

**(Cont. next page)**

(52) Cooperative Patent Classification (CPC): (Cont.)

C-Sets
**C12Q 1/6806, C12Q 2521/331, C12Q 2525/191;
C12Q 1/6855, C12Q 2521/331, C12Q 2525/155,
C12Q 2525/191;
C12Q 1/6869, C12Q 2535/122**

**Description**

**FIELD OF THE INVENTION**

The present invention relates to methods and systems for profiling genetic and epigenetic characteristics of DNA samples, particularly cell-free DNA samples obtained from biological fluids such as plasma and urine. The methods and systems of the present invention involve digestion of DNA with methylation-sensitive or methylation-dependent restriction enzymes, preparation of a sequencing library, high throughput sequencing (e.g., next generation sequencing) and analysis of sequence reads. Advantageously, the methods and systems of the present invention are sensitive yet accurate, and enable working with very low amounts of DNA and receive vast amount of information, including methylation data, mutation data and more, based on sequencing data from a single run. The methods and systems of the present invention are useful for both discovery, e.g., of new methylation markers, and diagnostic applications at the clinics.

**BACKGROUND OF THE INVENTION**

Genetic and epigenetic changes are known to occur in many types of cancer, including mutations, DNA methylation changes (e.g., hypomethylation of isolated CpGs and hypermethylation occurring mostly at CpG islands), copy number variation and more. For example, hypermethylation of CpG islands in the promotor regions of tumor suppressor genes, leading to gene silencing, has been studied extensively and demonstrated in many different types of cancer.

Tumors release DNA fragments, or "cell-free DNA", into body fluids and consequently genetic and epigenetic changes of tumor derived DNA molecules can be detected in "liquid biopsies" obtained from body fluids such as blood plasma and urine. In contrast to traditional biopsies, liquid biopsies are non-invasive and may better represent the full genetic spectrum of tumor sub-clones. Consequently, detection of genetic and epigenetic changes associated with cancer in liquid biopsies holds great promise for early detection, prognosis, and therapeutic surveillance. However, in order to detect tumor derived DNA in liquid biopsies, ultra-sensitive biochemical methods are required, as the concentration of cell-free DNA in biological fluids may be low, and furthermore because the tumor DNA can be present in extremely low quantities in relation to the large background of normal DNA.

Several techniques have been developed for detection of methylated DNA molecules in liquid biopsies based on sodium bisulfite treatment of DNA to convert unmethylated cytosine to uracil, followed by quantitative PCR or sequencing of the converted DNA to detect methylation changes. Converted bases are identified (after PCR) as thymine in the sequencing data, and read counts can be used to determine percentage (%) of methylated cytosines. Bisulfite conversion sequencing can be done with targeted methods or with whole-genome bisulfite sequencing. The advances in high-throughput sequencing, such as next-generation sequencing (NGS), allow both genome-wide analysis and targeted approaches for identifying and analyzing methylation patterns at a single nucleotide level.

Despite its popularity, conversion of DNA by sodium bisulfite is a cumbersome assay, with disadvantages including degradation of the template DNA, non-specific or incomplete conversion that introduces noise to the assay, and reduction in the complexity of the genome from a 4-base genome to roughly a 3-base genome, which causes decreased specificity of PCR, increased biases in DNA amplification, and increase in the level of noise in sequencing of the DNA. In addition, since bisulfite treatment changes the sequence of the DNA, mutation analysis is hampered, because transition events are obscured on one of the DNA strands by the change of sequence.

Ball et al. (2009) Nat Biotechnol., 27(4): 361-368, report two techniques for cytosine methylation profiling utilizing next generation sequencing technology: bisulfite padlock probes (BSPPs) and methyl sensitive cut counting (MSCC).

Brunner et al. (2009) Genome Res., 19(6): 1044-1056, report Methyl-seq, a method that assays DNA methylation at more than 90,000 regions throughout the genome. Methyl-seq combines DNA digestion by a methyl-sensitive enzyme with next-generation (next-gen) DNA sequencing technology.

Jelinek et al. (2012) Epigenetics, 7:12, 1368-1378, report a method entitled Digital Restriction Enzyme Analysis of Methylation (DREAM), which is based on next generation sequencing analysis of methylation-specific signatures created by sequential digestion of genomic DNA with a pair of neoschizomeric restriction enzymes that recognize the same sequence: Sma, a methylation-sensitive enzyme, and XmaI, a methylation-insensitive enzyme.

Marsh and Pasqualone (2014) Front Physiol, 5:173, report the characterization of patterns of methyl-cytosine composition in the marine polychaete *Spiophanes tcherniai* from McMurdo Sound, Antarctica. Methylation patterns were characterized using DNA digestion by a methylation sensitive restriction endonuclease followed by next-generation sequencing.

Marsh et al. (2016) Front Genet., 7:191, report a quantification methodology for computationally reconstructing site-specific CpG methylation status from next generation sequencing (NGS) data using methyl-sensitive restriction endonucleases (MSRE).

Viswanathan et al. (2019) Nucleic Acids Research, 47(19): e122, report a single-tube enzymatic method, DNA Analysis by Restriction Enzymes (DARE), that enables quantitative analysis of both unmethylated and methylated DNA in the same sample. Information of both methylation status is captured by differential adapter tagging of DNA fragments that are sequentially digested by a pair of methylation sensitive and insensitive restriction enzymes.

Pereira et al. (2020) PLoS ONE, 15(6): e0233800, report a technique named Methyl Sensitive DArT-seq (MS-DArT-seq), which is based on the combination of double digestion of genomes, followed by special adapter ligation and next generation sequencing. Two libraries are constructed in parallel using restriction enzymes that target CCGG sites and show contrasting methylation sensitivity (*Msp*I, methylation insensitive, and *Hpa*II which does not cleave if the internal cytosine is 5'-methylated).

Tanaka et al. (2020) Analytical Biochemistry, 609: 113977, report an approach combining methylation-sensitive restriction enzyme (MSRE) and next-generation sequencing (NGS) to identify differentially methylated regions between chorionic villi (CV) and maternal blood cells (MBC).

US Patent 10,392,666 discloses determination of a methylation pattern (methylome) of DNA, and more particularly analysis of a biological sample (e.g., plasma) that includes a mixture of DNA from different genomes (e.g., from fetus and mother, or from tumor and normal cells) to determine the methylation pattern (methylome) of the minority genome.

WO 2016/061624 discloses methods for identifying sites and regions within a gene or genome that are amenable to analysis of methylation. The methods allow the efficient identification on a genome-wide scale of target restriction sites and fragments that provide targets for subsequent analysis.

WO 2018/195211 discloses compositions, kits, and methods for constructing libraries for simultaneous detection of genomic variants and DNA methylation status on limited DNA inputs, such as circulating polynucleotide fragments in the body of a subject, including circulating tumor DNA.

WO 2011/070441, WO 2017/006317, WO 2019/142193 and WO 2020/188561, assigned to the Applicant of the present invention, disclose methods for detecting methylation changes in DNA samples.

It would be highly beneficial to have methods and systems that can produce various types of genetic and epigenetic data from cell-free DNA obtained from a single sampling from a subject and using sequencing data from a single run.

## SUMMARY OF THE INVENTION

The present invention provides methods and systems for profiling genetic and epigenetic characteristics of DNA samples, particularly cell-free DNA samples obtained from biological fluids such as plasma and urine. The methods and systems of the present invention involve digestion with at least one methylation-sensitive restriction enzyme, preferably a plurality of methylation-sensitive restriction enzymes applied simultaneously, preparation of a sequencing library using library preparation methods that preserve the sequence information at the ends of DNA molecules in the sample, high throughput sequencing and analysis of sequence reads.

The present invention provides a more simple and accurate assay compared to hitherto described methods, yielding high-quality sequencing data compared to bisulfite sequencing enabling sensitive detection of cancer-associated changes. Vast amount of information from a single run and based on the same sequencing data can be obtained, including methylation data, mutation data and more, thus avoiding the need of parallel assays to obtain comprehensive genetic and epigenetic information. Importantly, it was surprisingly found that high-quality sequencing data can be obtained even from very low amounts of DNA, without a need for amplification prior to library preparation. As exemplified hereinbelow, the methods disclosed herein are able to detect genetic and epigenetic changes of early-stage cancer, when the amounts of tumor-derived DNA in the plasma are very low, based on the amount of cell-free DNA that can be achieved from a single standard blood test tube.

The methods and systems of the present invention do not involve or require bisulfite conversion. The methods and systems of the present invention do not require changing the sequence of the DNA and enable co-analysis of, e.g., methylation, mutation, copy number and nucleosome positioning, based on the same sequencing data.

As exemplified hereinbelow, a comparison of sequencing data obtained for cell-free DNA samples subjected to methylation-sensitive enzymatic digestion followed by high-throughput sequencing to sequencing data obtained following bisulfite conversion and high-throughput sequencing showed significantly better sequencing metrics (number of reads, mapping rate, etc.), copy number integrity and nucleosome positioning integrity for enzyme-treated DNA compared to bisulfite-treated DNA. Analysis of pooled cell-free DNA samples compared to individual samples showed that there is loss of information when low amounts of DNA are used. However, while the quality of the sequencing data of the enzyme-treated DNA samples remained high and enabled reliable analysis, bisulfite sequencing showed significantly reduced number of reads and mapping rate, and practically lost all copy number and nucleosome positioning information. In addition, sequencing noise was high for bisulfite-treated DNA samples, such that mutations were indistinguishable from the sequencing noise. With respect to methylation detection, methylation analysis in enzyme-treated DNA samples detected significantly more methylation changes in the plasma compared to bisulfite-treated DNA samples.

As further exemplified hereinbelow, while bisulfite-treated DNA samples resulted in broader CG coverage at the lower end of sequencing depths compared to enzyme-treated samples, a continuous and sharp decrease was seen in the number of CGs that were covered in bisulfite-treated DNA samples as the depth increased. In contrast, enzyme-treated samples showed substantially constant coverage even at depths over 250-300. At high depths, methylation-sensitive digestion provided significantly better CG coverage compared to bisulfite. Methylation-sensitive digestion provided coverage of millions of CGs at very high depths, thus enabling the detection of rare methylation signals, for example, methylated DNA molecules from a tumor in the plasma at an early stage of the tumor, which may be present in the plasma at very low amounts - 1% or even less of the total cell-free DNA. The data showed that at depths required for identification of rare signals, bisulfite does not provide sufficient coverage, and such rare signals are likely to be missed when using bisulfite sequencing on low amounts of DNA.

The present invention further discloses an improved method for determining methylation values for genomic loci of interest. Methylation analysis according to the present invention is carried out for restriction loci, namely, restriction sites of the restriction enzyme(s) used in the assay. Methylation analysis as disclosed herein is based on analyzing alignments covering a predefined genomic region of at least 50 bps in length, preferably at least 100 bps in length, that contains a restriction locus of interest, and determining a read count of sequence reads covering the predefined genomic region. Such alignments represent DNA molecules of at least 50 bps in length (preferably at least 100 bps in length), in which the analyzed restriction locus, as well as any additional restriction loci within the DNA molecule, were all methylated in the DNA sample and therefore the DNA molecules remained intact following digestion with the enzymes used in the assay. Analyzing alignments which are at least 50 or at least 100 bps in length and containing a plurality of restriction loci which were all methylated in the DNA sample increases the specificity of the cancer-related hypermethylation signal and enables an improved, more accurate detection of differences between normal and cancerous samples. In addition, the analysis of such alignments is advantageous for evaluating nucleosome positioning in cell-free DNA in addition to methylation because the copy numbers of such alignments reflect nucleosomal boundaries, wherein a high copy number is typical of the middle of the nucleosome, and a low copy number is typical of the boundaries between nucleosomes.

The present invention further discloses a method for direct calculation of both methylated and unmethylated levels of DNA based on sequencing data generated following methylation-sensitive/-dependent restriction of a DNA sample. Advantageously, the methods and systems of the present invention allow independently determining the methylated and unmethylated levels of DNA in a single assay and based on the same sequencing data, thus providing an improved identification of methylation changes. More particularly, the methods and systems disclosed herein comprise according to some embodiments digestion of a DNA sample with at least one methylation-sensitive restriction endonuclease, followed by high-throughput sequencing producing a plurality of sequence reads. Sequence reads may be aligned against a reference genome and restriction loci, namely, restriction sites within the genome, are selected and analyzed. The level of methylated DNA at the selected restriction loci is determined based on the read count of each restriction locus, which represents the number of DNA molecules in the sample in which the restriction locus was methylated and therefore remained intact. The level of unmethylated DNA at the selected restriction loci is determined by a unique analysis of the ends of sequence reads, by determining the number of reads starting or ending at a nucleotide within each restriction locus. This number of reads represents the number of DNA molecules in the sample in which the restriction locus was unmethylated and therefore cut by the restriction endonuclease. Such direct analysis of unmethylated DNA molecules is advantageous over indirect assessment based on the level of methylated DNA, as carried out by existing methods. The direct determination of unmethylation in addition to methylation using the same sequencing data provides complementary methylation information of genomic regions and thus improved methylation profiling, a more accurate and valid assessment of potential DNA methylation markers, and better detection of methylation differences between samples. It also provides an increased sensitivity of methylation analysis, particularly beneficial for genomic regions with extremely high or extremely low methylation levels.

According to one aspect, the present invention provides a method for profiling genetic and epigenetic characteristics of a cell-free DNA (cfDNA) sample from a subject, the method comprising:

(a) subjecting the cell-free DNA sample to digestion with at least one methylation-sensitive restriction endonuclease, to obtain restriction endonuclease-treated DNA in which methylated restriction sites are intact and unmethylated restriction sites are cut;

(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules;

(c) sequencing the sequencing library by a high-throughput sequencing method to provide sequencing data; and

(d) determining from the sequencing data a methylation value for at least one restriction locus and optionally at least one additional genetic or epigenetic characteristic of the cell-free DNA sample selected from DNA mutation, copy number variation and nucleosome positioning,

wherein an amount of cell-free DNA comprising 3000 haploid equivalents is sufficient for the method, wherein the cell-free DNA sample is not subjected to amplification prior to library preparation, and wherein determining the methylation value and the at least one additional genetic or epigenetic characteristic of the cell-free DNA sample is carried out based on the same sequencing data.

According to another aspect, the present invention provides a method for processing a cell-free DNA sample to obtain sequencing data for genetic and epigenetic analysis, the method comprising:

(a) subjecting the cell-free DNA sample to digestion with at least one methylation-sensitive restriction endonuclease, to obtain restriction endonuclease-treated DNA in which methylated restriction sites are intact and unmethylated restriction sites are cut;
(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules; and
(c) sequencing the sequencing library by a high-throughput sequencing method to obtain sequencing data,

wherein an amount of cell-free DNA comprising 3000 haploid equivalents is sufficient to achieve at least one of: unique mapping rate of at least 85%, a copy number integrity characterized by Pearson correlation of at least 0.65 compared to undigested sample and nucleosome positioning integrity characterized by Pearson correlation of at least 0.55 compared to undigested sample,
and wherein genetic and epigenetic analysis is performed based on the same sequencing data.

In some embodiments, an amount of cell-free DNA comprising 6,000 haploid equivalents is sufficient for the methods disclosed herein.

In some embodiments, the cell-free DNA is plasma cell-free DNA, and the amount of the cell-free DNA is an amount obtained from 9-10 ml of blood.

In some embodiments, the amount of cell-free DNA is between 10-200 ng. In additional embodiments, the amount of cell-free DNA is between 20-100 ng.

In some embodiments, the at least one methylation-sensitive restriction endonuclease produces non-blunt ends, and the method further comprises subjecting the restriction endonuclease-treated DNA to end repair prior to the ligation of sequencing adapters, to obtain DNA molecules with blunt ends.

In some embodiments, the high-throughput sequencing is whole genome high-throughput sequencing.

In some embodiments, the high-throughput sequencing is target-specific high-throughput sequencing.

In some embodiments, determining a methylation value for at least one restriction locus comprises:

(i) selecting at least one restriction locus and determining the number of sequence reads covering a predefined genomic region of at least 50 bps in length that contains said restriction locus; and
(ii) calculating a methylation value for the at least one restriction locus based on the read count determined in step (i) and a reference read count.

In some embodiments, step (i) comprises determining the number of sequence reads covering a predefined genomic region of at least 100 bps in length that contains said restriction locus.

In some embodiments, the at least one restriction locus is a plurality of restriction loci.

In some embodiments, the at least one methylation-sensitive restriction endonuclease is a plurality of methylation-sensitive restriction endonucleases, and the digestion with the plurality of methylation-sensitive restriction endonucleases is a simultaneous digestion.

In some embodiments, the plurality of methylation-sensitive restriction endonucleases comprises HinP1I. In additional embodiments, the plurality of methylation-sensitive restriction endonucleases comprises AciI. In additional embodiments, the digestion is carried out using HinP1I and AciI. In some embodiments, the digestion is carried out using HinP1I and AciI at a ratio between 1:1 to 5:1 (enzyme units) (Hinp:AciI).

In some embodiments, the step of subjecting the cell-free DNA sample to digestion with at least one methylation-sensitive restriction endonuclease further comprises determining digestion efficacy, and proceeding to preparing a sequencing library if the digestion efficacy is above a predefined threshold.

According to another aspect, the present invention provides a method for detecting cancer-related genetic and epigenetic changes in a cell-free DNA sample (cfDNA) from a subject, the method comprising: profiling methylation and optionally at least one additional genetic and epigenetic characteristics of the cfDNA sample as disclosed herein, to obtain a genetic and epigenetic profile of the cfDNA sample; and comparing the genetic and epigenetic profile of the cfDNA sample to one or more reference genetic and epigenetic profile selected from a cancer profile and a non-cancer profile, to detect cancer-associated genetic and epigenetic changes in the cfDNA sample.

In some embodiments, the cell-free DNA sample is from a subject suspected of having cancer or at risk of having cancer, and the method further comprises administering to the subject active cancer surveillance and follow-up testing when cancer-associated changes are detected, the cancer surveillance and follow-up testing comprises one or more of blood tests, urine tests, cytology, imaging, endoscopy and biopsy.

According to a further aspect, the present invention provides a method for assessing the presence or absence of cancer in a subject, the method comprising:

(a) subjecting a cell-free DNA (cfDNA) sample of the subject to digestion with at least one methylation-sensitive restriction endonuclease, to obtain restriction endonuclease-treated DNA in which methylated restriction sites are intact and unmethylated restriction sites are cut;
(b) sequencing the restriction endonuclease-treated DNA by a high-throughput sequencing method;
(c) selecting at least one multiomic genomic region, comprising a tumor hypermethylated restriction locus and a tumor mutation locus within 150 bps of each other; and
(d) determining the likelihood that the subject has cancer based on analysis of sequence reads covering the at least one multiomic region.

In some embodiments, the at least one multiomic region comprises a tumor hypermethylated restriction locus and a tumor mutation locus within 100 bps of each other.

In some embodiments, analysis of sequence reads covering the at least one multiomic region comprises:

-    determining for each multiomic region at least one of:

(i) the number of methylated-mutated sequence reads covering said multiomic region, which include all nucleotides of the restriction locus and present a mutated genotype at the mutation locus;
(ii) the number of methylated-wild type sequence reads covering said multiomic region, which include all nucleotides of the restriction locus and present a wild type genotype at the mutation locus;
(iii) the number of unmethylated-mutated sequence reads covering said multiomic region, which start or end at a nucleotide within the restriction locus and present a mutated genotype at the mutation locus; and
(iv) the number of unmethylated-wild type sequence reads covering said multiomic region, which start or end at a nucleotide within the restriction locus and present a wild type genotype at the mutation locus;
and

-    comparing the number of reads determined in (i)-(iv) to reference values of cancer patients and/or healthy individuals in order to assess the likelihood that the subject has cancer.

According to a further aspect, the present invention provides a method for characterizing a cell-free DNA (cfDNA) sample of a subject suspected of having cancer or at risk of having cancer, the method comprising:

(a) subjecting the cell-free DNA sample to digestion with at least one methylation-sensitive restriction endonuclease, to obtain restriction endonuclease-treated DNA in which methylated sites are intact and unmethylated sites are cut;
(b) sequencing the restriction endonuclease-treated DNA by a high-throughput sequencing method;
(c) selecting at least one multiomic genomic region, comprising a tumor hypermethylated restriction locus and a tumor mutation locus within 150 bps of each other; and
(d) determining for each multiomic region at least one of:

(i) the number of methylated-mutated sequence reads covering said multiomic region, which include all nucleotides of the restriction locus and present a mutated genotype at the mutation locus;
(ii) the number of methylated-wild type sequence reads covering said multiomic region, which include all nucleotides of the restriction locus and present a wild type genotype at the mutation locus;
(iii) the number of unmethylated-mutated sequence reads covering said multiomic region, which start or end at a nucleotide within the restriction locus and present a mutated genotype at the mutation locus; and
(iv) the number of unmethylated-wild type sequence reads covering said multiomic region, which start or end at a nucleotide within the restriction locus and present a wild type genotype at the mutation locus,

thereby characterizing the cell-free DNA sample.

According to another aspect, the present invention provides a method for profiling methylation of a DNA sample from a subject, the method comprising:

(a) subjecting the DNA sample to digestion with at least one methylation-sensitive restriction endonuclease, to obtain restriction endonuclease-treated DNA in which methylated sites are intact and unmethylated sites are cut;

(b) preparing a sequencing library from the restriction endonuclease-treated DNA, wherein preparing the sequencing library comprises ligating sequencing adapters to DNA fragments in the restriction endonuclease-treated, wherein each adapter is capable of ligation to both the digested and undigested DNA molecules;

(c) sequencing the sequencing library by a high-throughput sequencing method to obtain sequence reads;

(d) selecting at least one restriction locus and determining the number of sequence reads covering a predefined genomic region of at least 50 bps in length that contains said restriction locus; and

(e) calculating a methylation value for the at least one restriction locus based on the read count determined in step (d) and a reference read count,

thereby profiling methylation of the cell-free DNA sample.

In some embodiments, the predefined region covering the restriction locus starts at least 25 bps upstream of the cut site within the restriction locus and ends at least 25 bps downstream of the cut site within the restriction locus.

In some embodiments, step (d) comprises determining the number of sequence reads covering a predefined genomic region of at least 100 bps in length that contains said restriction locus. In some embodiments, the predefined region covering the restriction locus starts at least 50 bps upstream of the cut site within the restriction locus and ends at least 50 bps downstream of the cut site within the restriction locus.

In some embodiments, the at least one restriction locus is located within a CG-island.

In some embodiments, the reference read count is a read count determined for the predefined genomic region of at least 50 bps in length that contains the restriction locus in an undigested control DNA sample, optionally corrected for sequencing depth differences.

In some embodiments, the reference read count is a read count determined using a reference region of at least 50 bps in length containing a reference locus that is not cut by the restriction endonuclease.

In some embodiments, the reference read count is an average read count determined using a plurality of reference regions of at least 50 bps in length containing reference loci that are not cut by the restriction endonuclease.

In some embodiments, calculating a methylation value comprises normalizing the read count determined in step (d) against a median read count of the DNA sample, to obtain a normalized read count, and calculating a ratio of the normalized read count to a normalized reference read count.

According to another aspect, the present invention provides a method for genetic and epigenetic profiling of a DNA sample, the method comprising determining a methylation value for at least one restriction locus as disclosed herein, and further determining from the sequencing data at least one additional genetic or epigenetic characteristic of the DNA sample selected from DNA mutation, copy number variation and nucleosome positioning.

In some embodiments, the DNA is cell-free DNA extracted from a biological fluid sample. In additional embodiments, the DNA is DNA extracted from a tumor sample.

According to a further aspect, the present invention provides a method for identifying genomic regions differentially methylated between a first and second source of DNA, the method comprising:

profiling methylation of at least one DNA sample from the first source as disclosed herein, to obtain a first DNA methylation profile;

profiling methylation of at least one DNA sample from the second source as disclosed herein, to obtain a second DNA methylation profile; and

comparing the first and second DNA methylation profiles to identify genomic regions differentially methylated between the first and second sources of DNA.

In some embodiments, the first source of DNA is a cancer DNA and the second source of DNA is a non-cancer DNA. In some embodiments, the first source of DNA is plasma cell-free DNA of a cancer patient and the second source of DNA is plasma cell-free DNA of one or more healthy individuals. In additional embodiments, the first and second sources of DNA are different stages of a cancer.

According to a further aspect, the present invention provides a method for profiling methylation of a DNA sample from a subject, the method comprising:

(a) providing a DNA sample from a subject;

(b) subjecting the DNA sample to digestion with at least one methylation-sensitive restriction endonuclease, thereby obtaining restriction endonuclease-treated DNA comprising restriction endonuclease-generated DNA fragments;

(c) performing high-throughput sequencing of the endonuclease-treated DNA to obtain sequence reads;

(d) determining from the sequence reads a read count of at least one restriction locus, the read count representing the number of DNA molecules in the DNA sample in which said at least one restriction locus was methylated and therefore remained intact;

(e) determining from the sequence reads a read count of sequence reads or ending at a nucleotide within the at least one restriction locus, the read count representing the number of DNA molecules in the DNA sample in which said at least one restriction locus was unmethylated and therefore cut by the restriction endonuclease;

(f) calculating a level of methylated DNA at the at least one restriction locus based on the read count of the at least one restriction locus determined in step (d) and a level of unmethylated DNA at the at least one restriction locus based on the read count of sequence reads starting or ending at a nucleotide within the at least one restriction locus determined in step (e),

thereby profiling methylation of the DNA sample.

In some embodiments, steps (c)-(e) comprise:

preparing a sequencing library from the restriction endonuclease-treated DNA using sequencing adapters ligated to a plurality of the restriction endonuclease-generated DNA fragments, and subjecting the sequencing library to high-throughput sequencing to obtain sequence reads;

mapping a plurality of the sequence reads against a reference genome to produce mapped sequence reads, and selecting at least one restriction locus within the reference genome;

determining from the mapped sequence reads a read count of the at least one restriction locus, the read count representing the number of DNA molecules in the DNA sample in which said at least one restriction locus was methylated and therefore remained intact; and

determining from the mapped sequence reads a read count of sequence reads starting or ending at a nucleotide within the at least one restriction locus, the read count representing the number of DNA molecules in the DNA sample in which said at least one restriction locus was unmethylated and therefore cut by the restriction endonuclease.

In some embodiments, the high-throughput sequencing is whole genome high-throughput sequencing. In other embodiments, the high-throughput sequencing is target-specific high-throughput sequencing.

In some embodiments, the reference genome is the complete human genome.

In some embodiments, the DNA is cell-free DNA extracted from a biological fluid sample. In some embodiments, the biological fluid sample is plasma, serum or urine. Each possibility of the biological sample is a separate embodiment of the present invention.

In some embodiments, the DNA is DNA extracted from a tumor sample.

In some embodiments, calculating a level of methylated DNA at the at least one restriction locus comprises calculating a ratio of the read count of the at least one restriction locus determined in step (d) to an expected read count of the at least one restriction locus.

In some embodiments, calculating a level of unmethylated DNA at the at least one restriction locus comprises calculating a difference between the read count of sequence reads starting or ending at a nucleotide within the at least one restriction locus determined in step (e) and an expected read count of sequences starting or ending at a nucleotide within the at least one restriction locus, and subsequently dividing the difference by an expected read count of the at least one restriction locus.

In some embodiments, calculating a level of methylated DNA at the at least one restriction locus comprises:

determining a total fragment number by summing the read count of the at least one restriction locus determined in step (d) and the read count of sequence reads starting or ending at a nucleotide within the at least one restriction locus determined in step (e), and subsequently subtracting from the sum an expected read count of sequences starting or ending at a nucleotide within the restriction locus; and

dividing the read count of the at least one restriction locus determined in step (d) by the total fragment number.

In some embodiments, calculating a level of unmethylated DNA at the at least one restriction locus comprises:

determining a total fragment number as described herein;

calculating a difference between the read count of sequence reads starting or ending at a nucleotide within the at least one restriction locus determined in step (e) and an expected read count of sequences starting or ending at a nucleotide within the restriction locus; and

dividing the difference by the total fragment number.

In some embodiments, the expected read counts are read counts determined using a reference locus of the same length as the at least one restriction locus, that is not cut by the restriction endonuclease.

In some embodiments, the expected read counts are average read counts determined using a plurality of reference loci of the same length as the at least one restriction locus, that are not cut by the restriction endonuclease.

In some embodiments, the expected read counts are read counts determined for the at least one restriction locus in an undigested control DNA sample, optionally corrected for sequencing depth differences.

In some embodiments, the at least one restriction locus is a plurality of restriction loci.

In some embodiments, the at least one methylation-sensitive restriction endonuclease is a plurality of methylation-sensitive restriction endonucleases.

In some embodiments, the method for profiling methylation further comprises identifying the presence or absence of a disease in the subject based on the methylation profile of the DNA sample, by comparing the methylation profile of the DNA sample to one or more reference methylation profile.

In some embodiments, the method further comprises preparing a report in paper or electronic form based on the methylation profile and communicating the report to the subject and/or to a healthcare provider of the subject.

According to another aspect, the present invention provides a method for detecting methylation changes in a DNA sample, the method comprising: profiling methylation of the DNA sample as disclosed herein, to obtain a methylation profile of the DNA sample; and comparing the methylation profile of the DNA sample to one or more reference methylation profile to detect methylation changes in the DNA sample.

In some embodiments, the one or more reference methylation profile comprises a healthy DNA methylation profile. In additional embodiments, the one or more reference methylation profile comprises a disease DNA methylation profile. In some embodiments, the DNA sample is from a subject suspected of having the disease and/or a subject at risk of developing the disease, and detecting methylation changes comprises determining whether the DNA sample is a healthy or disease DNA sample. In some embodiments, the disease is a cancer.

According to a further aspect, the present invention provides a method for identifying genomic regions differentially methylated between a first and second source of DNA, the method comprising:

profiling methylation of at least one DNA sample from the first source according to the method disclosed herein, to obtain a first DNA methylation profile;

profiling methylation of at least one DNA sample from the second source according to the method disclosed herein, to obtain a second DNA methylation profile; and

comparing the first and second DNA methylation profiles to identify genomic regions differentially methylated between the first and second sources of DNA.

In some embodiments, the first source of DNA is a disease DNA and the second source of DNA is a non-disease DNA. In additional embodiments, the first and second sources of DNA are different stages of a disease. In some embodiments, the disease is a cancer.

According to a further aspect, the present invention provides a method for profiling genetic and epigenetic characteristics of a DNA sample, the method comprising:

profiling methylation of the DNA sample as disclosed herein; and

determining at least one additional genetic or epigenetic characteristic of the DNA sample, wherein the at least one additional genetic or epigenetic characteristic is selected from DNA mutation, copy number variation and nucleosome positioning,

wherein profiling the methylation and determining the at least one additional genetic or epigenetic characteristic are carried out using the same sequencing data,

thereby profiling genetic and epigenetic characteristics of the DNA sample.

These and further aspects and features of the present invention will become apparent from the detailed description, examples and claims which follow.

**BRIEF DESCRIPTION OF THE FIGURES**

**Figure 1A.** Copy number data of pooled plasma cell-free DNA samples subjected to methylation-sensitive digestion, bisulfite conversion or no treatment prior to sequencing. Data are presented as the number of hits (count) per genomic position.

**Figure 1B.** Correlation of hits between test (treated) and control (untreated) pooled plasma cell-free DNA samples.

**Figure 2A.** Nucleosome positioning data of pooled plasma cell-free DNA samples subjected to methylation-sensitive digestion, bisulfite conversion or no treatment prior to sequencing. Data are presented as "hits span 100" (= number of reads that start > 50bp upstream and end > 50 bp downstream of an analyzed genomic position).

**Figure 2B.** Correlation of "hits span 100" between test (treated) and control (untreated) pooled plasma cell-free DNA samples.

**Figure 3.** Copy number integrity of plasma cell-free DNA of patient BMD LNG165 (**3A**) and patient BMD LNG166 (**3B**) that were subjected to methylation-sensitive digestion or bisulfite conversion prior to sequencing.

**Figure 4.** Nucleosome positioning integrity of plasma cell-free DNA of patient BMD LNG165 (**4A**) and patient BMD LNG166 (**4B**) that was subjected to methylation-sensitive digestion or bisulfite conversion prior to sequencing. Data are presented as "hits span 100" (= number of reads that start > 50bp upstream and end > 50 bp downstream of an analyzed genomic position).

**Figure 5.** CG depths of plasma cell-free DNA of patient BMD LNG165 (**5A**) and patient BMD LNG166 (**5B**) that was subjected to methylation-sensitive digestion or bisulfite conversion prior to sequencing.

**Figure 6.** Detection of hypermethylated marker loci in plasma cell-free DNA of patient BMD LNG165 and patient BMD LNG166 using methylation-sensitive digestion or bisulfite conversion of the DNA.

**Figure 7.** Detection of tumor mutations in plasma cell-free DNA of patient BMD LNG165 compared to control (**7A**) and in plasma cell-free DNA of patient BMD LNG166 compared to control (**7B**) using methylation-sensitive digestion or bisulfite conversion of the **DNA.**

**Figure 8.** Sample preparation for genetic and epigenetic profiling. (**8A**) lung cancer samples; (**8B**) control samples.

**Figure 9.** Clinical data and methylation data of patients BMD LNG165 (**9A**) and BMD LNG166 (**9B**).

**Figure 10.** Methylation loci with a strong hypermethylation signal in the plasma of patient BMD LNG165 (**10A**) and BMD LNG166 (**10B**).

**Figure 11.** Mutation data of patients BMD LNG165 (**11A**) and BMD LNG166 (**11B**).

**Figure 12.** A multiomic region in patient BMD LNG165.

**Figure 13.** Types of multiomic alignments.

**Figure 14.** Illustration of the methylation-sensitive HinP1I site before and after digestion and end repair.

**Figure 15.** Illustration of DNA fragments obtained following digestion and end repair of DNA molecules spanning a HinP1I restriction site which are either methylated or unmethylated at the cut site.

**Figure 16.** Analysis of sequence reads according to embodiments of the present invention. (**16A**) read count of a restriction locus; (**16B**) read count of sequence reads starting at a nucleotide within the restriction locus; (**16C**) read count of sequence reads ending at a nucleotide within the restriction locus.

**Figure 17.** Analysis of sequence reads according to embodiments of the present invention of exemplary locus CG#1 (**17A**), exemplary locus CG#4 (**17B**) and exemplary locus CG#5 (**17C**).

**Figure 18.** Flowchart describing an exemplary method for profiling methylation of a DNA sample at lung cancer-associated genomic regions according to embodiments of the present invention.

**Figure 19.** Flowchart describing an additional exemplary method for profiling methylation of a DNA sample at lung cancer-associated genomic regions according to embodiments of the present invention.

**Figure 20.** Flowchart describing an exemplary method for determining whether a DNA sample is positive or negative for lung cancer according to embodiments of the present invention.

**Figure 21.** Flowchart describing an additional exemplary method for determining whether a DNA sample is positive or negative for lung cancer according to embodiments of the present invention.

## DETAILED DESCRIPTION OF THE INVENTION

The present invention relates to methods and systems for profiling genetic and epigenetic characteristics of DNA samples, particularly cell-free DNA samples, using digestion of DNA with methylation-sensitive/ methylation-dependent restriction enzymes followed by high throughput sequencing and analysis of sequence reads. Advantageously, the methods and systems of the present invention are sensitive yet accurate, and enable working with very low amounts of DNA and receive vast amount of information, including methylation data, mutation data and more, based on sequencing data from a single run.

Remarkably, even though very low amounts of DNA may be used, the quality of the sequencing data, and accordingly the genetic and epigenetic information that can be derived therefrom, is very high and enable sensitive and comprehensive identification of cancer-associated changes.

The methods disclosed herein require according to some embodiments preserving the sequence information at the 5' and/or 3' ends of DNA molecules, including natural ends (e.g., for nucleosome positioning evaluation of cell-free DNA) and ends generated following digestion with restriction enzymes as disclosed herein (e.g., for analysis of DNA molecules that were unmethylated at the DNA sample). Preserving the sequence information at the ends of DNA molecules, or "end-preserving", according to the present invention encompasses avoiding PCR to enrich genomic regions of interest and/or introduce sequencing adapters. In some particular embodiments, end-preserving according to the present invention is preserving sequence information at the ends of DNA molecules pertaining to the methylation status of the DNA molecules.

In some embodiments, library preparation according to the present invention is carried out in an end-preserving manner, indicating that the library preparation process does not include PCR to enrich genomic regions of interest and/or introduce sequencing adapters. According to these embodiments, library preparation comprises adding sequencing adapters via ligation (e.g., enzymatic ligation). If enrichment of certain genomic regions is desired, library preparation according to these embodiments comprises enriching the genomic regions of interest using capture agents.

The methods of the present invention do not require the use of restriction enzyme isoschizomers, where one of the enzymes recognizes both the methylated and unmethylated forms of the restriction site while the other recognizes only the unmethylated form, or require a combined use of methylation-sensitive and methylation-insensitive restriction enzymes.

Also, the methods of the present invention do not require or employ size selection of DNA fragments of a particular size range following digestion, or filtering of read counts with a particular size range following sequencing.

In some embodiments, the present invention provides an improved method for determining methylation values for genomic loci of interest. The improved method is based on determining a read count of sequence reads covering a predefined genomic region of at least 50 bps in length, preferably at least 100 bps in length, that contains a restriction locus of interest.

In some embodiments, the present invention relates to systems and methods for high resolution DNA methylation profiling. In some embodiments, the present invention provides the use of methylation-sensitive/methylation-dependent restriction enzymes and high-throughput sequencing in the analysis of DNA methylation. In some particular embodiments, the present invention provides the use of methylation-sensitive/methylation-dependent restriction enzymes and high-throughput sequencing for direct calculation of methylated and unmethylated DNA levels.

Methylation in the human genome occurs in the form of 5-methyl cytosine and is confined to cytosine residues that are part of the sequence CG, also denoted as CpG dinucleotides (cytosine residues that are part of other sequences are not methylated). Some CG dinucleotides in the human genome are methylated, and others are not. In addition, methylation is cell and tissue specific, such that a specific CG dinucleotide can be methylated in a certain cell and at the same time unmethylated in a different cell, or methylated in a certain tissue and at the same time unmethylated in different tissues. DNA methylation is an important regulator of gene transcription.

The methylation pattern of cancer DNA differs from that of normal DNA, wherein some loci are hypermethylated while others are hypomethylated. In some embodiments, the present invention provides methods and systems for sensitive detection of differentially methylated (e.g., hypermethylated) genomic loci associated with cancer.

In some embodiments, there is provided herein a method for profiling genetic and epigenetic characteristics of a cell-free DNA (cfDNA) sample from a subject, the method comprising:

(a) subjecting the cell-free DNA sample to digestion with at least one methylation-sensitive restriction endonuclease, to obtain restriction endonuclease-treated DNA in which methylated sites are intact and unmethylated sites are cut;
(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules;
(c) sequencing the sequencing library by a high-throughput sequencing method to provide sequencing data; and
(d) determining from the sequencing data a methylation value for at least one restriction locus and optionally at least one additional genetic or epigenetic characteristic of the cell-free DNA sample selected from DNA mutation, copy number variation and nucleosome positioning,

wherein an amount of cell-free DNA comprising no more than 3,000 haploid equivalents is sufficient for the method, wherein the cell-free DNA sample is not subjected to amplification prior to library preparation, and wherein determining the methylation value and the at least one additional genetic or epigenetic characteristic of the cell-free DNA sample is carried out based on the same sequencing data.

In some embodiments, there is provided herein a method for processing a cell-free DNA sample to obtain sequencing data for genetic and epigenetic analysis, the method comprising:

(a) subjecting the cell-free DNA sample to digestion with at least one methylation-sensitive restriction endonuclease, to obtain restriction endonuclease-treated DNA in which methylated sites are intact and unmethylated sites are cut;
(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules; and
(c) sequencing the sequencing library by a high-throughput sequencing method to obtain sequencing data,

wherein an amount of cell-free DNA comprising no more than 3,000 haploid equivalents is sufficient to achieve at least one of: unique mapping rate of at least 85%, a copy number integrity characterized by Pearson correlation of at least 0.65 compared to undigested sample and nucleosome positioning integrity characterized by Pearson correlation of at least 0.55 compared to undigested sample,

and wherein genetic and epigenetic analysis is performed based on the same sequencing data.

As used herein, 3.3 pg of DNA corresponds to 1 haploid equivalent.

In some embodiments, 10ng of DNA are sufficient for the methods disclosed herein. In additional embodiments, 20ng of DNA are sufficient for the methods disclosed herein. In additional embodiments, the methods disclosed herein are carried out using an initial amount of DNA ranging from 10-200ng, for example between 20-200ng, between 20-100ng, including each value within the ranges. Each possibility represents a separate embodiment.

In some embodiments, 3,000 haploid equivalents are sufficient for the methods disclosed herein. In additional embodiments, 6,000 haploid equivalents are sufficient for the methods disclosed herein. In additional embodiments, the methods disclosed herein are carried out using an initial amount of DNA comprising 3,000-60,000 haploid equivalents, for example between 6,000-60,000 haploid equivalents, between 6,000-30,000 haploid equivalents, including each value within the ranges. Each possibility represents a separate embodiment.

In some embodiments, an amount of cell-free DNA as disclosed herein is sufficient to achieve a unique mapping rate of at least 85%, at least 86%, at least 87%, at least 88%, at least 89%. Each possibility represents a separate embodiment.

In some embodiments, an amount of cell-free DNA as disclosed herein is sufficient to achieve a copy number integrity characterized by Pearson correlation of at least 0.6 compared to undigested sample, for example at least 0.65, at least 0.66, at least 0.67, at least 0.68, at least 0.69 compared to undigested sample. Each possibility represents a separate embodiment.

In some embodiments, an amount of cell-free DNA as disclosed herein is sufficient to achieve nucleosome positioning integrity characterized by Pearson correlation of at least 0.55 compared to undigested sample, for example at least 0.56, at least 0.57, at least 0.58, at least 0.59 compared to undigested sample. Each possibility represents a separate embodiment.

In some embodiments, there is provided herein a method for profiling methylation of a DNA sample from a subject, the method comprising:

(a) subjecting the DNA sample to digestion with at least one methylation-sensitive restriction endonuclease, to obtain restriction endonuclease-treated DNA in which methylated sites are intact and unmethylated sites are cut;
(b) preparing a sequencing library from the restriction endonuclease-treated DNA;
(c) sequencing the sequencing library by a high-throughput sequencing method to obtain sequence reads;
(d) ) selecting at least one restriction locus and determining the number of sequence reads covering a predefined genomic region of at least 50 bps in length that contains said restriction locus; and
(e) determining a methylation value for the at least one restriction locus based on the read count determined in step (d) and a reference read count,

thereby profiling methylation of the cell-free DNA sample.

In some embodiments, profiling methylation of a DNA sample comprises determining the number of sequence reads covering a predefined genomic region of at least 60 bps in length that contains said restriction locus, for example a predefined genomic region of at least 70 bps, at least 80 bps, at least 90 bps, at least 100 bps, between 50-150 bps, between 50-120 bps, between 50-100 bps that contains the restriction locus. Each possibility represents a separate embodiment.

In some embodiments, the at least one restriction locus is located within a CG-island. "CG islands" (or CpG islands) are regions of DNA with a high G/C content and a high frequency of CG dinucleotides relative to the whole genome of an organism of interest. CG islands are typically between 200-3,000 bps in length and are typically characterized by a GC content greater than 50% and an observed:expected CG ratio of more than 0.6. Genomic regions of lower CG density are termed "CG oceans" and comprise most of the genome.

In some embodiments, there is provided a method for profiling methylation of a DNA sample from a subject, the method comprising (i) subjecting a DNA sample from the subject to digestion with at least one methylation-sensitive/-dependent restriction endonuclease (ii) sequencing the digested DNA by a high-throughput sequencing method; wherein the method can independently determine the methylated and unmethylated levels of DNA in a single assay and based on the same sequencing data.

In some embodiments, there is provided a method for profiling methylation of a DNA sample from a subject, the method comprising:

(a) providing a DNA sample from a subject;

(b) subjecting the DNA sample to digestion with at least one methylation-sensitive restriction endonuclease, thereby obtaining restriction endonuclease-treated DNA comprising restriction endonuclease-generated DNA fragments;

(c) performing high-throughput sequencing of the endonuclease-treated DNA to obtain a plurality of sequence reads;

(d) determining from the sequence reads a read count of at least one restriction locus, the read count representing the number of DNA molecules in the DNA sample in which said at least one restriction locus was methylated and therefore remained intact;

(e) determining from the sequence reads a read count of sequence reads starting or ending at a nucleotide within the at least one restriction locus, the read count representing the number of DNA molecules in the DNA sample in which said at least one restriction locus was unmethylated and therefore cut by the restriction endonuclease;

(f) calculating a level of methylated DNA at the at least one restriction locus based on the read count of the at least one restriction locus determined in step (d), and a level of unmethylated DNA at the at least one restriction locus based on the read count of sequence reads starting or ending at a nucleotide within the at least one restriction locus determined in step (e), thereby profiling methylation of the DNA sample.

In additional embodiments, there is provided a method for profiling methylation of a DNA sample from a subject, the method comprising:

(A) providing a DNA sample from a subject;

(B) subjecting the DNA sample to digestion with at least one methylation-sensitive restriction endonuclease, thereby obtaining restriction endonuclease-treated DNA comprising a plurality of restriction endonuclease-generated DNA fragments;

(C) preparing a sequencing library from the restriction endonuclease-treated DNA and subjecting the sequencing library to high-throughput sequencing to obtain sequence reads;

(D) mapping a plurality of the sequence reads against a reference genome to produce mapped sequence reads, and selecting at least one restriction locus within the reference genome;

(E) determining from the mapped sequence reads a read count of the at least one restriction locus, the read count representing the number of DNA molecules in the DNA sample in which said at least one restriction locus was methylated and therefore remained intact;

(F) determining from the mapped sequence reads a read count of sequence reads starting or ending at a nucleotide within the at least one restriction locus, the read count representing the number of DNA molecules in the DNA sample in which said at least one restriction locus was unmethylated and therefore cut by the restriction endonuclease;

(G) calculating a level of methylated DNA at the at least one restriction locus based on the read count of the at least one restriction locus determined in step (E) and a level of unmethylated DNA at the at least one restriction locus based on the read count of sequence reads starting or ending at a nucleotide within the at least one restriction locus determined in step (F), thereby profiling methylation of the DNA sample.

In some embodiments, in a method for profiling methylation of a DNA sample from a subject comprising subjecting the DNA sample to digestion by at least one methylation-sensitive restriction endonuclease, performing high-throughput sequencing of the digested sample, determining a read count of at least one restriction locus and calculating a level of methylated DNA at the at least one restriction locus based on the read count, the improvement comprises:

determining a read count of sequence reads starting or ending at a nucleotide within the at least one restriction locus, the read count representing the number of DNA molecules in the DNA sample in which said at least one restriction locus was unmethylated and therefore cut by the restriction endonuclease;

calculating a level of unmethylated DNA at the at least one restriction locus based on the read count of sequence reads starting or ending at a nucleotide within the at least one restriction locus; and

profiling methylation of the DNA sample using the levels of methylated and unmethylated DNA at the at least one restriction locus.

The term "plurality" as used herein refers to 'at least two' or 'two or more'.

In some embodiments, there is provided a method for identifying the presence or absence of a disease in a subject, comprising: profiling methylation of a DNA sample from the subject as disclosed herein; comparing the methylation profile of the DNA sample to one or more reference methylation profile; and determining the presence or absence of the disease in the subject based on the comparison.

In some embodiments, there is provided a method for identifying a DNA methylation marker indicative of the source of a DNA sample comprising profiling methylation as disclosed herein. In additional embodiments, there is provided herein a method for assessing the quality of a DNA methylation marker comprising profiling methylation as disclosed herein. In some embodiments, the DNA methylation marker is a marker indicative of the presence or absence of a disease, e.g., a type of cancer. In additional embodiments, the DNA methylation marker is a marker indicative of a stage of a disease, e.g., a cancer stage. In additional embodiments, the DNA methylation marker is a marker indicative of a type of tissue (e.g., lung tissue, breast tissue, colon tissue etc.).

In some embodiments, there is provided the use of: (i) at least one methylation-sensitive restriction enzyme and/or at least one methylation-dependent restriction enzyme; and (ii) high-throughput sequencing, for direct determination of methylated and unmethylated DNA levels of at least one restriction locus in a DNA sample.

In some embodiments, there is provided the use of: (i) at least one methylation-sensitive restriction enzyme and/or at least one methylation-dependent restriction enzyme; and (ii) high-throughput sequencing, for profiling methylation of a DNA sample by direct determination of methylated and unmethylated DNA levels of at least one restriction locus in a DNA sample, wherein said determination of methylated and unmethylated DNA levels is based on the same sequencing data.

In some embodiments, there is provided the use of sequence reads produced following digestion of a DNA sample with at least one methylation-sensitive restriction enzyme and/or at least one methylation-dependent restriction enzyme and high-throughput sequencing, for profiling methylation of the DNA sample by direct determination of methylated and unmethylated DNA levels of at least one restriction locus in the DNA sample, wherein said determination of methylated and unmethylated DNA levels is based on the same sequencing data

In general, embodiments which can be performed with methylation-sensitive restriction enzyme(s) can be done alternatively with methylation-dependent restriction enzyme(s), and downstream steps will be adjusted accordingly. For example, in some embodiments, following high-throughput sequencing and generation of sequence reads, a method for profiling methylation according to the present invention comprises: selecting at least one restriction locus and determining the number of sequence reads covering a predefined genomic region of at least 50 bps in length that contains said restriction locus; and calculating a methylation value based on the read count of the predefined genomic region and a reference read count, the calculated methylation value reflects the number of molecules that were unmethylated in the DNA sample and therefore remained intact following digestion with methylation-dependent restrictions enzymes(s).

As another example, in some embodiments, for calculating a level of methylated DNA of a restriction locus, following high-throughput sequencing and generation of sequence reads, the method comprises: determining from the sequence reads a read count of sequence reads starting or ending at a nucleotide within the restriction locus, the read count representing the number of DNA molecules in the DNA sample in which said restriction locus was methylated and therefore cut by the restriction endonuclease; and calculating a level of methylated DNA at the restriction locus based on the determined read count of sequence reads starting or ending at a nucleotide within the restriction locus. For calculating a level of unmethylated DNA of a restriction locus, in some embodiments, the method comprises: determining from the sequence reads a read count of the restriction locus, the read count representing the number of DNA molecules in the DNA sample in which said restriction locus was unmethylated and therefore remained intact; and calculating a level of unmethylated DNA at the restriction locus based on the determined read count of the restriction locus.

## DNA sample

A DNA sample for use according to the present invention may be obtained from any biological sample of a subject from which nucleic acids can be obtained, including biological fluid samples such as blood, plasma, serum, urine, cerebrospinal fluid, semen, stool, sputum and amniotic fluid. Each possibility represents a separate embodiment of the present invention. Biological samples also include tissue and organ samples.

A "subject" according to the present invention is typically a human subject. The subject may be suspected of having a certain disease. In some embodiments, the subject is diagnosed with a disease of interest. In other embodiments, the subject is a healthy subject that does not have the disease of interest. The subject may also be at risk of developing the disease, for example, based on previous history of the disease, genetic predisposition, and/or family history, and/or a subject who exhibits suspicious clinical signs of the disease and/or a subject that is suspected of having the disease based on other prior assay(s) e.g., based on testing of other biomarker(s). In some embodiments, the subject is at risk of recurrence of the disease. In some embodiments, the subject shows at least one symptom or characteristic of the disease. In other embodiments, the subject is asymptomatic.

In some embodiments, the DNA sample is cell-free DNA extracted from a biological fluid sample. The term "cell-free DNA" (abbreviated "cfDNA") refers to DNA molecules which are freely circulating in body fluids and are not contained within intact cells. The origin of cfDNA is not fully understood but believed to be related to apoptosis, necrosis and active release from cells. cfDNA is released by both normal and tumor cells. cfDNA is highly fragmented, with fragments typically ranging between 120-220 bps in length, mostly between 150-180 bps in length. It is to be understood that the term "cell-free DNA" as used herein refers to DNA which is already cell-free in the body of the subject. It is to be understood that for cell-free DNA samples, "restriction endonuclease-treated DNA" comprises fragments generated as a result of the digestion, and also natural cell-free DNA fragments, for example, cell-free DNA fragments that do not contain a recognition sequence of the enzyme(s) used in the assay and cell-free DNA fragments that contain one or more recognition sequences of the enzyme(s) that are all methylated and therefore not cut by the enzyme.

Alternatively, the DNA sample may be DNA extracted from cells, for example, DNA extracted from tissue or organ samples or from blood cells. Typically, cell lysis is required in order to extract the DNA. DNA may be obtained from tumor samples or from healthy tissues. A "tumor sample" as used herein encompasses a whole tumor resected by surgery or portions thereof. A "tumor sample" also encompasses a sample taken from a tumor by biopsy, and a sample taken from a lesion or a tissue suspected of being cancerous. Tumor samples for use according to the present invention include fresh tumor samples as well as frozen/preserved tumor samples.

For DNA extracted from cells, a step of fragmenting the DNA into fragments suitable for high-throughput sequencing may be carried out before, after or during the digestion with the at least one methylation-sensitive or methylation-dependent restriction endonuclease according to the present invention, to simplify downstream processing and preparation of a sequencing library. Such fragmentation can be carried out, for example, using sonication, or using a restriction endonuclease which is insensitive to methylation, namely, cleaves its recognition sequence regardless of methylation status. It can also be carried out using a restriction endonuclease with a recognition sequence that does not include CG dinucleotides.

The present invention encompasses whole-genome sequencing as well as target-specific sequencing (e.g., sequencing of CpG islands, exons or specific loci of interest). For target-specific sequencing, genomic regions of interest are enriched, for example, using capture agents such as sequence-specific probes attached to beads. Typically, enrichment of genomic regions of interest is carried out after the methylation-sensitive/-dependent digestion according to the present invention and after sequencing library preparation, as will be described in more detail below. In some embodiments, enrichment may be carried out prior to digestion and library preparation.

Thus, in some embodiments, the DNA sample that is subjected to methylation-sensitive or methylation-dependent digestion according to the present invention is an unprocessed DNA sample, namely, a DNA sample as extracted from a biological sample. In other embodiments, the DNA sample is a processed DNA sample, for example, enriched for certain regions of interest and/or fragmented to reduce size prior to the digestion with the at least one methylation-sensitive or methylation-dependent restriction endonuclease according to the present invention.

Preferably, the DNA sample on which the methylation analysis is carried out is substantially free of single-stranded DNA (ssDNA). As used herein, "substantially free of ssDNA" or "substantially devoid of ssDNA" indicates a DNA sample in which less than 7% of the DNA is ssDNA, preferably less than 5% of the DNA is ssDNA, more preferably less than 1% of the DNA is ssDNA (namely, at least 99% of the DNA is double-stranded) (by number of molecules). In some embodiments, the DNA sample contains less than 0.1% ssDNA. In some embodiments, the DNA sample contains less than 0.01% ssDNA. In some embodiments, the DNA sample contains no ssDNA (free of ssDNA). Extraction of DNA to obtain a DNA sample substantially free of ssDNA is described, for example, in WO 2020/188561, assigned to the Applicant of the present invention. An exemplary kit for extracting cell-free DNA which is suitable for use with the method of the present invention is QIAamp® Circulating Nucleic Acid Kit (QIAGEN, Hilden, Germany). An exemplary kit for extracting DNA from cells is QIAamp® Blood Mini Kit.

<u>DNA digestion</u>

According to the present invention, following extraction (and optionally enrichment for regions of interest and/or fragmentation to reduce size) the DNA is subjected to digestion with at least one methylation-sensitive restriction endonuclease and/or at least one methylation-dependent restriction endonuclease, preferably with a plurality of methylation-sensitive restriction endonucleases (or a plurality of methylation-dependent restriction endonucleases) applied simultaneously. As used herein, "restriction endonucleases applied simultaneously" or "simultaneous digestion" means that the enzymes are present together in the reaction mixture in an active form, without inactivation of one prior to application of another.

For example, one, two, three, four or five methylation-sensitive and/or methylation-dependent restriction endonucleases may be used. Each number of endonucleases used in the assay represents a separate embodiment of the present invention.

In some embodiments, the entire DNA that was extracted is used in the digestion step. In some embodiments, the DNA is not quantified prior to being subjected to digestion. In other embodiments, the DNA is quantified prior to digestion thereof. In some embodiments, the DNA is aliquoted into a first aliquot that is subjected to digestion and a second aliquot that is kept as an undigested control.

A "restriction endonuclease", used herein interchangeably with a "restriction enzyme", refers to an enzyme that cuts DNA at or near specific recognition sequences, also known as restriction sites. Restriction sites are usually 4 to 8 nucleotide long and are typically palindromic (i.e., the DNA sequences are the same in both directions).

A "methylation-sensitive" restriction endonuclease is a restriction endonuclease that cleaves its recognition sequence only if it is unmethylated (while methylated sites remain intact). Thus, the extent of digestion of a DNA sample by a methylation-sensitive restriction endonuclease depends on the methylation level, where a higher methylation level protects from cleavage and accordingly results in less digestion. A DNA sample treated with a methylation-sensitive restriction endonuclease is characterized by intact methylated sites and cut unmethylated sites. It is to be understood that there is no need for 100% digestion efficiency and thus some unmethylated sites might remain intact. In some embodiments, the methods of the present invention comprise determining the digestion efficacy, and proceeding to preparing a sequencing library if the digestion efficacy is above a predefined threshold/level.

A "methylation-dependent" restriction endonuclease is a restriction endonuclease that cleaves its recognition sequence only if it is methylated (while unmethylated sites remain intact). Thus, the extent of digestion of a DNA sample by a methylation-dependent restriction endonuclease depends on the methylation level, where a higher methylation level results in more extensive digestion.

Methylation-sensitive restriction endonuclease(s) for use according to the present invention may be selected from the group consisting of: AatII, Acc65I, AccI, AciI, ACII, Afel, Agel, Apal, ApaLI, AscI, AsiSI, Aval, AvaII, BaeI, BanI, BbeI, BceAI, BcgI, BfuCI, BglI, BmgBI, BsaAI, BsaBI, BsaHI, BsaI, BseYI, BsiEI, BsiWI, BsII, BsmAI, BsmBI, BsmFI, BspDI, BsrBI, BsrFI, BssHII, BssKI, BstAPI, BstBI, BstUI, BstZl7I, Cac8I, ClaI, DpnI, DrdI, EaeI, EagI, Eagl-HF, EciI, EcoRI, EcoRI-HF, FauI, Fnu4HI, FseI, FspI, HaeII, HgaI, HhaI, HincII, HincII, Hinfl, HinPII, HpaI, HpaII, Hpyl66ii, Hpyl88iii, Hpy99I, HpyCH4IV, KasI, MluI, MmeI, MspAlI, MwoI, NaeI, NacI, NgoNIV, Nhe-HFI, NheI, NlaIV, NotI, Notl-HF, NruI, Nt.BbvCI, Nt.BsmAI, Nt.CviPII, PaeR7I, PleI, PmeI, PmlI, PshAI, PspOMI, PvuI, RsaI, RsrII, SacII, SalI, Sall-HF, Sau3AI, Sau96I, ScrFI, SfiI, SfoI, SgrAI, SmaI, SnaBI, TfiI, TscI, TseI, TspMI, and ZraI. Each possibility represents a separate embodiment of the present invention. In some particular embodiments, the at least one methylation-sensitive restriction endonuclease comprises HinP1I. In additional particular embodiments, the at least one methylation-sensitive restriction endonuclease comprises HhaI. In yet additional particular embodiments, the at least one methylation-sensitive restriction endonuclease comprises AciI.

Methylation-dependent restriction endonuclease(s) may be selected from the group consisting of: McrBC, McrA, and MrrA. Each possibility represents a separate embodiment of the present invention.

In some embodiments, a DNA sample according to the present invention is subjected to digestion with a single methylation-sensitive restriction endonuclease. In some particular embodiments, the methylation-sensitive restriction endonuclease is HinP1I. In additional particular embodiments, the methylation-sensitive restriction endonuclease is HhaI. In additional embodiments, the DNA sample is subjected to digestion with two methylation-sensitive restriction endonucleases.

In some particular embodiments, the methylation-sensitive restriction endonucleases HinP1I and AciI are used.

In some embodiments, there is provided a method for profiling methylation of a DNA sample, the method comprising: subjecting the DNA sample to digestion with the methylation-sensitive restriction endonucleases HinP1I and AciI; and analyzing methylation of at least one restriction locus of HinP1I and/or at least one restriction locus of AciI, thereby profiling methylation of the DNA sample. In some embodiments, the method comprises subjecting the DNA sample to digestion with the methylation-sensitive restriction endonucleases HinP1I and AciI; and determining a level of methylated DNA and optionally a level of unmethylated DNA of at least one restriction locus of HinP1I and/or at least one restriction locus of AciI, thereby profiling methylation of the DNA sample. In some embodiments, the DNA sample is cell-free DNA extracted from a biological fluid.

In some embodiments, HinP1I and AciI at a ratio between 1:1 to 5:1 (enzyme units) (Hinp:AciI) are used with the methods and systems of the present invention, for example 2:1, 2.5:1, 3:1, 3.5:1, 4:1 and 4.5:1(enzyme units) (Hinp:AciI). Each possibility represents a separate embodiment of the present invention. In some embodiments, HinP1I and AciI at a ratio between 2:1 to 4.5:1 (enzyme units) (Hinp:AciI) are used with the methods and systems of the present invention.

In some embodiments, there is provided a method for detecting methylation changes in a DNA sample, the method comprising: profiling methylation of the DNA sample using HinP1I and AciI digestion; and comparing the methylation profile to one or more reference methylation profile. In some embodiments, the DNA sample is cell-free DNA extracted from a biological fluid.

In some embodiments, there is provided a method for profiling methylation of a DNA sample, the method comprising: subjecting the DNA sample to digestion with the methylation-sensitive restriction endonucleases HinP1I and AciI, thereby obtaining restriction endonuclease-treated DNA comprising restriction endonuclease-generated DNA fragments; performing high-throughput sequencing of the endonuclease-treated DNA to obtain a plurality of sequence reads; determining from the sequence reads a level of methylated DNA and optionally a level of unmethylated DNA of at least one restriction locus of HinP1I and/or at least one restriction locus of AciI, thereby profiling methylation of the DNA sample. In some embodiments, the DNA sample is cell-free DNA extracted from a biological fluid.

In some embodiments, there is provided a reaction mixture comprising: human cell-free DNA extracted from a biological fluid; and the methylation-sensitive restriction endonucleases HinP1I and AciI. The reaction mixture further comprises a buffer suitable for activity of HinP1I and AciI. In some embodiments, HinP1I and AciI are present in the reaction mixture at a ratio between 1:1 to 5:1 (enzyme units) (Hinp:AciI), for example 2:1, 2.5:1, 3:1, 3.5:1, 4:1 and 4.5:1(enzyme units) (Hinp:AciI). Each possibility represents a separate embodiment of the present invention. In some embodiments, the reaction mixture comprises HinP1I and AciI at a ratio between 2:1 to 4.5:1 (enzyme units) (Hinp:AciI).

In some embodiments, there is provided a method of processing a cell-free DNA sample for genetic and epigenetic analysis, the method comprising providing the reaction mixture disclosed herein, incubating the reaction mixture to obtain restriction endonuclease-treated cell-free DNA in which methylated restriction sites are intact and unmethylated restriction sites are cut, and subjecting the restriction endonuclease-treated cell-free DNA to high-throughput sequencing.

Digestion efficacy can be evaluated either internally to the examined sample, or externally. Internal evaluation can be performed by measuring intact cut sites of genomic positions that are known to be ubiquitously unmethylated. An example of such a locus can be any site on the mitochondrion DNA. External evaluation of digestion efficacy can be performed either by including an unmethylated sample in the digestion step, digesting both samples in parallel, and then verifying that the unmethylated sample was indeed digested (by measuring numbers of intact cut sites). Such an unmethylated sample could be, for example, PCR amplicons, plasmid DNA, commercial unmethylated DNA species, or cell line DNA that is known to be unmethylated in certain genomic positions. Alternatively, external evaluation of digestion efficacy can be achieved in a single step, by spiking in an unmethylated sample into the interrogated sample, and measuring the digestion of the unmethylated DNA sample in the same step as the interrogated sample. For this purpose, it is possible to use all types of unmethylated DNA species mentioned above. In some embodiments, the use of small targets is preferred, such as PCR amplicons or plasmid DNA.

In some embodiments, DNA digestion may be carried out to complete digestion. In some exemplary embodiments, the methylation-sensitive restriction endonuclease is HinP1I and/or AciI, and complete digestion may be achieved following one to two hours incubation with the enzyme(s) at 37°C.

Library preparation and sequencing

"High throughput sequencing," (also termed "next generation sequencing") includes sequence determination using methods that determine many (typically thousands to billions) of nucleic acid sequences in parallel. High throughput sequencing generally involves three basic steps: library preparation, sequencing and data analysis. Examples of high throughput sequencing techniques include sequencing-by-synthesis and sequencing-by-ligation (employed, for example, by Illumina Inc., Life Technologies Inc., Roche), nanopore sequencing methods and electronic detection-based methods such as Ion Torrent™ technology (Life Technologies Inc.).

Library preparation for the major high-throughput sequencing platforms requires the ligation of specific adapter oligonucleotides to fragments of the DNA to be sequenced. As disclosed herein, restriction digestion is preferably carried out before adapter ligation to avoid possible digestion of the adapters by the enzymes. The digestion of DNA by the methylation-sensitive/dependent restriction endonuclease(s) as disclosed herein typically does not result in homogeneous, blunt-ended fragments. Thus, end repair is needed to ensure that each DNA molecule is free of overhangs, and contains 5' phosphate and 3' hydroxyl groups. A typical blunting enzyme mix includes a polymerase and a polynucleotide kinase, for example, T4 DNA polymerase and T4 polynucleotide kinase (PNK). T4 DNA polymerase (in the presence of dNTPs) can fill-in 5' overhangs and trim 3' overhangs down to the dsDNA interface to generate the blunt ends. The T4 PNK can then phosphorylate the 5' terminal nucleotide. For Illumina libraries, incorporation of a non-templated deoxyadenosine 5'-monophosphate (dAMP) onto the 3' end of blunted DNA fragments, a process known as dA-tailing, is also required for library preparation. dA-tails prevent concatamer formation during downstream ligation steps, and enable DNA fragments to be ligated to adapter oligonucleotides with complementary dT-overhangs.

As disclosed herein, adapter oligonucleotides, also termed "sequencing adapters", are ligated to the DNA fragments using end-preserving methods such as enzymatic ligation in which a ligase enzyme covalently links a sequencing adapter to a DNA fragment, making a complete library molecule. Sequencing adapters are ligated at the 5' and 3' ends of DNA fragments in the sequencing library. Sequencing adapters typically include platform-specific sequences for fragment recognition by a particular sequencer: for example, sequences that enable library fragments to bind to the flow cells of Illumina platforms. Each sequencing instrument provider typically uses a specific set of sequences for this purpose.

Sequencing adapters may also include sample indices. "Sample indices", also termed "sample barcodes" are sequences that enable multiple samples to be sequenced together (i.e., multiplexed) on the same instrument flow cell or chip. Each sample index, typically 6-10 bases, is specific to a given sample library and is used for de-multiplexing during data analysis to assign individual sequence reads to the correct sample. Sequencing adapters may contain single or dual sample indexes depending on the number of libraries combined and the level of accuracy desired.

Sequencing adapters may include unique molecular identifiers (UMIs). UMIs are a type of molecular barcodes that provide molecular tracking, error correction and increased accuracy during sequencing. UMIs are short sequences, typically 5 to 20 bases in length, used to uniquely tag each molecule in a sample library. Since each nucleic acid in the starting material is tagged with a unique molecular barcode, bioinformatics software can filter out duplicate reads and PCR errors with a high level of accuracy and report unique reads, removing the identified errors before final data analysis.

In some embodiments, both a sample barcode sequence and a UMI are incorporated into a nucleic acid target molecule.

The methods disclosed herein do not require differential adapter tagging of digested vs. undigested DNA molecules (namely, differential adapter tagging of methylated vs. unmethylated DNA molecules), and the same population of adapters are used for the entire sample, such that any adapter in the mixture is capable of ligation to both the digested and undigested DNA.

High-throughput sequencing according to the present invention may be performed using various high-throughput sequencing instruments and platforms, including but not limited to: Novaseq™, Nextseq™ and MiSeq™ (Illumina), 454 Sequencing (Roche), Ion Chef™ (ThermoFisher), SOLiD® (ThermoFisher) and Sequel II™ (Pacific Biosciences). The appropriate platform-designed sequencing adapters are used for preparing the sequencing library.

In some embodiments, whole genome sequencing is performed on libraries prepared from endonuclease-treated DNA. The libraries are prepared using sequencing adapters suitable for the sequencing platform being used.

In other embodiments, region(s) of interest in the endonuclease-treated DNA can be captured using, for example, a solution-phase or solid-phase hybridization-based process, followed by the high-throughput sequencing. Enrichment of regions of interest followed by high-throughput sequencing is referred to herein as "target-specific high-throughput sequencing". Target-specific high-throughput sequencing includes, for example, CpG island sequencing and exome sequencing. Target-specific high-throughput sequencing also includes sequencing of specific informative genomic regions, for example, regions known to be differentially methylated between cancer and non-cancer tissues. Capture of genomic regions for target-specific sequencing is typically carried out after library preparation. In some embodiments, the methods disclosed herein comprise enriching genomic regions of interest. In order to preserve the ends of the DNA fragments in the DNA sample (e.g., to allow analysis of sequences starting or ending at nucleotides within restriction loci), enrichment according to the present invention is typically not carried out using PCR amplification of the genomic regions of interest.

In some embodiments, a method for genetic and epigenetic profiling of DNA samples according to the present invention comprises:

extracting DNA from a biological sample;
subjecting the extracted DNA to digestion with at least one methylation-sensitive restriction endonuclease, thereby obtaining restriction endonuclease-treated DNA;
preparing a sequencing library from the restriction endonuclease-treated DNA using sequencing adapters ligated to DNA fragments in the restriction endonuclease-treated DNA;
enriching at least one (preferably a plurality of) genomic regions of interest from the sequencing library using capture agents, to obtain a sequencing library enriched with the at least one (preferably a plurality of) genomic regions of interest;
subjecting the sequencing library enriched with the at least one (preferably a plurality of) genomic regions of interest to high-throughput sequencing; and
determining from the sequencing data a methylation value for at least one restriction locus and optionally at least one additional genetic or epigenetic characteristic of the cell-free DNA sample selected from DNA mutation, copy number variation and nucleosome positioning as disclosed herein.

In some embodiments, a method for profiling methylation according to the present invention comprises:

extracting DNA from a biological sample;
subjecting the extracted DNA to digestion with at least one methylation-sensitive restriction endonuclease, thereby obtaining restriction endonuclease-treated DNA;
preparing a sequencing library from the restriction endonuclease-treated DNA using sequencing adapters ligated to DNA fragments in the restriction endonuclease-generated DNA;
enriching at least one (preferably a plurality of) genomic regions of interest from the sequencing library using capture agents, to obtain a sequencing library enriched with the at least one (preferably a plurality of) genomic regions of interest;
subjecting the sequencing library enriched with the at least one (preferably a plurality of) genomic regions of interest to high-throughput sequencing to obtain sequence reads; and
determining a level of methylated DNA and a level of unmethylated DNA of at least one restriction locus within the genomic regions of interest as disclosed herein.

Analysis of sequence reads

In some embodiments, "sequence reads" (or simply, "reads"), namely, nucleotide sequences produced by the sequencing process, are mapped against a reference genome. A "reference genome" as used herein refers to a previously identified genome sequence, whether partial or complete, assembled as a representative example of a species or subject. A reference genome is typically haploid, and typically does not represent the genome of a single individual of the species but rather is a mosaic of the genomes of several individuals. A reference genome for the methods of the present invention is typically a human reference genome. In some embodiments, the reference genome is the complete human genome, such as the human genome assemblies available at the website of the National Center for Biotechnology Information (NCBI) or at the University of California, Santa Cruz (UCSC) Genome Browser. An example of a suitable reference genome for human studies is the 'hg18' genome assembly. As an alternative, the more recent GRCh38 major assembly can be used (going up to patch p13).

Read mapping is the process to align the reads on a reference genome in order to identify the location of the reads within the reference genome. The sequence reads that align are designated as being "mapped". The alignment process aims to maximize the possibility for obtaining regions of sequence identity across the various sequences in the alignment, allowing mismatches, indels and/or clipping of some short fragments on the two ends of the reads. The number of reads mapped to a certain genomic locus of interest is referred to herein as the "read count" or "copy number" of this genomic locus. Computer software may be used to analyze sequence reads, map sequence reads against a reference genome and quantify the number of reads.

The terms "genomic locus" and "locus" as used herein are interchangeable and refer to a DNA sequence at a specific location within the genome. A "locus" may include a single position (a single nucleotide at a defined position in the genome) or a stretch or nucleotides starting and ending at defined positions in the genome. The specific position(s) may be identified by the molecular location, namely, by the chromosome and the numbers of the starting and ending base pairs on the chromosome. A variant of a DNA sequence at a given genomic position is called an allele. Alleles of a locus are located at identical sites on homologous chromosomes. Genomic loci include gene sequences as well as other genetic elements (e.g., intergenic sequences).

A "restriction locus" is used herein to describe a genomic locus which is a restriction site of a methylation-sensitive/-dependent restriction endonuclease applied in the digestion step according to the present invention. Restriction loci according to the present invention may be differentially methylated between normal and disease DNA, meaning that for a given disease for which the analysis is carried out, for example, a certain type of cancer, the restriction loci differ in their methylation level between normal DNA and DNA derived from cancer cells. For example, DNA from the cancer cells may have an increased methylation level at the restriction loci compared to normal non-cancerous DNA. More particularly, the restriction loci contain CG dinucleotides that are more methylated in cancer DNA compared to normal non-cancerous DNA. According to the present invention, the differentially methylated CG dinucleotides are located within recognition sites of the at least one restriction enzyme applied in the digestion step.

In some embodiments, a restriction locus according to the present invention contains a CG dinucleotide which is more methylated in cell-free DNA, e.g., plasma DNA, of subjects with a certain type of cancer than in cell-free DNA of healthy subjects. In some embodiments, plasma samples of the cancer patients contain a greater proportion of DNA molecules that are methylated at the restriction locus compared to plasma samples of healthy subjects.

In additional embodiments, a restriction locus according to the present invention contains a CG dinucleotide which is more methylated in DNA from a cancerous tissue (e.g., a tumor sample) than in DNA from a non-cancerous tissue, meaning that in the cancerous tissue a greater proportion of DNA molecules are methylated at this position compared to the non-cancerous tissue.

A methylation-sensitive restriction enzyme cleaves its recognition sequence only if it is unmethylated. A methylation-dependent restriction enzyme cleaves its recognition sequence only if it is methylated. Thus, differences in methylation levels between samples result in differences in the degree of digestion, and subsequently different amounts of sequence reads in the following sequencing and quantification steps. Such differences enable distinguishing between DNA from different samples, for example, between DNA samples from subjects with cancer and DNA samples from healthy subjects.

The terms "level of methylated DNA", "methylation level" or "methylation value" of a restriction locus is a numerical value representing the number of DNA molecules that are methylated at this restriction locus (namely, methylated at a CG dinucleotide within the restriction locus) out of the total number of DNA molecules containing the restriction locus in the sample. In some embodiments, the level of methylated DNA of a restriction locus is calculated herein from the read count of the restriction locus following digestion with at least one methylation-sensitive restriction endonuclease. In additional embodiments, the level of methylated DNA of a restriction locus is calculated herein from the read count of a predefined genomic region of at least 50 bps that contains the restriction locus. As methylation-sensitive restriction endonucleases cleave their recognition sequence only if it is unmethylated, the read count of the restriction locus represents the number of DNA molecules in the DNA sample in which the restriction locus was methylated and therefore remained intact.

In some embodiments, the methylation level of the restriction locus is calculated by dividing the read count of the restriction locus, or the read count of a predefined genomic region of at least 50 bps that contains the restriction locus, by an expected read count of the restriction locus or the predefined genomic region of at least 50 bps that contains the restriction locus. An expected read count of the restriction locus/ predefined genomic region may be determined, for example, using: (i) read count of a reference locus/genomic region of the same length as the restriction locus/genomic region, that is not cut by the restriction endonuclease; (ii) average read count of a plurality of reference loci/genomic regions of the same length as the restriction locus/genomic region, that are not cut by the restriction endonuclease; or (iii) read count of the restriction locus/predefined genomic region in an undigested control DNA sample, optionally corrected for sequencing depth differences. Exemplary calculations are provided in the Examples section that follows.

In additional embodiments, the methylation level is calculated by determining a total fragment number, which is determined from the read count of the restriction locus and read count of sequence reads starting or ending at a nucleotide within the restriction locus. Exemplary calculations are provided in the Examples section that follows.

In some embodiments, methylation level is expressed as percentage (%) of methylation, representing the percentage of DNA molecules that are methylated at the restriction locus out of the total number of DNA molecules containing the restriction locus in the sample.

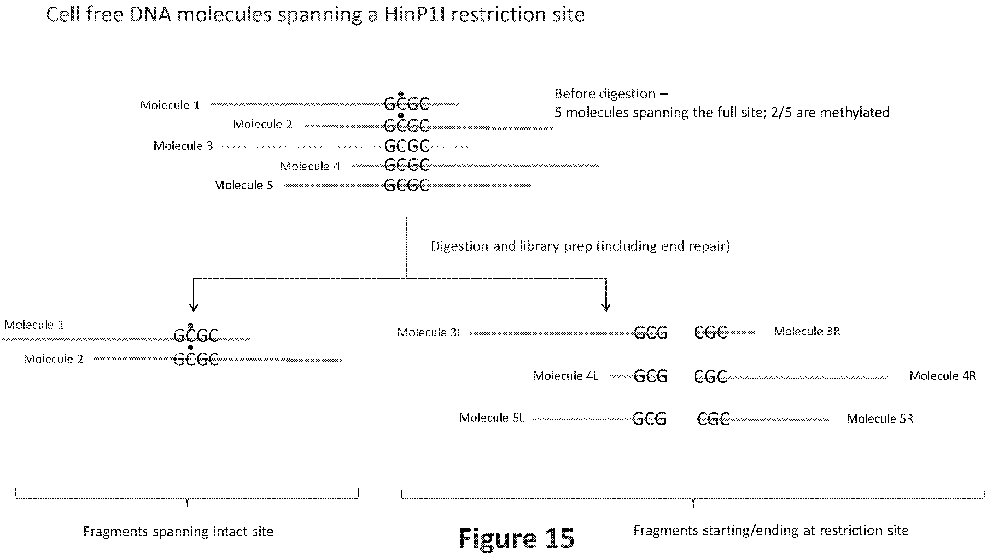

The terms "level of unmethylated DNA" or "unmethylation level" of a restriction locus is a numerical value representing the number of DNA molecules that are unmethylated at this restriction locus (namely, unmethylated at a CG dinucleotide within the restriction locus) out of the total number of DNA molecules containing the restriction locus in the sample. As disclosed herein, the level of unmethylated DNA of a restriction locus is calculated from the number of reads starting or ending at a nucleotide within the restriction locus following digestion with at least one methylation-sensitive restriction endonuclease and any subsequent end repair. The exact nucleotide within the restriction locus in which the sequence reads start or end depends on the type of restriction endonuclease used in the digestion step and the length of its recognition sequence. For example, for restriction endonucleases that produce non-blunt ends with 5' overhangs, digestion and end repair result in fragments that start at the second nucleotide of the recognition sequence and fragments that end at the penultimate nucleotide of the recognition sequence. For example, for a 4-base cutter that produces non-blunt ends with 5' overhangs, digestion and end repair result in fragments that start at the second nucleotide of the recognition sequence and fragments that end at the third nucleotide of the recognition sequence (**Figure 15**). Thus, for a restriction endonuclease which produces non-blunt ends with 5' overhangs, "start" analysis of its restriction loci is carried out on sequence reads that start at the second nucleotide of the restriction loci (second nucleotide of the recognition sequence), and "end" analysis is carried out on sequence reads that end at the penultimate nucleotide of the restriction loci (penultimate nucleotide of the recognition sequence).

As methylation-sensitive restriction endonucleases cleave their recognition sequence only if it is unmethylated, the number of reads starting or ending at a nucleotide within the restriction locus represent the number of DNA molecules in the DNA sample in which the restriction locus was unmethylated and therefore cut by the restriction endonuclease.

Each DNA molecule that is cut by the restriction endonuclease as disclosed herein results in two fragments, one that starts at a nucleotide within the restriction locus and another that ends at a nucleotide within the restriction locus. Thus, for a given DNA molecule it may be possible to obtain two different sequence reads. For correct analysis of the number of unmethylated DNA molecules that were present in the sample, the level of unmethylation may be calculated based on the number of sequence reads starting at the restriction locus, the number of sequence reads ending at the restriction locus or by an average between the two values, but not based on a sum of the values. As disclosed herein, calculating a level of unmethylated DNA at a restriction locus based on the read count of sequence reads starting or ending at nucleotides within the restriction locus encompasses calculating a level of unmethylated DNA using an average between the two values.

It is further noted that some library preparation methods may result in depletion of small fragments which are subsequently not sequenced. Such depletion could result in underestimation of the unmethylated level and overestimation of the methylated level. In addition, the number of sequence reads that start at a restriction locus may differ from the number of sequence reads that end at the restriction locus. The present invention advantageously addresses such library preparation bias. To reduce this bias and achieve a more accurate result, it is preferable to determine both the number of reads staring at the restriction locus and the number of reads ending at the restriction locus, and subsequently select the orientation which provides the larger number of reads for further analysis and calculations, or calculate an average between the two values and use the average for further analysis and calculations.

Thus, in some embodiments, the method of the present invention comprises: determining a number of sequence reads starting at a nucleotide within the restriction locus; determining a number of sequence reads ending at a nucleotide within the restriction locus; and calculating a level of unmethylated DNA at the restriction locus using the orientation that provides the larger number of sequence reads. In additional embodiments, the method of the present invention comprises: determining a number of sequence reads starting at a nucleotide within the restriction locus; determining a number of sequence reads ending at a nucleotide within the restriction locus; calculating an average between the two values; and using the average to calculate a level of unmethylated DNA at the restriction locus.

The number of sequence reads starting or ending at a nucleotide within the restriction locus may be normalized by subtracting an expected number of sequence reads starting or ending at a nucleotide within the restriction locus. An expected number of sequence reads starting or ending at a nucleotide within the restriction locus may be determined, for example, using: (i) number of sequence reads starting or ending at a reference locus of the same size as the restriction locus, that is not cut by the restriction endonuclease; (ii) average number of sequence reads starting or ending at a plurality of reference loci of the same size as the restriction locus, that are not cut by the enzyme; or (iii) number of reads starting or ending at the restriction locus in an undigested control DNA sample, optionally corrected for sequencing depth differences. Exemplary calculations are provided in the Examples section that follows. The normalized value can be used to calculate the levels of unmethylated DNA, by making a ratio between the normalized number of sequence reads starting or ending at a nucleotide within the restriction locus and an expected read count of the restriction locus.

In some embodiments, the level of unmethylated DNA is obtained by calculating a difference between the number of reads starting or ending at a nucleotide within the restriction locus and an expected number of reads starting or ending at a nucleotide within the restriction locus, and subsequently dividing the difference by an expected read count of the restriction locus.

In additional embodiments, the level of unmethylated DNA is calculated by determining a total fragment number, which is determined from the read count of the restriction locus and read count of sequence reads starting or ending at a nucleotide within the restriction locus. Exemplary calculations are provided in the Examples section that follows.

In some embodiments, the level of unmethylated DNA is expressed as percentage (%) of unmethylation, representing the percentage of DNA molecules that are unmethylated at the restriction locus out of the total number of DNA molecules containing the restriction locus in the sample.

Methylation level (or level of unmethylated DNA) may also be calculated for regions in the genome spanning a plurality of restriction loci (namely, genomic regions containing a plurality of restriction sites). A genomic region spanning a plurality of restriction loci may be a gene, an intergenic region, a promoter region, a part of a chromosome (e.g., a chromosomal arm), a whole chromosome, and more. Each possibility represents a separate embodiment of the present invention.

Detecting methylation changes

As used herein, "detecting methylation changes" refers to detecting whether a tested DNA sample contains methylation changes compared to one or more reference DNA samples, detecting whether a DNA sample is characterized by a different methylation profile at selected genomic loci compared to a reference methylation profile, and/or determining whether the methylation profile of a DNA sample is normal or contains methylation changes indicative of the presence of a disease. Each possibility represents a separate embodiment of the present invention. Detecting methylation changes also encompasses comparing methylation data obtained as disclosed herein between samples in order to identify genomic regions differentially methylated between the samples, which may be used as DNA methylation markers. For example, methylation data obtained as disclosed herein may be analyzed to identify genomic regions differentially methylated between different types of tissues, between cancer and non-cancer DNA, between different types of cancer, or between different stages of a certain type of cancer. In some embodiments, the methods disclosed herein provide genome-wide methylation analysis. In other embodiments, the methods disclosed herein provide target-specific methylation analysis. Computer software may be used in the analysis of the sequencing and methylation data.

The methods of the present invention may be applied for identifying and analyzing DNA methylation marker regions which may be used as pan-cancer diagnostic markers, namely, DNA methylation markers which are indicative of a group of cancer types. For example, in some embodiments, pan-cancer markers according to the present invention are indicative of a plurality of cancer types selected from lung cancer, colorectal cancer, liver cancer, breast cancer, pancreatic cancer, uterine cancer, ovarian cancer, head & neck cancer, gastric cancer, esophageal cancer, hematological cancers (e.g. lymphoma) and sarcoma. The methods may also be applied for identifying differential methylation between different types of cancer, for example, determining methylation profiles characteristic of different types of cancer, that can differentiate between different types of cancer. The methods disclosed herein are applicable to any type of cancer, including, but not limited to: lung cancer, bladder cancer, breast cancer, colorectal cancer, prostate cancer, gastric cancer, skin cancer (e.g. melanoma), cancer affecting the nervous system, bone cancer, ovarian cancer, liver cancer (e.g. hepatocellular carcinoma), hematologic malignancies, pancreatic cancer, kidney cancer, cervical cancer. Each type of cancer is a separate embodiment of the present invention. The methods of the present invention may also be applied to identify tissue-specific methylation markers. For example, to identify methylation markers specific for: lung, bladder, breast, colorectal, prostate, gastric, ovarian, pancreas, kidney, cervical tissue. Each type of tissue is a separate embodiment of the present invention. Such markers may be used, for example, to identify the tissue source of circulating cell-free DNA.

The methods of the present invention may also be applied for identifying a disease (e.g., a cancer) in a subject. "Identifying a disease" as used herein encompasses any one or more of screening for the disease, detecting the presence or absence of the disease, detecting recurrence of the disease, detecting susceptibility to the disease, detecting response to treatment, determining efficacy of treatment, determining stage (severity) of the disease, determining prognosis and early diagnosis of the disease in a subject. Each possibility represents a separate embodiment of the present invention.

"Assessing cancer " or "assessing the presence of cancer" or "assessing the presence or absence of cancer" as used herein refer to determining the likelihood that a subject has cancer. The terms encompass determining whether a subject should be subjected to confirmatory cancer testing to confirm (or rule out) the presence of cancer, such as confirmatory blood tests, urine tests, cytology, imaging, endoscopy and/or biopsy. The terms further encompass aiding the diagnosis of cancer in a subject. The terms further encompass quantifying cancer-related changes in cell-free DNA samples which are indicative for the presence of cancer. Assessing the presence of cancer according to the present invention includes one or more of screening for cancer, assessing recurrence of cancer, assessing susceptibility or risk to cancer, assessing and/or monitoring response to treatment, assessing efficacy of treatment, assessing severity (stage) of cancer and assessing prognosis of cancer in a subject. Each possibility represents a separate embodiment of the present invention. It is to be understood that a negative result in the assays disclosed herein is still considered an assessment for the presence of cancer according to the present invention.

The methods of the present invention may further include a step of determining a tumor fraction, or fractional concentration of tumor DNA. Tumor fraction is the proportion of tumor molecules in a cfDNA sample.

Determining a "methylation profile" (or "DNA methylation profile" or "methylation profile of a DNA sample") as disclosed herein refers to determining methylation values at one or more restriction loci, preferably at a plurality of restriction loci. In some embodiments, determining a methylation profile comprises determining levels of methylated and unmethylated DNA at one or more restriction loci, preferably at a plurality of restriction loci.

A "reference methylation profile" as disclosed herein refers to a methylation profile determined in DNA from a known source. A "reference DNA sample" is a DNA sample from a known source. In some embodiments, a reference methylation profile is a profile determined in a plurality of reference DNA samples. In addition, the methods of the present invention may be used for analyzing (e.g., measuring) methylation changes between DNA samples taken from a single subject at different time points, for example, taken at different stages of a disease, or taken before and after treatment of a disease. The methylation profile of the DNA sample taken at a first time point may be used as a reference for the methylation profile of a DNA sample taken at a second (later) time point.

A "reference methylation level" for a particular restriction locus or a particular genomic region spanning a plurality of restriction loci is the level of methylation measured for the particular restriction locus/genomic region in DNA from a known source. A "reference methylation value" for a particular restriction locus or a particular genomic region spanning a plurality of restriction loci is a numerical value representing the level of methylation of the particular restriction locus/genomic region in DNA from a known source.

A "reference level of unmethylated DNA" for a particular restriction locus or a particular genomic region spanning a plurality of restriction loci is the level of unmethylated DNA measured for the particular restriction locus/genomic region in DNA from a known source.

The reference methylation/unmethylation level/value may be a distribution of methylation/unmethylation levels/values determined for the particular restriction locus or the particular genomic region in a large set of DNA samples from a known source. In some embodiments, the reference methylation/unmethylation level/value may be a reference scale.

A reference scale for a particular restriction locus/genomic region may include methylation/unmethylation levels/values measured for this restriction locus in a plurality of DNA samples from the same reference source. For example, a reference scale of reference cancer patients or a reference scale of reference healthy individuals. Alternatively, a reference scale for a given restriction locus may include methylation/unmethylation levels/values from both healthy and diseased individuals, i.e., a single scale combining reference methylation values from both sources. Generally, when a single scale is used, the values are distributed such that the values from the healthy individuals are at one end of the scale, e.g., below a cutoff, while the values from the patients are at the other end of the scale, e.g., above the cutoff. In some embodiments, methylation/unmethylation levels/values calculated for a tested DNA sample from an unknown source may be compared against a reference scale of healthy and/or disease reference values, and a score may be assigned to the calculated methylation/unmethylation levels/values based on its relative position within the scale.

The terms "disease reference methylation" (for example: "cancer reference methylation"), "disease reference unmethylation" or "reference methylation (or unmethylation) in disease DNA" (for example: "reference methylation in cancer DNA") interchangeably refer to the methylation values and/or unmethylation values measured for a particular restriction locus or a particular genomic region in DNA samples of subjects with the disease for which the analysis is carried out, for example, subjects with a certain type of cancer. The disease reference methylation and/or unmethylation represents the methylation/unmethylation values in disease DNA, namely, DNA from samples of subjects with the disease. The disease reference methylation/unmethylation may be a single value or a plurality of values (e.g., distribution), as detailed above.

The term "disease DNA methylation profile" (e.g., "cancer DNA methylation profile") refers to methylation values and/or unmethylation values at a plurality of restriction loci, determined from samples (e.g., plasma samples) of subjects with the disease for which the analysis is carried out, for example, subjects with a certain type of cancer that is being analyzed.

The terms "healthy reference methylation", "normal reference methylation" or reference methylation in healthy/-normal DNA" interchangeably refer to the methylation values measured for a particular restriction locus/genomic region in DNA samples from normal individuals. Similarly, "healthy reference unmethylation", "normal reference unmethylation" or reference unmethylation in healthy/normal DNA" interchangeably refer to the unmethylation values measured for a particular restriction locus/genomic region in DNA samples from normal individuals. "Normal" or "healthy" is defined with respect to the particular disease for which the analysis is carried out. A "healthy" or "normal" individual is defined herein as an individual without detectable symptoms and/or pathological findings of the disease, as determined by conventional diagnostic methods. Healthy reference values may be a single value or a plurality of values (e.g., distribution), as detailed above.

The terms "healthy DNA methylation profile" or "normal DNA methylation profile") refer to methylation values and/or unmethylation values at a plurality of restriction loci, determined from DNA samples of normal individuals, as defined above.

In some embodiments, diagnostic methods disclosed herein comprise pre-determination of reference methylation and/or unmethylation from disease DNA. In some embodiments, diagnostic methods of the present invention comprise pre-determination of reference methylation and/or unmethylation from normal DNA as disclosed herein.

Tissue-specific methylation profile can also be characterized using the methods disclosed herein, in order to establish normal non-cancer DNA methylation profile of the tissue. Alternatively or additionally, tissue-specific methylation profile can be characterized in order to identify the tissue source of circulating cell-free DNA.

In some embodiments, detecting methylation changes according to the present invention comprises identifying the presence or absence of a certain disease in a subject, based on the methylation profile of a DNA sample from the subject.

In some embodiments, a method for identifying the cell source or tissue source of a DNA sample is provided (e.g., identifying what is the type of tissue from which the DNA is derived, and/or identifying whether the DNA is derived from normal or diseased cells/tissue).

A person of skill in the art would appreciate that the comparison of DNA methylation values and/or unmethylation values calculated for a tested sample to one or more corresponding reference values may be performed in a number of ways, using various statistical means.

In some embodiments, comparing a test methylation/unmethylation value calculated for a particular restriction locus/genomic region to a reference value comprises comparing the test value against a single reference value. The single reference value may correspond to a mean value obtained for reference methylation/unmethylation value from a large population of healthy subjects or subjects with the disease for which the analysis is carried out. In other embodiments, comparing a test value to a reference value comprises comparing the test value against a distribution, or a scale, of a plurality of reference values. Known statistical means may be employed in order to determine whether the value calculated for a tested sample corresponds to disease reference value or to normal reference value.

In some embodiments, disease diagnosis according to the present invention is based on analyzing whether a methylation value and/or unmethylation value of a tested DNA sample is a disease value, namely, indicative of a disease in question. In some embodiments, the method comprises comparing a calculated value to its corresponding healthy reference value to obtain a score reflecting the likelihood that the calculated value is a disease value. In some embodiments, methods disclosed herein comprise comparing a calculated value to its corresponding disease reference value to obtain a score reflecting the likelihood that the calculated value is a disease value. In some embodiments, the higher the score, the higher the likelihood that the calculated value is a disease value. In some embodiments, the score is based on the relative position of the calculated value within the distribution of disease reference values.

In some embodiments, the methods disclosed herein comprise comparing a plurality of values calculated for a plurality of restriction loci to their corresponding healthy and/or disease references values. In some embodiments, a pattern of values is analyzed using statistical means and computerized algorithm to determine if it represents a pattern of a disease in question or a normal, healthy pattern. Exemplary algorithms include, but are not limited to, machine learning and pattern recognition algorithms.

In some exemplary embodiments, a value calculated for a tested sample may be compared against a scale of reference values generated from a large set of cancer samples, non-cancer samples, or both. The scale may exhibit a threshold value, also termed hereinafter 'cutoff' or 'pre-defined threshold', above which are reference values corresponding to the cancer and below are reference values corresponding to healthy individuals, or the other way around. In some embodiments, the lower values, at the bottom of the scale and/or below a cutoff, may be from samples of normal individuals (healthy, i.e., not afflicted with the cancer in question), while the higher values at the top of the scale and/or above a predetermined cutoff, may be from the cancer patients. For diagnosis based on analysis of a plurality of restriction loci, the value calculated for each locus may be given a score based on its relative position within the scale, and the individual scores (for each locus) are combined to give a single score. In some embodiments, the individual scores may be summed to give a single score. In other embodiments, the individual scores may be averaged to give a single score. In some embodiments, the single score may be used for determining whether the subject is having the cancer in question, where a score above a pre-defined threshold is indicative of the cancer.

In additional exemplary embodiments, for diagnosis based on analysis of a plurality of restriction loci, for each calculated value the probability that it represents cancer DNA may be determined, based on comparison to a corresponding cancer reference value and/or normal reference value. A score may be allocated for each locus, and subsequently the individual scores calculated for each locus are combined (e.g., summed or averaged) to give a combined score. The combined score may be used for determining whether the subject is positive or negative for the cancer, wherein a combined score above a predefined threshold is indicative of the cancer. Thus, in some embodiments, a threshold, or cutoff, score is determined, above (or below) which the subject is identified as positive for the disease in question, e.g., the type of cancer in question. The threshold score differentiates the population of healthy subjects from the population of non-healthy subjects.

In some embodiments, diagnostic methods according to the present invention comprises providing a threshold score.

Statistical significance is often determined by comparing two or more populations, and determining a confidence interval (CI) and/or a p value. In some embodiments, the statistically significant values refer to confidence intervals (CI) of about 90%, 95%, 97.5%, 98%, 99%, 99.5%, 99.9% and 99.99%, while preferred p values are less than about 0.1, 0.05, 0.025, 0.02, 0.01, 0.005, 0.001 or less than 0.0001. Each possibility represents a separate embodiment of the present invention. According to some embodiments, the p value of the threshold score is at most 0.05.

In some embodiments, the diagnostic sensitivity of the diagnostic methods disclosed herein is at least 75%. In some embodiments, the diagnostic sensitivity is at least 80%. In some embodiments, the diagnostic sensitivity is least 85%. In some embodiments, the diagnostic sensitivity of the methods is at least 90%.

In some embodiments, the "diagnostic sensitivity" of a diagnostic assay as used herein refers to the percentage of diseased individuals who test positive (percent of "true positives"). Accordingly, diseased individuals not detected by the assay are "false negatives". Subjects who are not diseased and who test negative in the assay are termed "true negatives." The "specificity" of the diagnostic assay is one (1) minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

In some embodiments, the diagnostic specificity of the diagnostic methods as disclosed herein may be at least about 65%. In some embodiments, the diagnostic specificity of the methods may be at least about 70%. In some embodiments, the diagnostic specificity of the methods may be at least about 75%. In some embodiments, the diagnostic specificity of the methods may be at least about 80%.

In some embodiments, diagnostic methods according to the present invention comprise preparing a report (in paper or electronic form) based on the methylation profile. The report may be communicated to the subject and/or to a healthcare provider of the subject.

In some embodiments, diagnostic methods according to the present invention comprise referring the subject to follow-up testing and screening.

<u>Additional genetic and epigenetic characterization</u>

In addition to DNA methylation/unmethylation values, it is possible to obtain from the same sequencing data disclosed herein information on DNA mutations, copy number changes, and nucleosome positioning for cell-free DNA. Generally, cell-free DNA circulates in fragments ranging between 120-220 bp. This pattern agrees with the length of DNA wrapped around a single nucleosome, plus a short stretch of ~ 20 bp (linker DNA) bound to a histone. As nucleosome positioning varies between different tissues, and in malignant cells, the pattern of fragmentation has been shown to aid in determining the predominant cell-type of origin contributing to the cfDNA pool.

Advantageously, determination of DNA methylation profile and determination of at least one additional genetic or epigenetic characteristic as disclosed herein may be carried out based on the same sequencing data.

In some embodiments, a sequencing-based assay as disclosed herein combines detection of methylation changes with mutation detection and analysis of additional epigenetic characteristics, all in one single assay. The assay advantageously allows combined analysis of small amounts of DNA in a single assay.

The combined analysis of methylation and additional genetic and epigenetic characteristics is useful in enhancing detection of cancer (or any other condition/tissue source).

In some exemplary embodiments, a method for detecting the presence or absence of a cancer in a subject comprises:

(A) profiling methylation of the DNA sample as disclosed herein, to detect the presence or absence of hypermethylation at one or more cancer-associated genomic region; and
(B) one or more of:

determining the presence or absence of one or more cancer-associated mutation (e.g., cancer-associated mutation in oncogenes/tumor suppressors);

determining the presence or absence of cancer-associated copy number variation; and

determining the presence or absence of cancer-associated nucleosomal positioning,

wherein (A) and (B) are carried out using the same sequencing data, and

wherein determining the presence of hypermethylation at one or more cancer-associated genomic region and at least one of: one or more cancer-associated mutation, cancer-associated copy number variation and cancer-associated nucleosomal positioning is indicative of the presence of cancer in the subject.

The non-methylation cancer-associated changes may be combined with methylation information in a dependent or independent manner, depending on whether or not the cancer-associated changes are found on the same DNA fragment, where changes that are found on the same fragment provide a stronger indication for the presence of cancer.

In some embodiments, there is provided a method for profiling genetic and epigenetic characteristics of a DNA sample, the method comprising: profiling methylation of the DNA sample as disclosed herein; and determining at least one additional genetic or epigenetic characteristic of the DNA sample, wherein the at least one additional genetic or epigenetic characteristic is selected from DNA mutation, copy number variation and nucleosome positioning, wherein profiling the methylation and determining the at least one additional genetic or epigenetic characteristic are carried out using the same sequencing data, thereby profiling genetic and epigenetic characteristics of the DNA sample.

In some embodiments, there is provided a method for detecting the presence or absence of a disease in a subject, the method comprising: profiling methylation of the DNA sample as disclosed herein; and determining at least one additional genetic or epigenetic characteristic of the DNA sample, wherein the at least one additional genetic or epigenetic characteristic is selected from DNA mutation, copy number variation and nucleosome positioning, wherein profiling the methylation and determining the at least one additional genetic or epigenetic characteristic are carried out using the same sequencing data, to obtain genetic and epigenetic characteristics of the DNA sample; and comparing the genetic and epigenetic characteristics of the DNA sample to one or more reference genetic and epigenetic characteristics, and determining the presence or absence of the disease based on the comparison. In some embodiments, the disease is a cancer.

Systems and kits

In some embodiments, there is provided herein systems for detecting methylation changes in a DNA sample. In some embodiments, there is provided herein systems and methods for detecting genetic and epigenetic changes in a DNA sample. In additional embodiments, there is provided herein kits for detecting methylation changes in a DNA sample. In additional embodiments, there is provided herein kits for detecting genetic and epigenetic changes in a DNA sample.

Systems according to the present invention comprise computer processor(s) for performing the assays and/or processing the results e.g., for performing the calculations. In some embodiments, computer-implemented methods are provided herein.

In some embodiments, the systems and kits are for profiling methylation of DNA samples according to the methods disclosed herein. In some embodiments, the systems and kits are for profiling genetic and epigenetic characteristics of DNA samples according to the methods disclosed herein. In additional embodiments, the systems and kits are for detecting methylation changes in a DNA sample according to the methods disclosed herein. In additional embodiments, the systems and kits are for detecting genetic and epigenetic changes in a DNA sample according to the methods disclosed herein.

In some embodiments, a system according to the present invention comprises:

a DNA sample;

at least one methylation-sensitive restriction endonuclease and/or at least one methylation-dependent restriction endonuclease for digesting the DNA sample;

components for preparing a sequencing library comprising a plurality of restriction-endonuclease-generated DNA fragments;

a high-throughput sequencer for sequencing the sequencing library and produce sequence reads; and

computer software stored on a non-transitory computer readable medium, the computer software directs a computer processor to profile genetic and epigenetic characteristics of the DNA sample based on a plurality of sequence reads according to the methods disclosed herein. In some embodiments, the computer software directs a computer processor to profile methylation of the DNA sample based on a plurality of sequence reads according to the methods disclosed herein.

In some embodiments, the computer software stored on a non-transitory computer readable medium directs the computer processor to determine genetic and epigenetic changes in the DNA sample based on a plurality of sequence reads according to the methods disclosed herein. In some embodiments, the computer software stored on a non-transitory computer readable medium directs the computer processor to determine methylation changes in the DNA sample based on a plurality of sequence reads according to the methods disclosed herein.

As used herein, "components" for preparing a sequencing library encompass biochemical components (e.g., enzymes, nucleotides), chemical components (e.g., buffers), and technical components (e.g., equipment such as tubes, vials, pipettes, and the like).

In some embodiments, a kit or a system according to the present invention comprises components needed for DNA digestion in addition to the restriction enzyme(s), such as one or more buffers.

In some embodiments, there is provided herein a system for profiling genetic and epigenetic characteristics of a cell-free DNA sample, the system comprising a cell-free DNA sample and a computer software stored on a non-transitory computer readable medium comprising instructions that when executed configure or direct a computer processor to perform the following steps:

(i) receiving sequencing data of a library of DNA molecules obtained following digestion of the cell-free DNA sample with at least one methylation-sensitive restriction endonuclease and preparation of a sequencing library comprising ligating sequencing adapters to DNA molecules in the restriction endonuclease-treated DNA, wherein each adapter is capable of ligation to both the digested and undigested DNA molecules; and
(ii) determining from the sequencing data a methylation value for at least one restriction locus and optionally at least one additional genetic or epigenetic characteristic of the cell-free DNA sample selected from DNA mutation, copy number variation and nucleosome positioning,

wherein an amount of cell-free DNA comprising 3000 haploid equivalents is sufficient for the method, wherein the cell-free DNA sample was not subjected to amplification prior to library preparation, and wherein determining the methylation value and the at least one additional genetic or epigenetic characteristic of the cell-free DNA sample is carried out based on the same sequencing data.

In some embodiments, there is provided herein a system for profiling methylation of a DNA sample, the system comprising a computer software stored on a non-transitory computer readable medium comprising instructions that when executed configure or direct a computer processor to perform the following steps:

(i) receiving sequence reads of a library of DNA molecules obtained following digestion of a DNA sample with at least one methylation-sensitive restriction endonuclease;
(ii) selecting at least one restriction locus and determining the number of sequence reads covering a predefined genomic region of at least 50 bps in length that contains said restriction locus; and
(iii) calculating a methylation value for the at least one restriction locus based on the read count determined in step (ii) and a reference read count.

In some embodiments, there is provided herein a system for profiling methylation of a DNA sample, the system comprising a computer software stored on a non-transitory computer readable medium comprising instructions that when executed configure or direct a computer processor to perform the following steps:

(i) receiving sequence reads of a library of DNA fragments obtained following digestion of a DNA sample with at least one methylation-sensitive restriction endonuclease;
(ii) mapping a plurality of the sequence reads against a reference genome to produce mapped sequence reads, and selecting at least one restriction locus within the reference genome;
(iii) determining from the mapped sequence reads a read count of the at least one restriction locus, the read count representing the number of DNA molecules in the DNA sample in which said at least one restriction locus was methylated and therefore remained intact;
(iv) determining from the mapped sequence reads a read count of sequence reads starting or ending at a nucleotide within the at least one restriction locus, the read count representing the number of DNA molecules in the DNA sample in which said at least one restriction locus was unmethylated and therefore cut by the restriction endonuclease; and
(v) calculating a level of methylated DNA at the at least one restriction locus based on the read count of the at least one restriction locus determined in step (iii) and a level of unmethylated DNA at the at least one restriction locus based on the read count of sequence reads starting or ending at a nucleotide within the at least one restriction locus determined in step (iv).

In some embodiments, the computer software further directs the computer processor to compare a genetic and epigenetic profile of a tested DNA sample to one or more reference genetic and epigenetic profiles, and based on the comparison, output whether the DNA sample is a normal DNA sample or a disease DNA sample.

In some embodiments, the computer software further directs the computer processor to compare a methylation profile of a tested DNA sample to one or more reference methylation profiles, and based on the comparison, output whether the DNA sample is a normal DNA sample or a disease DNA sample.

In some embodiments, a computer software according to the present invention receives as an input raw data of a high-throughput sequencing run. In some embodiments, the computer software directs a computer processor to analyze the sequencing data to determine a genetic and epigenetic profile as disclosed herein. In some embodiments, the computer software directs a computer processor to analyze the sequencing data to determine DNA methylation values and/or DNA unmethylation values as disclosed herein.

The computer software includes processor-executable instructions that are stored on a non-transitory computer readable medium. The computer software may also include stored data. The computer readable medium is a tangible computer readable medium, such as a compact disc (CD), magnetic storage, optical storage, random access memory (RAM), read only memory (ROM), or any other tangible medium.

It is understood that the computer-related methods, steps, processes described herein are implemented using software stored on non-volatile or non-transitory computer readable instructions that when executed configure or direct a computer processor or computer to perform the instructions.

Each of the system, server, computing device, and computer described in this application can be implemented on one or more computer systems and be configured to communicate over a network. They all may also be implemented on one single computer system. In one embodiment, the computer system includes a bus or other communication mechanism for communicating information, and a hardware processor coupled with bus for processing information.

The computer system also includes a main memory, such as a random-access memory (RAM) or other dynamic storage device, coupled to bus for storing information and instructions to be executed by processor. Main memory also may be used for storing temporary variables or other intermediate information during execution of instructions to be executed by processor. Such instructions, when stored in non-transitory storage media accessible to processor, render computer system into a special-purpose machine that is customized to perform the operations specified in the instructions.

The computer system further includes a read only memory (ROM) or other static storage device coupled to bus for storing static information and instructions for processor. A storage device, such as a magnetic disk or optical disk, is provided and coupled to bus for storing information and instructions.

The computer system may be coupled via bus to a display, for displaying information to a computer user.

An input device, including alphanumeric and other keys, is coupled to bus for communicating information and command selections to processor. Another type of user input device is cursor control, such as a mouse, a trackball, or cursor direction keys for communicating direction information and command selections to processor and for controlling cursor movement on display.

According to one embodiment, the techniques herein are performed by the computer system in response to the processor executing one or more sequences of one or more instructions contained in main memory. Such instructions may be read into main memory from another storage medium, such as storage device. Execution of the sequences of instructions contained in main memory causes the processor to perform the process steps described herein. In alternative embodiments, hard-wired circuitry may be used in place of or in combination with software instructions.

The term storage media as used herein refers to any non-transitory media that store data and/or instructions that cause a machine to operation in a specific fashion. Common forms of storage media include, for example, a floppy disk, a flexible disk, hard disk, solid state drive, magnetic tape, or any other magnetic data storage medium, a CD-ROM, any other optical data storage medium, any physical medium with patterns of holes, a RAM, a PROM, and EPROM, a FLASH-EPROM, NVRAM, any other memory chip or cartridge.

Storage media is distinct from but may be used in conjunction with transmission media. Transmission media participates in transferring information between storage media. For example, transmission media includes coaxial cables, copper wire and fiber optics, including the wires that comprise bus.

The following examples are presented in order to more fully illustrate certain embodiments of the invention. They should in no way, however, be construed as limiting the broad scope of the invention. One skilled in the art can readily devise many variations and modifications of the principles disclosed herein without departing from the scope of the invention.

**EXAMPLES**

**Example 1 -Methylation-sensitive DNA digestion followed by next-generation sequencing (NGS) vs. bisulfite treatment + NGS**

In the following example sequencing data obtained for cell-free DNA samples subjected to methylation-sensitive enzymatic digestion followed by NGS were compared to the sequencing data obtained following bisulfite conversion and NGS. First, sequencing data of pooled plasma DNA samples were examined. Next, sequencing data of individual plasma DNA samples, each containing ~10-200 ng DNA (corresponding to ~3,000-60,000 haploid equivalents of DNA), were examined.

## A. Pooled plasma samples of healthy individuals

DNA was extracted from plasma samples of 56-60 healthy control subjects using QIAamp® Circulating Nucleic Acid Kit (QIAGEN, Hilden, Germany) and pooled. An aliquot of 500ng was kept as an untreated control DNA, an aliquot of 1700ng was subjected to bisulfite conversion using EZ DNA Methylation-Gold™ Kit (Zymo research), and the remaining DNA (770ng) was subjected to digestion with the methylation-sensitive restriction enzymes HinP1I and AciI. Methylation-sensitive digestion was carried out by incubating the sample with 10 units of HinP1I and 5 units of AciI for 2h at 37°C followed by inactivation for 20 min at 65°C.

Next, a sequencing library was prepared from each sample (enzyme-treated, bisulfite-treated and untreated control sample) by using NEBNext Ultra DNA Library Prep Kit for the enzyme-treated and untreated control samples, and ACCEL-NGS® METHYL-SEQ DNA LIBRARY kit (swift) for the bisulfite-treated sample. The sequencing library was prepared while preserving the information at the ends of the DNA molecules, by adding Illumina platform sequencing adapters using enzymatic ligation. The libraries were subjected to whole-genome next generation sequencing using Illumina NovaSeq 6000 sequencing platform with S4 flow cell. The sequence reads from each sample were mapped against the complete human genome (hg18 genomic build).

Sequencing metrics

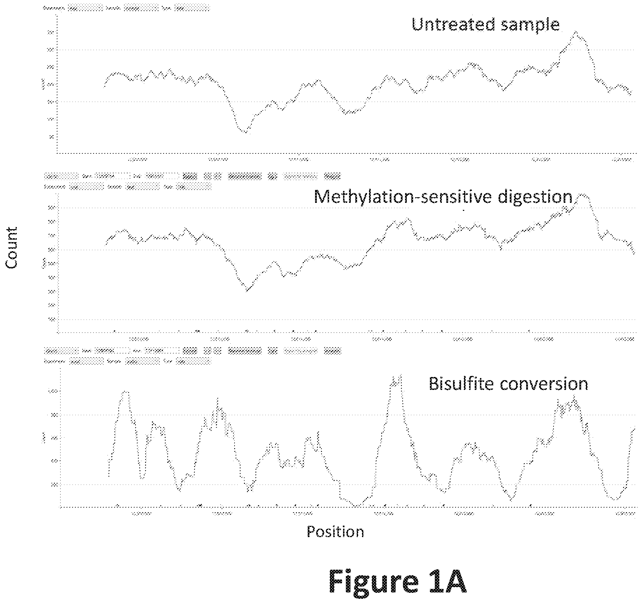

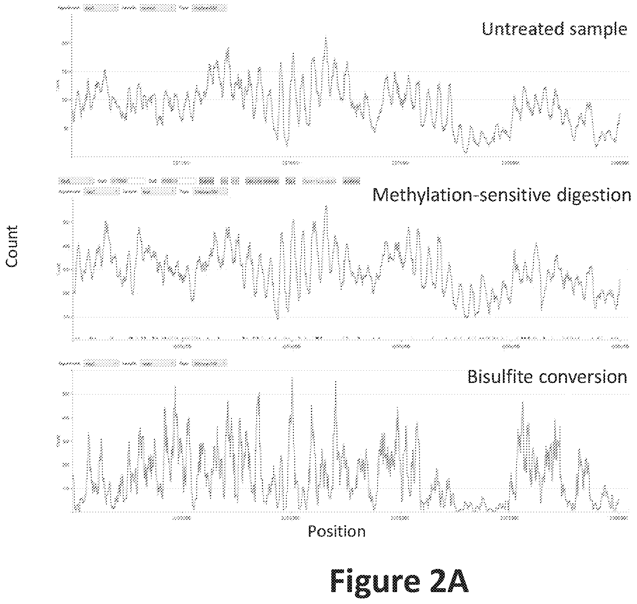

**Table 1** and **Figures 1A-B, 2A-B** summarize sequencing metrics, copy number integrity data and nucleosome positioning integrity data obtained for the pooled plasma DNA samples. For the copy number integrity analysis, the number of hits at each genomic position that is situated > 100bp from a restriction locus in the methylation sensitive-digested aliquot was compared to the corresponding number of hits obtained from the untreated aliquot. The same analysis was also performed on the bisulfite-treated aliquot (compared to the untreated aliquot). A Pearson correlation was calculated for all data points in each experimental setup (methylation sensitive-digested DNA and bisulfite-treated). The Pearson correlation yields a number between -1 and 1, in which a number closer to 1 represents a better correlation.

For the nucleosomal positioning integrity analysis, the same type of procedure was followed as for the copy number analysis, except that "hits span 100" (= number of reads that start > 50bp upstream and end > 50 bp downstream of an analyzed genomic position) was used instead of hits.

Table 1 - Sequencing metrics of pooled plasma DNA samples

|  | Untreated | Methyl.-sensitive enzymes + NGS | Bisulfite + NGS |
|---|---|---|---|
| **Number of reads** | 18,323,363,820 | 18,271,591,166 | 18,829,580,944 |
| **Mapping rate** | 98.20% | 98.38% | 81.2% |
| **Unique mapping rate** | 91.91% | 92.26% | 80.30% |
| **Total number of uniquely mapped reads** | 16,841,387,127 | 16,858,481,551 | 15,120,724,256 |

As can be seem from the data, methylation-sensitive digestion resulted in a mapping rate and a unique mapping rate which were substantially the same as those obtained for the untreated control sample, reaching over 92% unique mapping rate. In contrast, the bisulfite-treated sample showed a significant loss of information, with a unique mapping rate of only about 80%.

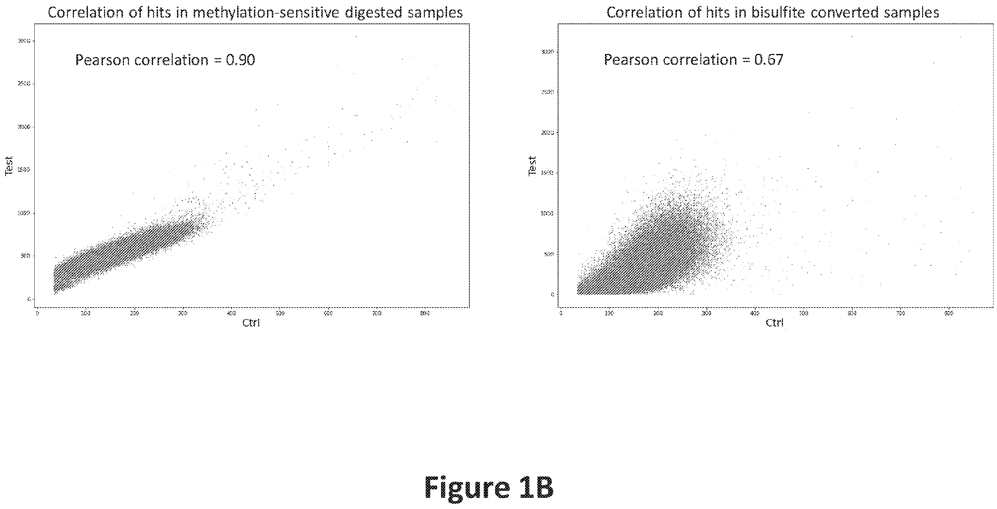

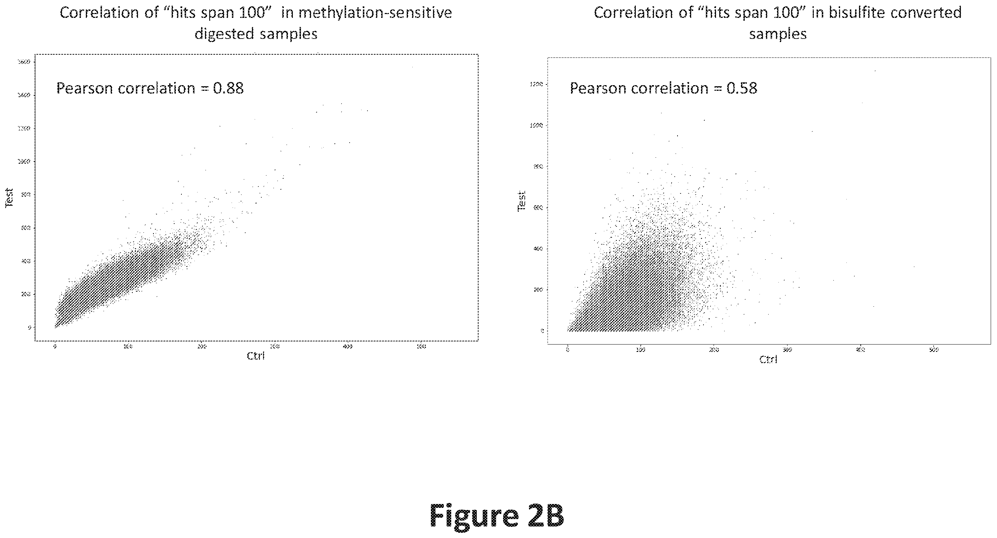

The loss of information in the bisulfite-treated sample was further demonstrated by copy number and nucleosome positioning integrity data: as can be seen in **Figure 1A** and **Figure 2A,** similar patterns of copy number and nucleosome positioning were observed for the methylation-sensitive digested sample and the untreated sample. The patterns were not maintained in the bisulfite-treated sample. Pearson correlation analysis showed a correlation of 0.9 in copy number and 0.88 in nucleosome positioning between the methylation-sensitive digested sample and the untreated control sample. In contrast, correlations of 0.67 (copy number) and 0.58 (nucleosome positioning) were obtained between the bisulfite-treated sample and the untreated control sample (**Figures 1B, 2B**).

## B. Plasma samples of lung-cancer patients

Section A above provides results using pooled plasma samples, containing relatively high amounts of DNA. It was of interest to check differences between methylation-sensitive digestion and bisulfite conversion using individual plasma samples, which contain much lower amounts of DNA for analysis.

To this end, DNA was extracted from plasma samples of treatment-naive non-small cell lung cancer (NSCLC) patients. The DNA was extracted as described in section A above and subjected to bisulfite conversion or to digestion with the methylation-sensitive restriction enzymes HinPII and AciI. The amount of DNA extracted from the plasma samples ranged from ~10-200 ng DNA per sample (corresponding to ~3,000-60,000 haploid equivalents of DNA). Following enzymatic digestion or bisulfite conversion the samples were subjected to library preparation and sequencing as described above.

Exemplary results of two patients, identified as BMD LNG165 (26 ng of cell-free DNA) and BMD LNG166 (94 ng of cell-free DNA) are provided below.

Sequencing metrics

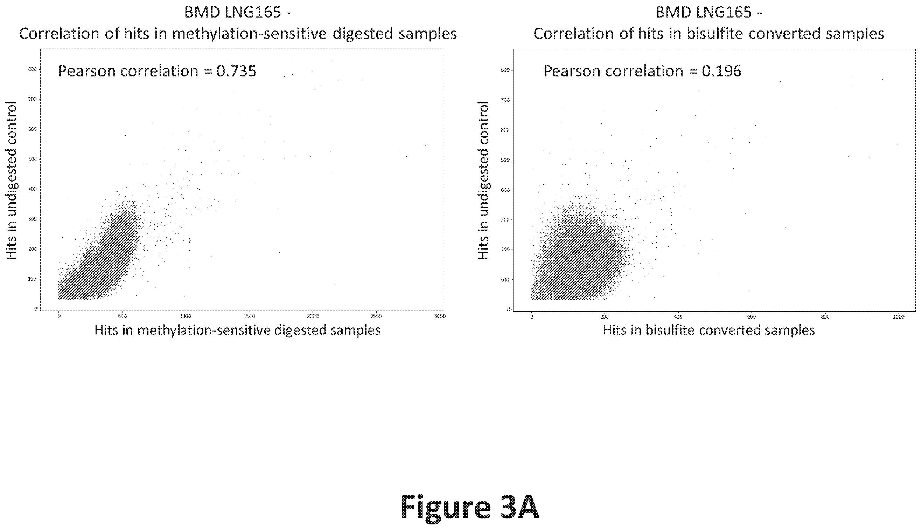

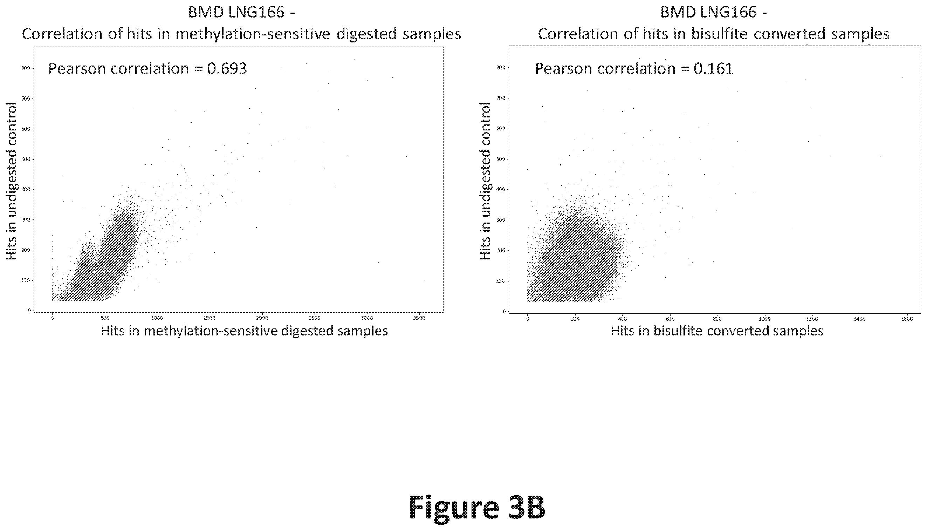

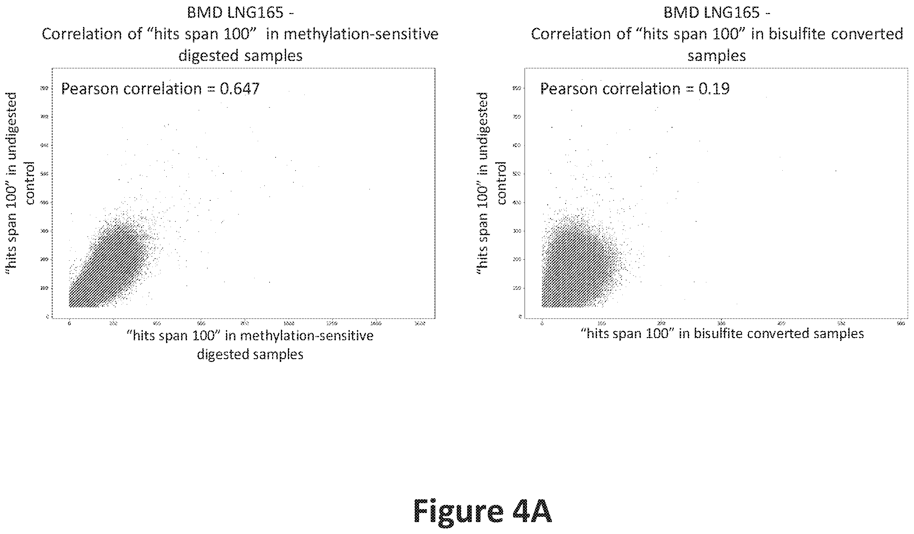

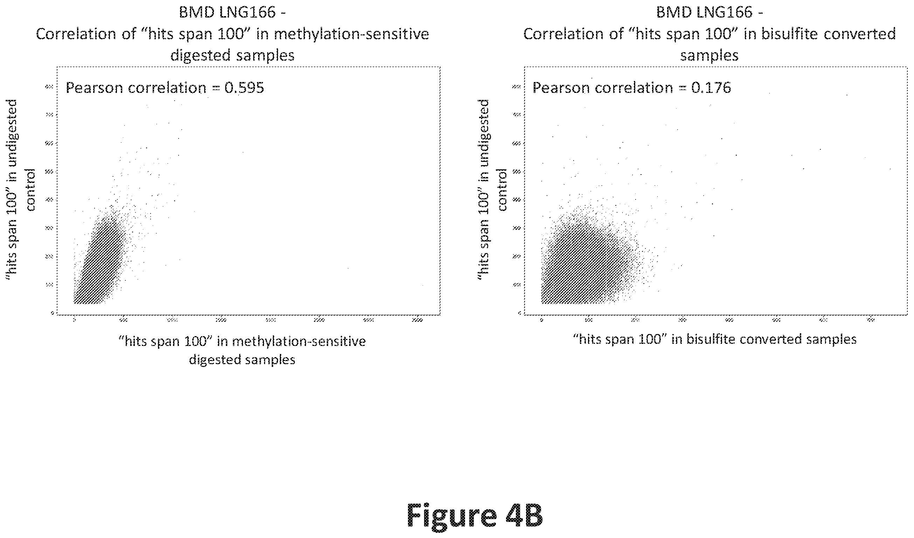

**Tables 2-3** and **Figures 3A-3B, 4A-4B** summarize sequencing metrics, copy number integrity data and nucleosome positioning integrity data obtained for each plasma DNA sample.

Table 2 - Sequencing metrics patient BMD LNG165

|  | **Methyl-sensitive enzymes + NGS** | **Bisulfite + NGS** |
|---|---|---|
| **Number of reads** | 13,988,252,662 | 6,206,319,156 |
| **Mapping rate** | 96.34% | 78.38% |
| **Unique mapping rate** | 89.64% | 77.29% |
| **Total number of uniquely mapped reads** | 12,540,361,228 | 4,797,333,458 |

Table 3 - Sequencing metrics patient BMD LNG166

|  | **Methyl-sensitive enzymes + NGS** | **Bisulfite + NGS** |
|---|---|---|
| **Number of reads** | 18,649,633,086 | 9,492,198,638 |
| **Mapping rate** | 97.87% | 79.24% |
| **Unique mapping rate** | 91.47% | 78.20% |
| **Total number of uniquely mapped reads** | 17,059,522,795 | 7,423,448,896 |

The results show that for low amounts of DNA, the number of reads that are obtained is significantly reduced when using bisulfite treatment versus methylation-sensitive digestion: the number of reads, and importantly, the number of uniquely mapped reads obtained for bisulfite-treated DNA was less than half the amount obtained for methylation-sensitive digested DNA. In addition, methylation-sensitive digested DNA showed a unique mapping rate of approximately 90%, whereas the unique mapping rate of bisulfite-treated DNA was only 77-78%.

The significant loss of information in the bisulfite-treated sample was further demonstrated by copy number and nucleosome positioning integrity data: Pearson correlation analysis showed correlations of 0.735 and 0.693 in copy number, and 0.647 and 0.595 in nucleosome positioning, between methylation-sensitive digested DNA and the untreated control sample. In contrast, correlations of 0.196 and 0.161 (copy number), and 0.19 and 0.176 (nucleosome positioning) were obtained between bisulfite-treated DNA and the untreated control sample (**Figures 3A-B, 4A-B**). Bisulfite sequencing practically lost all copy number and nucleosome positioning information when low amounts of DNA were used for the assay.

CG coverage

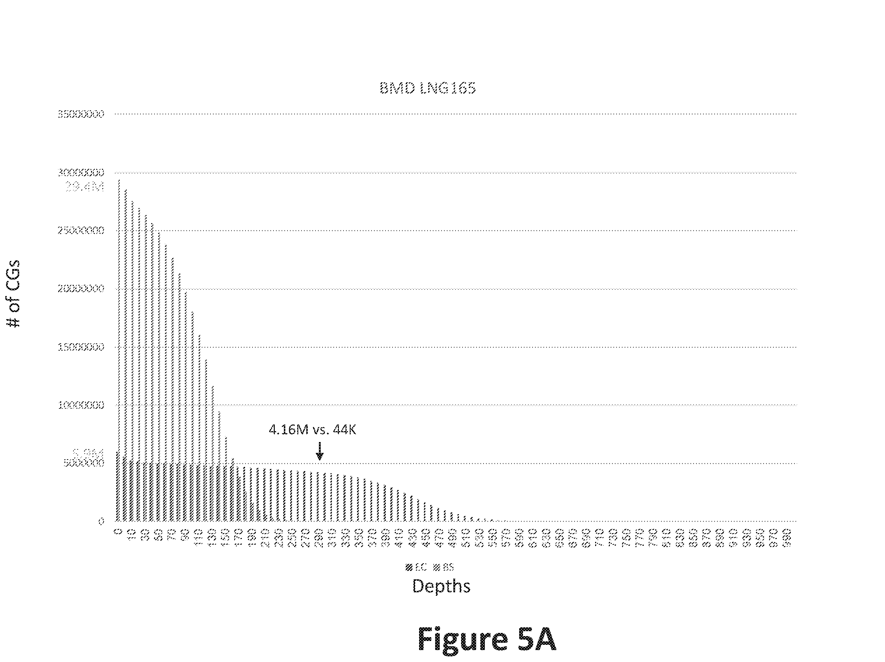

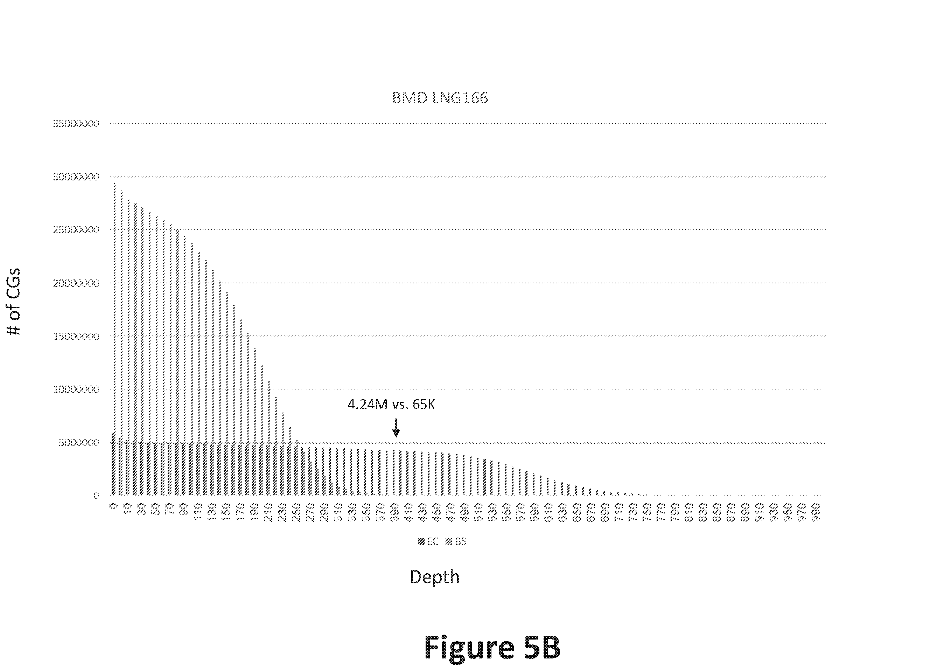

**Figures 5A-5B** show distribution of CG depths in bisulfite-treated DNA and methylation-sensitive digested DNA. More particularly, the graphs show the number of CG sites in the genome that were covered at each depth by each method. Figure 5A shows the data obtained for the sample from patient BMD LNG165. Figure 5B shows the data obtained for the sample from patient BMD LNG166.

Genome-wide methylation analysis using methylation-sensitive enzymatic digestion is limited to CGs located within recognition site(s) of the enzyme(s) used in the assay, while bisulfite sequencing in principle covers all CG sites in the genome. The ability to investigate only a fraction of the CG sites in the genome has been considered one of the main limitations of restriction enzyme-based methylation analysis. However, the data presented herein show that while bisulfite provides broader CG coverage at the lower end of depths compared to methylation-sensitive digestion, a continuous and sharp decrease is seen in the number of CGs that are covered in bisulfite-treated DNA as the depth increases. In contrast, methylation-sensitive digestion shows substantially constant coverage even at depths over 250-300. At high depths, methylation-sensitive digestion provides significantly better CG coverage compared to bisulfite.

For example, in the DNA sample from patient BMD LNG165 (**Figure 5A**), methylation-sensitive digestion covered more genomic CGs than bisulfite at depths above 165. At a depth of 300, the methylation-sensitive digested sample covered 4.16M CGs, compared to only 44K CG sites covered in the bisulfite-treated sample. In the DNA sample from patient BMD LNG166 (**Figure 5B**), methylation-sensitive digestion covered more genomic CGs than bisulfite at depths above 255. At a depth of 400, the methylation-sensitive digested sample covered 4.24M CGs, compared to only 65K CG sites covered in the bisulfite-treated sample.

Methylation-sensitive digestion therefore provides coverage of millions of CGs at very high depths, enabling the detection of rare methylation signals, for example, methylated DNA molecules from a tumor in the plasma at an early stage of the tumor, which may be present in the plasma at very low amounts - 1% or even less. The data show that at depths required for identification of rare signals, bisulfite does not provide sufficient coverage, and such rare signals are likely to be missed when using bisulfite sequencing on low amounts of DNA.

Detection of methylation changes

A set of low background hypermethylated marker loci was compiled, which show hypermethylation in tumor vs. normal tissue and are characterized by low background methylation in plasma of healthy individuals. Methylation levels were determined as described in Example 2 below. This set of marker loci was compiled based on samples from the two lung cancer patients (BMD LNG165 and patient BMD LNG166) and a pooled plasma sample of healthy individuals, and included low background hypermethylated loci that were observed using both methods of detection, namely, methylation-sensitive digestion+NGS and bisulfite conversion+NGS. In addition, a set of isomethylated marker loci, namely, loci which do not show different methylation levels between tumor and normal tissue, was compiled.

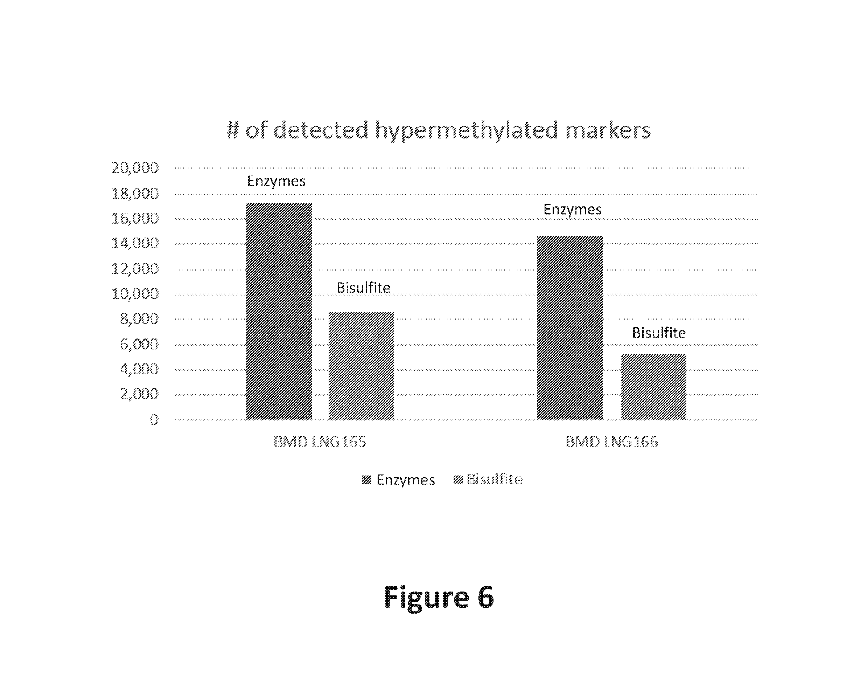

Next, plasma DNA from each patient was analyzed using methylation-sensitive digestion+NGS or bisulfite conversion+NGS in order to determine methylation levels of the low background hypermethylated marker loci in the patients' plasma. A threshold methylation level was set, above which a marker locus was considered as "detected". The threshold was determined based on the set of isomethylated marker loci in order to obtain detection specificity of 95%. The number of marker loci that crossed the threshold (namely, detected) in the methylation-sensitive digested DNA and in the bisulfite converted DNA from each patient was compared. The results are summarized in **Figure 6.** Methylation analysis using methylation-sensitive digestion+NGS detected significantly more methylation changes in the plasma compared to bisulfite +NGS in both samples.

Mutation detection

Tumor mutations were defined as genotypes found in the tumor DNA that are different from the most prevalent genotype in the corresponding normal tissue from the same patient. The fraction of reads with mutated genotypes in the tumor DNA represented the tumor mutational level, and the fraction of reads with the same mutated genotypes in the plasma of the patient represented the plasma mutation level. For each sample, the average tumor and plasma mutation levels were calculated across all mutations and a tumor mutational burden was calculated (i.e., average plasma mutation level / average tumor mutation level). The tumor mutational burden represents the fraction of tumor DNA in the plasma of the patient. In order to control for sequencing noise, the tumor mutational burden of patient A was compared to a control tumor mutational burden, calculated from the tumor mutations of patient B (i.e., the average mutation level of the tumor mutations of patient B in the plasma of patient A / the tumor mutation level of patient A).

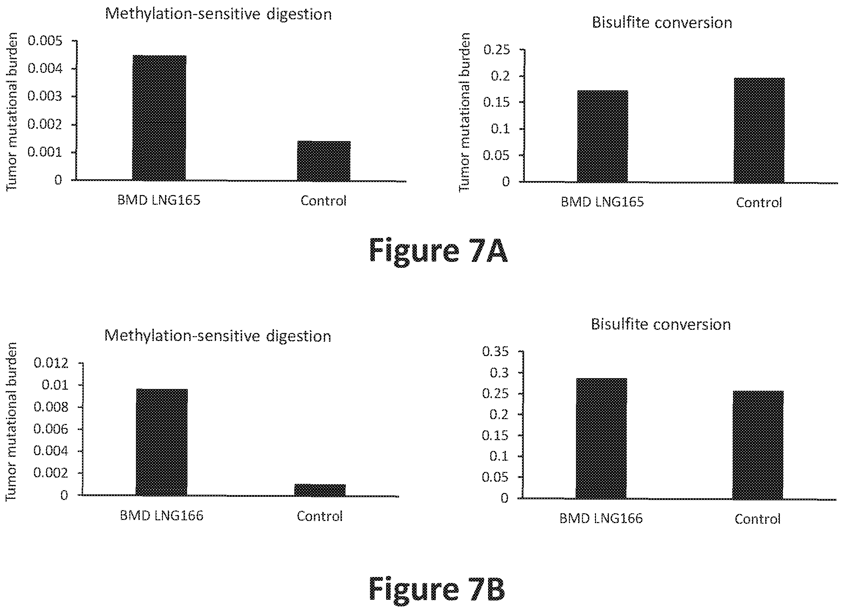

The results are summarized in **Figures 7A-7B.** Tumor mutations were detected in plasma by methylation-sensitive digestion + NGS at levels clearly above the sequencing noise, whereas in bisulfite + NGS mutations were indistinguishable from the high sequencing noise.

**Example 2** - **Genetic and epigenetic profiling of tumor and plasma DNA of lung cancer patients**

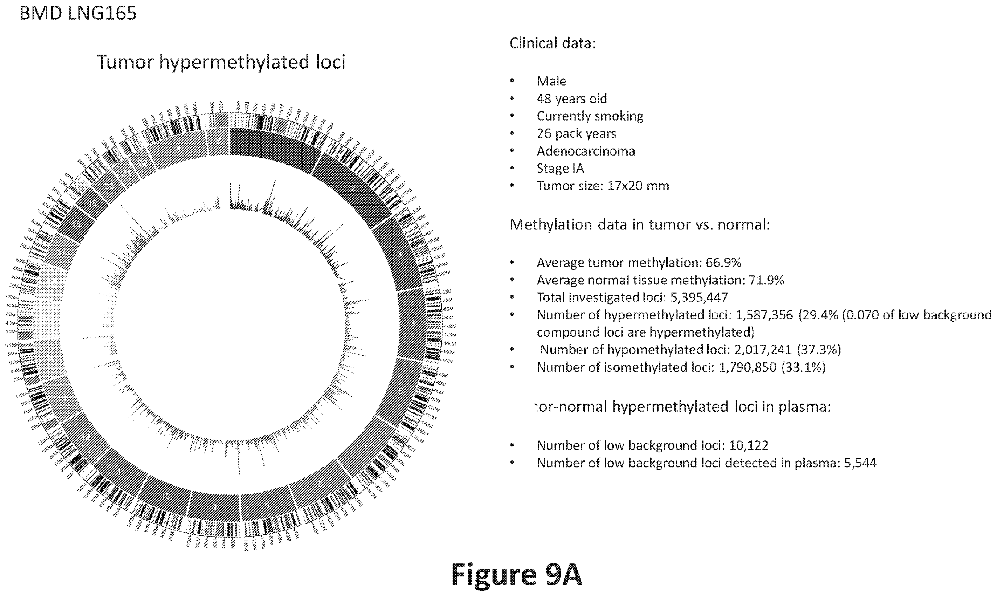

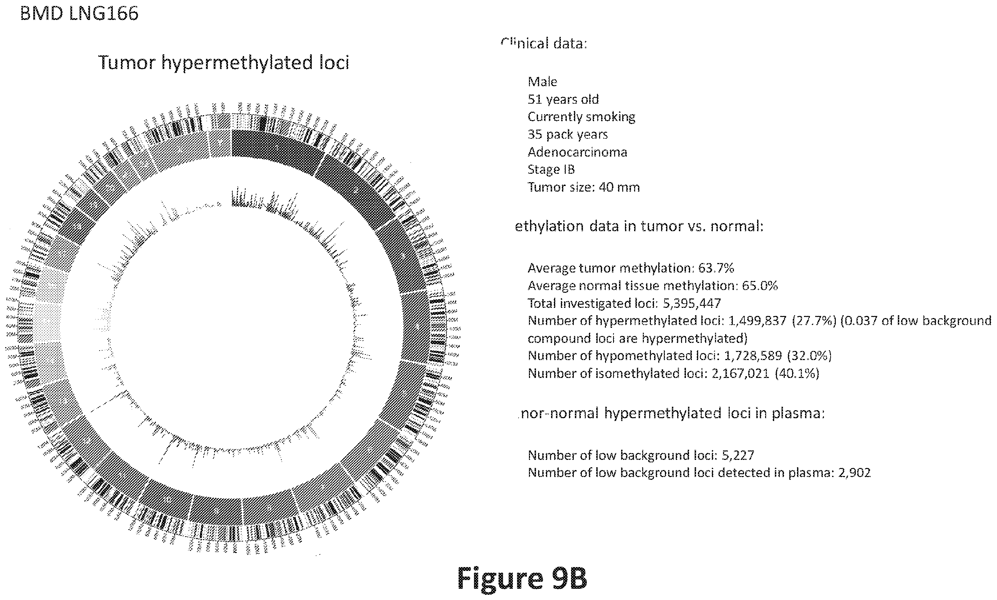

Methylation and mutation analysis was carried on samples from the two lung cancer patients identified as BMD LNG165 and BMD LNG166. The clinical data of each patient are detailed in **Figures 9A-9B.**

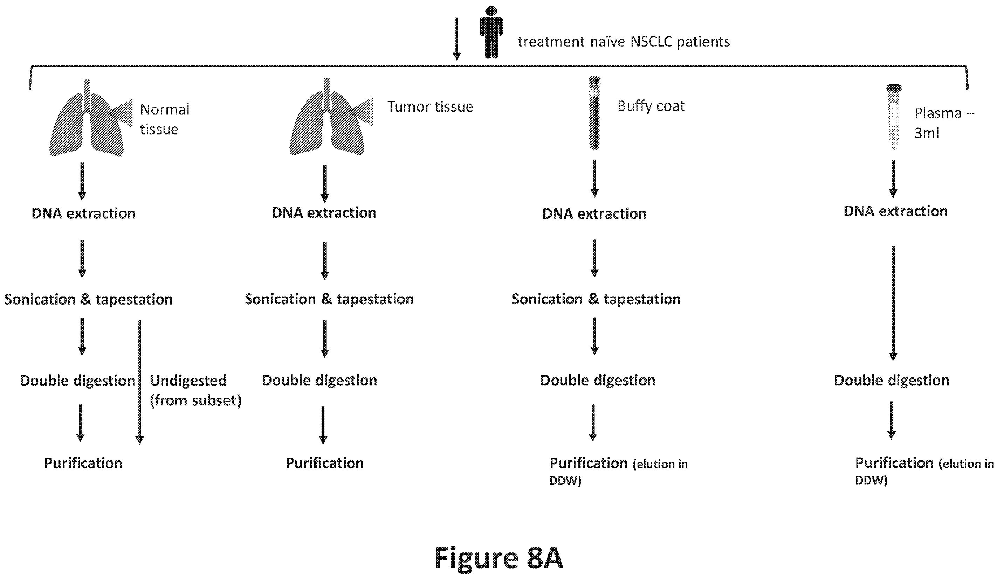

Sample preparation for analysis is set forth in **Figure 8A.** Normal lung tissue sample, tumor lung tissue sample and blood sample were provided for each patient. The blood samples were separated to buffy coat and plasma samples.

DNA was extracted from each sample as indicated in the figure. Normal tissue DNA, tumor tissue DNA and buffy coat DNA were fragmented by sonication. Next, DNA was subjected to digestion by the methylation-sensitive restriction enzymes HinP1I and AciI as described in Example 1 and purified. An aliquot of the normal tissue DNA from each patient was left undigested and kept as a control. The purified DNA samples were subjected to library preparation and sequencing as described above.

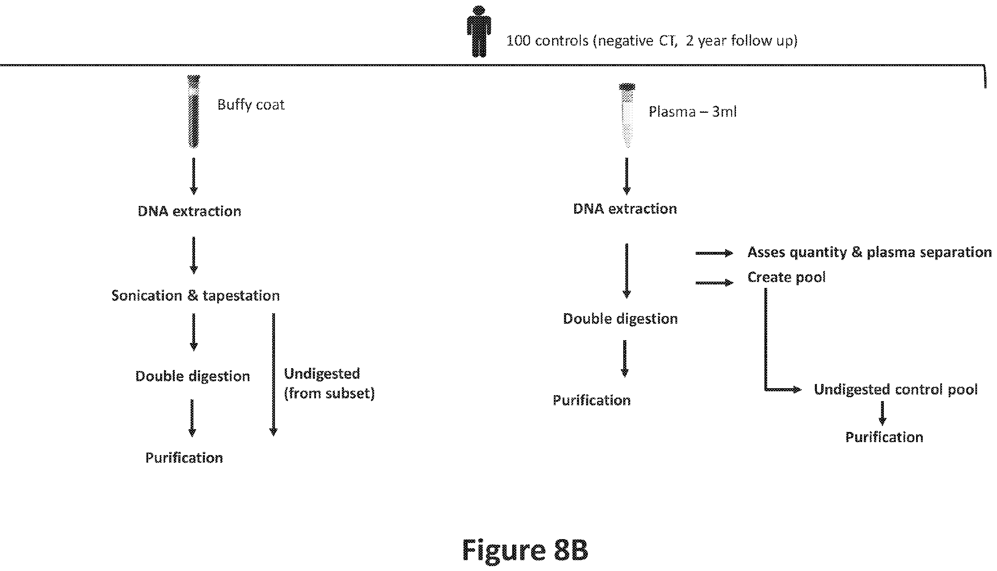

**Figure 8B** shows sample preparation of control samples taken from 100 healthy control subjects. The control samples included a buffy coat sample and a plasma sample from each control subject. DNA was extracted from each sample as indicated in the figure. Buffy coat DNA was fragmented by sonication, subjected to digestion by HinP1I and AciI, and subsequently purified. An aliquot of the buffy coat DNA from each control subject was left undigested and kept as a control. Plasma DNA was subjected to digestion by HinP1I and AciI and purified. An aliquot of the plasma DNA was taken for quality control (e.g., assessing the quality of plasma separation) and for creating an undigested control pool of plasma DNA. The purified DNA samples were subjected to library preparation and sequencing as described above.

Sequence reads from each sample were mapped against the complete human genome (hg18 genomic build). Alignments with CIGAR & MAPQ > 0 & abs(TLEN) ≤ 500 bp were selected for further analysis of methylation and mutation in order to identify methylation changes and mutations in the tumor and their representation in the plasma.

For each genomic position, "hits span 100" was determined, namely, the number of reads that start > 50bp upstream and end > 50 bp downstream of the genomic position.

"Hits span 100" are alignments of at least 100bps, representing DNA molecules of at least 100 bps in length in the DNA sample that remained after the methylation-sensitive digestion and library preparation. The analysis of such alignments is advantageous for evaluating nucleosome positioning in cell-free DNA in addition to methylation, because the copy numbers of such alignments reflect nucleosomal boundaries, wherein a high copy number is typical of the middle of the nucleosome, and a low copy number is typical of the boundaries between nucleosomes.

Also, many cancer-associated methylation changes occur within CG islands, namely, regions of the genome that are reach in CG sites that undergo methylation. "Hits span 100" regions around an analyzed CG site located within a restriction locus of an enzyme used in the assay typically include additional restriction loci of the enzyme, containing additional CG sites. "Hits span 100" alignments therefore represent DNA molecules of at least 100 bps in length, in which an analyzed restriction locus, as well as any additional restriction loci within the DNA molecule, were all methylated in the DNA sample and remained intact following digestion with the enzymes used in the assay. Analyzing alignments which are at least 100 bps in length and containing a plurality of restriction loci which were all methylated in the DNA sample increases the specificity of the cancer-related hypermethylation signal and enables an improved, more accurate detection of differences between normal and cancerous samples. Such methylation analysis is particularly advantageous for CG sites located within CG islands.

"Hits span 100" values were normalized against the median "hits span 100" value in the same sample. For example: normalized "hits span 100" at a specific locus = # of "hits span 100" at that locus / median # of "hits span 100" in the sample. Normalizations were to the median value across chromosomes 1-22.

Whole-genome methylation analysis

Methylation loci were defined as restriction loci with a number of normalized "hits span 100" above a predefined threshold in undigested normal tissue pool.

- Background methylation level of methylation loci was determined as follows:
  normalized "hits span 100" in digested normal plasma pool/ normalized "hits span 100" in undigested normal plasma pool

If normalized "hits span 100" in undigested normal plasma pool = 0, the value 1/median "hits span 100" was used instead.

- Tumor methylation level of methylation loci was determined as follows:
  normalized "hits span 100" in tumor/ normalized "hits span 100" in undigested normal tissue pool
- Normal methylation level of methylation loci was determined as follows:
  normalized "hits span 100" in normal tissue/ normalized "hits span 100" in undigested normal tissue pool
- Plasma methylation level of methylation loci was determined as follows:
  normalized "hits span 100" in plasma/ normalized "hits span 100" in undigested normal plasma pool

For each patient, a set of low background methylation loci was compiled, by selecting methylation loci with background methylation level below a predefined threshold.

In addition, a set of hypermethylated loci was compiled, which show hypermethylation in tumor vs. normal tissue.

The set of hypermethylated loci was compiled by determining tumor-normal differential methylation level of methylation loci (=tumor methylation level - normal methylation level) and selecting methylation loci with tumor-normal differential methylation level that exceeds a predefined threshold.

A set of hypomethylated loci was compiled, which show hypomethylation in tumor vs. normal tissue. The set of hypomethylated loci was compiled by determining tumor-normal differential methylation level of methylation loci (=tumor methylation level - normal methylation level) and selecting methylation loci with tumor-normal differential methylation level below a predefined threshold.

A set of isomethylated loci was compiled, which do not show different methylation levels between tumor and normal tissue. The set of isomethylated loci was compiled by determining tumor-normal differential methylation level of methylation loci (=tumor methylation level - normal methylation level) and selecting methylation loci which are neither tumor-normal hypermethylated nor tumor-normal hypomethylated.

The results of the analysis for each patient are set forth in **Figures 9A-9B.** Millions of hypermethylation and hypomethylation events were detected in the tumors of each patient. In addition, thousands of low-background hypermethylation events were detected in each patient's plasma. The detected events represent putative methylation markers.

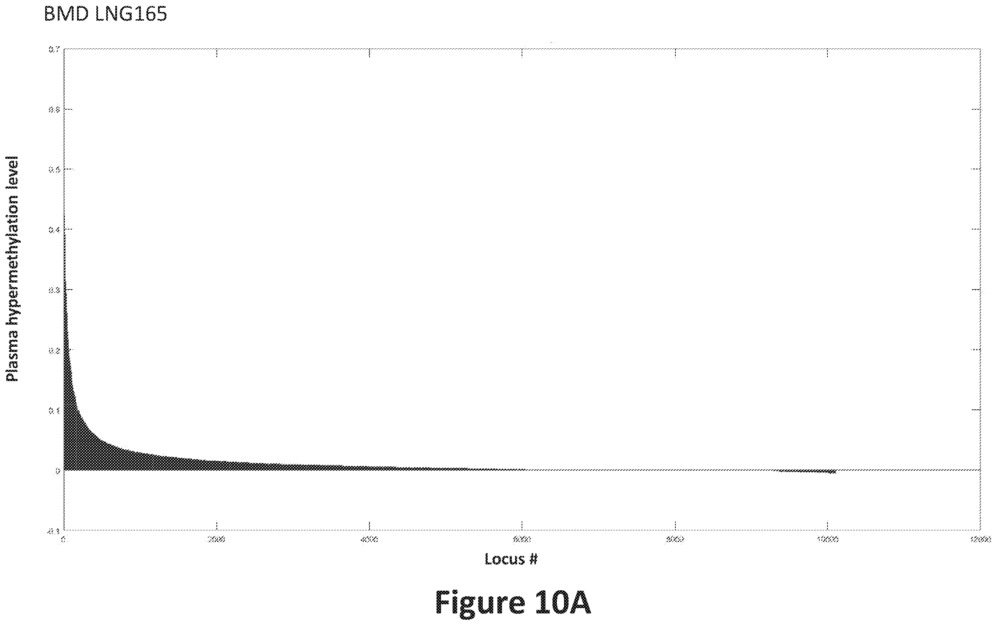

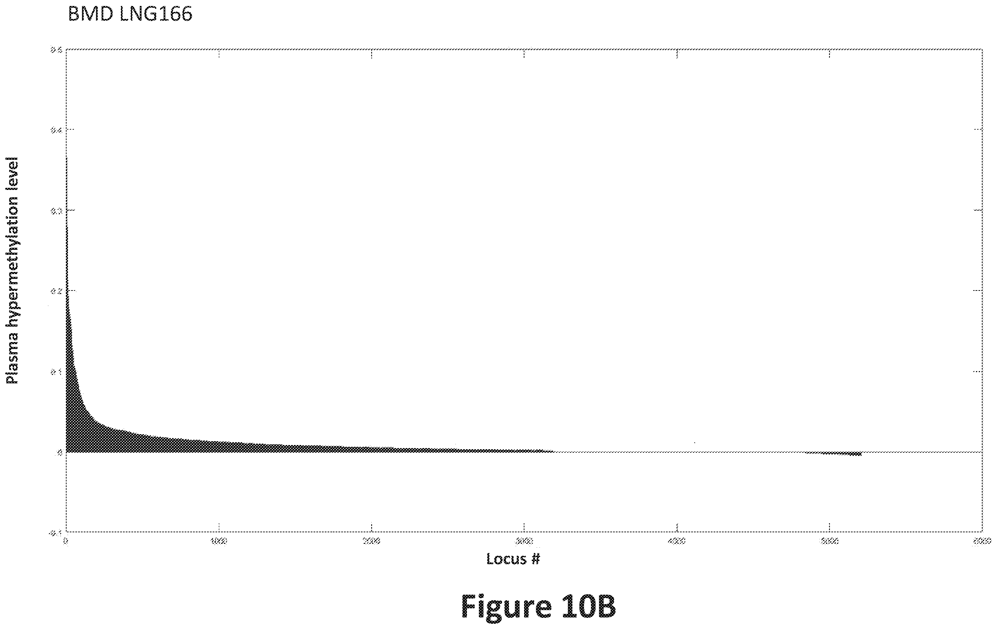

**Figures 10A-10B** show that thousands of methylation loci with a particularly strong hypermethylation signal in the plasma could be identified.

Whole-genome mutation analysis

Tumor mutations were defined as genotypes found in the tumor DNA that are different from the most prevalent genotype in the corresponding normal tissue from the same patient.

The fraction of reads with mutated genotypes in the tumor DNA represented the tumor mutational level, and the fraction of reads with the same mutated genotypes in the plasma of the patient represented the plasma mutation level. For each sample, the average tumor and plasma mutation levels were calculated across all mutations and a tumor mutational burden was calculated (i.e., average plasma mutation level / average tumor mutation level). The tumor mutational burden represents the fraction of tumor DNA in the plasma of the patient.

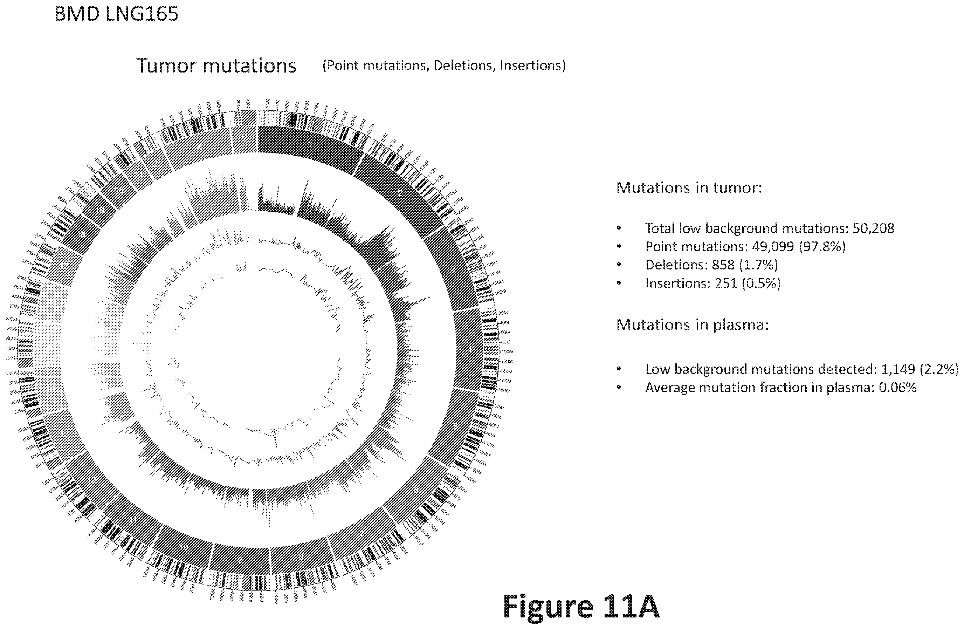

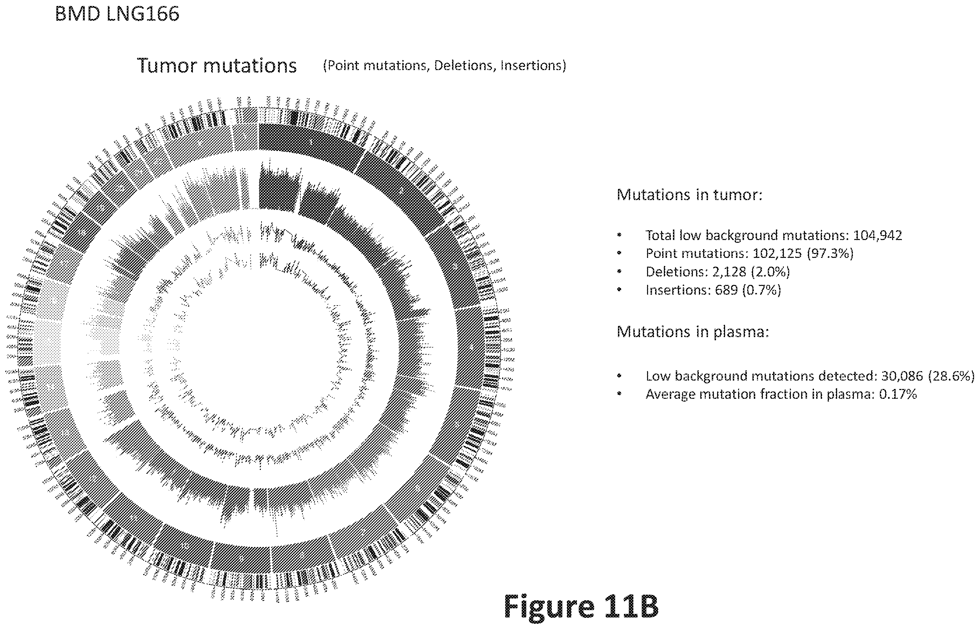

For each patient, a set of low background mutations was compiled, by selecting mutations with plasma mutation background (= fraction of mutation in normal plasma pool) below a predefined threshold. In addition, average mutation fraction in plasma was determined for each patient. The results of the analysis for each patient are set forth in **Figures 11A-11B.**

Multiomic regions

A multiomic region is defined herein as a genomic region with a tumor hypermethylated site (hypermethylated in tumor compared to normal tissue) and a tumor mutation site within a predefined distance. The methods of the present invention aim at detecting cancer-associated genetic and epigenetic changes in cell-free DNA samples. Thus, multiomic regions of up to 150 bps are preferred, in order to identify DNA molecules containing both the tumor hypermethylated site and tumor mutation site on the same molecule (and subsequently on the same sequence read). In the current Example, multiomic regions in which a tumor hypermethylated locus and a tumor mutation within 100 bps of each other were searched in tumor samples of patient BMD LNG165 and patient BMD LNG166.

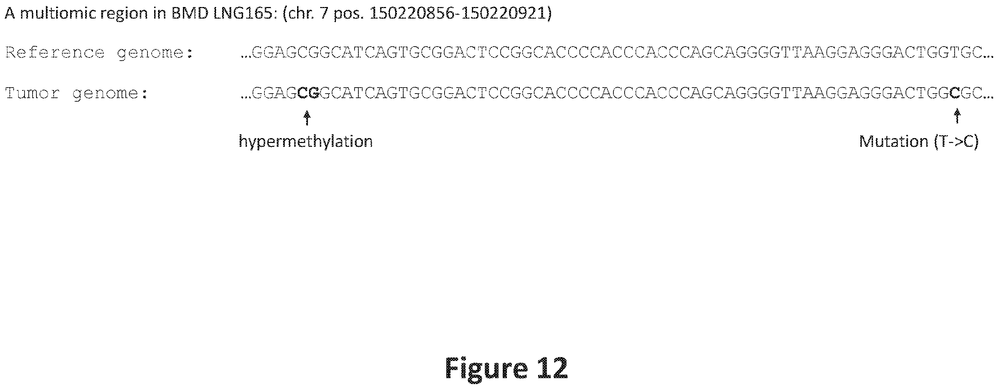

The analysis identified 6,060 multiomic regions in patient BMD LNG165, and 9,471 multiomic regions in patient BMD LNG166. An example of a multiomic region in BMD LNG165 is set forth in **Figure 12** (chr. 7 pos. 150220856-150220921).

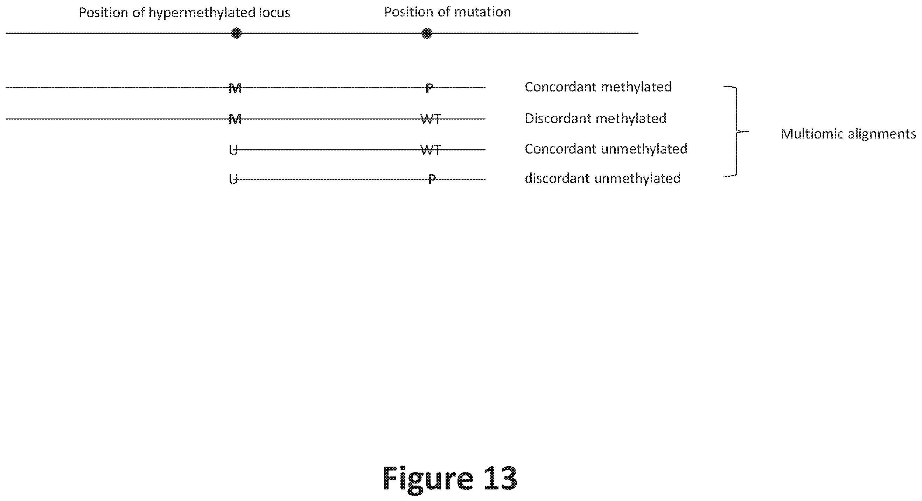

A multiomic alignment was defined as an alignment with CIGAR & MAPQ > 0 & TLEN > 0 & TLEN ≤ 500 bp that spans a multiomic region. Examples of multiomic alignment types are set forth in **Figure 13** and include:
A concordant methylated alignment, in which a cancer phenotype is seen both at the methylation position (the position is methylated) and at the mutation position (the mutant variant is present): the alignment spans all of the hypermethylated restriction site (all letters of the recognition sequence of the restriction enzyme used in the assay are present in the alignment, for example, GCGC for HinP1I,) and contains the mutated genotype in the read.

A discordant methylated alignment, in which a cancer phenotype is seen at the methylation position (the position is methylated) and a normal phenotype is seen at the mutation position (the WT variant is present): the alignment spans all of the hypermethylated restriction site (e.g., all letters of GCGC are present in the alignment) and contains the WT (reference) genotype in the read.

A concordant unmethylated alignment, in which a normal phenotype is seen both at the methylation position (the position is unmethylated) and at the mutation position (the WT variant is present): the alignment starts or ends at the exact cut site (starts at the n position or ends at the n + 1 position of the restriction site) and contains the WT (reference) genotype in the read.

A discordant unmethylated alignment, in which a normal phenotype is seen at the methylation position (the position is unmethylated) and a cancer phenotype is seen at the mutation position (the mutant variant is present): the alignment starts or ends at the exact cut site (starts at the n position or ends at the n + 1 position of the restriction site) and contains the mutated genotype in the read.

The above shows that the methods disclosed herein employing methylation-sensitive digestion followed by next-generation sequencing are sensitive yet accurate enough to work with low amounts of DNA and receive vast amount of information, including methylation data, mutation data and more. The methos are advantageous for both discovery, e.g., of new methylation markers, and for diagnostic applications at the clinics. The methods enable detecting signals which cannot be detected with bisulfite.

## Example 3 - Direct calculation of methylated and unmethylated DNA levels

In the following example calculation of methylation/unmethylation was carried out by digesting cell-free DNA from plasma samples with the methylation-sensitive restriction enzymes HinP1I and AciI, followed by library preparation, next generation sequencing and analysis of sequence reads.

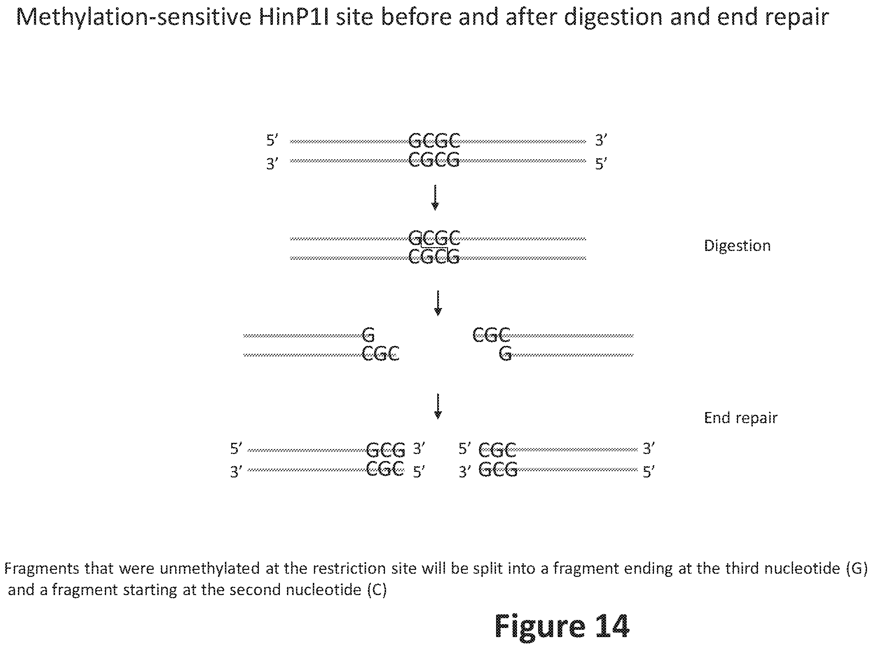

**Figure 14** illustrates the methylation-sensitive HinP1I site before and after digestion and end repair. Cell-free DNA molecules that are unmethylated at the HinP1I site undergo digestion, resulting in double-stranded DNA molecules with non-blunt (sticky) ends corresponding to the HinP1I cut site. Specifically, since HinP1I has a cut site of four bases, the digestion produces a pair of double-stranded DNA molecules, with a two-base 5' overhang in one DNA molecule and a complementary 5' overhang in the other. The non-blunt ends are subjected to end repair (e.g., using NEBNext Ultra DNA Library Prep Kit) to produce blunt-end DNA molecules. Following end repair, two types of DNA fragments are obtained: fragments ending (3' end) at the third nucleotide of the HinP1I recognition sequence (G nucleotide) and fragments starting (5' end) at the second nucleotide of the HinP1I recognition sequence (C nucleotide).

**Figure 15** illustrates differences in DNA fragments obtained following digestion and end repair of cell free DNA molecules spanning a HinP1I restriction site which are either methylated or unmethylated at the cut site. Black dots represent methylation. DNA molecules which are methylated at the cut site remain intact following digestion and the result is DNA fragments spanning the cut site. DNA molecules which are unmethylated at the cut site are digested by the enzyme. Following end repair the result is DNA fragments that start or end at the recognition sequence (specifically, fragments ending at the third nucleotide G and fragments starting at the second nucleotide C of the recognition sequence).

### Experimental procedure

Plasma samples were collected from 56 healthy control subjects. DNA was extracted from the samples using QIAamp® Circulating Nucleic Acid Kit (QIAGEN, Hilden, Germany) and pooled. An aliquot of 450ng was kept as an undigested control DNA and the remaining DNA (800ng) was subjected to digestion: sample was incubated with 10 units of HinP1I and 5 units of AciI for 2h at 37°C followed by inactivation for 20 min at 65°C. Following digestion, a sequencing library was prepared by using NEBNext Ultra DNA Library Prep Kit. The library was subjected to next generation sequencing using Illumina NovaSeq 6000 sequencing platform with S4 flow cell. The sequence reads from the digested and undigested DNA samples were mapped against the complete human genome (hg18 genomic build).

### Calculating levels of methylated DNA

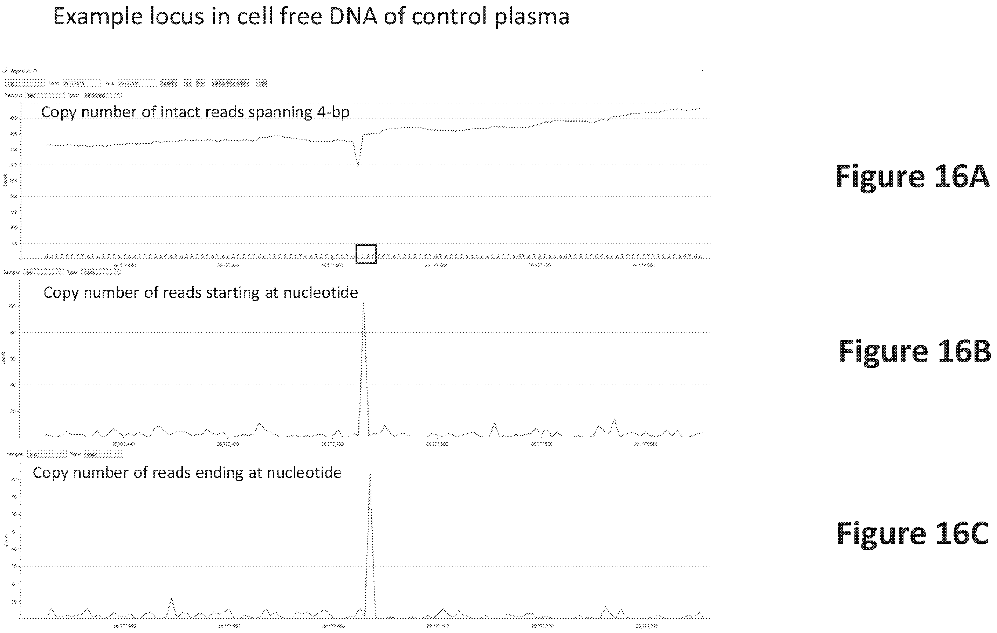

To calculate levels of methylated DNA, sequence reads were plotted as read counts per 4-bp loci. Loci corresponding to cut sites of the restriction enzyme (HinP1I) were analyzed and the number of reads spanning the full intact site was recorded. **Figure 16A** shows exemplary analysis of a 4-bp locus corresponding to a HinP1I site (marked with a rectangle) in the digested DNA sample. As can be seen in the figure, a decrease in read count is observed at this HinP1I site. The decrease indicates digestion by the enzyme, where the read count at this position corresponds to the number of DNA fragments in which the locus remained intact. As noted above, HinP1I is methylation-sensitive and therefore does not cut methylated DNA. Thus, the read count of this restriction locus corresponds to the number of DNA molecules in the DNA sample in which the restriction locus was methylated.

The level of DNA methylated at the restriction locus is calculated as follows:

$$\text{level of methylated DNA} = \frac{\text{actual read count of the restriction locus}}{\text{expected read count of the restriction locus}}$$

where the expected read count of the restriction locus may be calculated from:

(i) read count of a 4-bp reference locus that is not cut by the enzyme;

(ii) average read count of a plurality of 4-bp reference loci that are not cut by the enzyme (optimally consisting of loci that have the same copy number as the restriction locus in the undigested sample); or

(iii) read count of the restriction locus in the undigested control sample (possibly corrected for depth differences).

A reference locus may be a 4-bp stretch located immediately upstream or downstream to the restriction locus, or a 4-bp locus located at a more distant location in the genome.

Correction for depth differences may be carried out as follows:

$$\text{expected read count of the restriction locus =}$$
$$\text{read count of the restriction locus in the undigested sample / (average sequencing depth in the undigested sample / average sequencing depth in digested sample)}$$

$$\text{average sequencing depth =}$$
$$\text{total number of reads*average read length/size of genome}$$

The obtained level of methylated DNA may be multiplied by 100 to obtain percentage (%) of methylated DNA at the tested HinP1I locus in the original DNA sample.

Calculating levels of unmethylated DNA

To calculate levels of unmethylated DNA, sequence reads were plotted as read counts that end at each base across the genome. Alternatively or additionally, sequence reads may be plotted as read counts that start at each base across the genome. Genomic loci corresponding to cut sites of the restriction enzyme (HinP1I) were analyzed.

**Figure 16B** shows "start" analysis for the HinP1I site analyzed above and flanking regions. As can be seen in the figure, a peak is observed at the second nucleotide of the cut site (C nucleotide). The peak height, namely, the number of sequence reads starting at the second nucleotide of the cut site, corresponds to the number of DNA fragments that were cut by the enzyme. As noted above, HinP1I is methylation-sensitive and therefore cuts unmethylated DNA. Thus, the peak height corresponds to the number of DNA molecules in the DNA sample in which the restriction locus was unmethylated.

**Figure 16C** shows "end" analysis for the HinP1I site analyzed above and flanking regions. As can be seen in the figure, a peak is observed at the third nucleotide of the cut site (G nucleotide). The peak height, namely, the number of sequence reads ending at the third nucleotide of the cut site, corresponds to the number of DNA fragments that were cut by the enzyme at this cut site. As noted above, HinP1I is methylation-sensitive and therefore cuts unmethylated DNA. Thus, the peak height corresponds to the number of DNA molecules in the DNA sample in which the restriction locus was unmethylated.

The level of DNA unmethylated at the restriction locus is calculated as follows:

$$\text{level of unmethylated DNA =}$$
$$\text{(actual number of reads starting or ending at the restriction locus – expected number of reads starting or ending at the restriction locus) / expected read count of the restriction locus}$$

The expected number of reads starting or ending at the restriction locus may be calculated from:

(i) number of reads starting or ending at a 4-bp reference locus that is not cut by the enzyme;

(ii) average number of reads starting or ending at a family of 4-bp reference loci that are not cut by the enzyme; or

(iii) number of reads starting or ending at the restriction locus in the undigested control sample (possibly corrected for depth differences).

The expected read count of the restriction locus may be calculated from:

(i) read count of a 4-bp reference locus that is not cut by the enzyme;

(ii) average read count of a family of 4-bp reference loci that are not cut by the enzyme (optimally consisting of loci that have the same copy number as the restriction locus in the undigested sample); or

(iii) read count of the restriction locus in the undigested control sample (possibly corrected for depth differences.

A reference locus may be a 4-bp stretch located immediately upstream or downstream to the restriction locus, or a 4-bp locus located at a more distant location in the genome.

It is noted that each DNA molecule that is cut by the restriction endonuclease as disclosed herein results in two fragments, one that starts at a nucleotide within the restriction locus and another that ends at a nucleotide within the restriction locus. Thus, for a given DNA molecule it may be possible to obtain two different sequence reads. For correct analysis of the number of unmethylated DNA molecules that were present in the sample the calculation of unmethylated DNA level may be carried based on the number of sequence reads that start at the restriction locus, the number of sequence reads that end at the restriction locus or by an average between the two values, but not based on a sum of the values. When using library preparation methods that deplete small fragments it is preferable to calculate unmethylated DNA level using the orientation with the larger number of sequence reads, to avoid bias due to the depletion of small fragments.

The obtained level of unmethylated DNA may be multiplied by 100 to obtain percentage (%) of unmethylated DNA at the tested HinP1I locus in the original DNA sample.

Such methylation/unmethylation analysis is particularly advantageous for CG sites located at genomic regions with a low CG content.

Simultaneous calculation of methylated + unmethylated DNA level

To simultaneously calculate levels of methylated and unmethylated DNA, a "total fragment number" is first calculated, as follows:

$$\text{Total fragment number} =$$
$$\text{read count of the restriction locus} + \text{number of reads starting or ending at the restriction locus} - \text{expected number of reads starting or ending at the restriction locus}$$

The expected number of reads starting or ending at the restriction locus is calculated as described above.

The levels of methylated and unmethylated DNA are calculated using the total fragment number, as follows:

$$\text{level of methylated DNA} = \frac{\text{read count of the restriction locus}}{\text{total fragment number}}$$

$$\text{level of unmethylated DNA} =$$
$$\frac{(\text{number of reads starting or ending at the restriction locus} - \text{expected number of reads starting or ending at the restriction locus})}{\text{total fragment number}}$$

The obtained levels of methylated and unmethylated DNA may be multiplied by 100 to obtain percentages (%) of methylated and unmethylated DNA at the tested HinP1I locus in the original DNA sample.

**Example 4** - **Analysis of restriction loci using methylated and unmethylated DNA levels**

Levels of methylated and unmethylated DNA were calculated for eight CG dinucleotides located within restriction loci of HinP1I, identified as CG# 1-8 (**Table 4**), in the pooled plasma DNA sample described in Example 3. As detailed in Example 3, the pooled DNA sample was digested with the methylation-sensitive restriction enzymes HinP1I and AciI, followed by library preparation, next generation sequencing and alignment of sequence reads against the complete human genome.

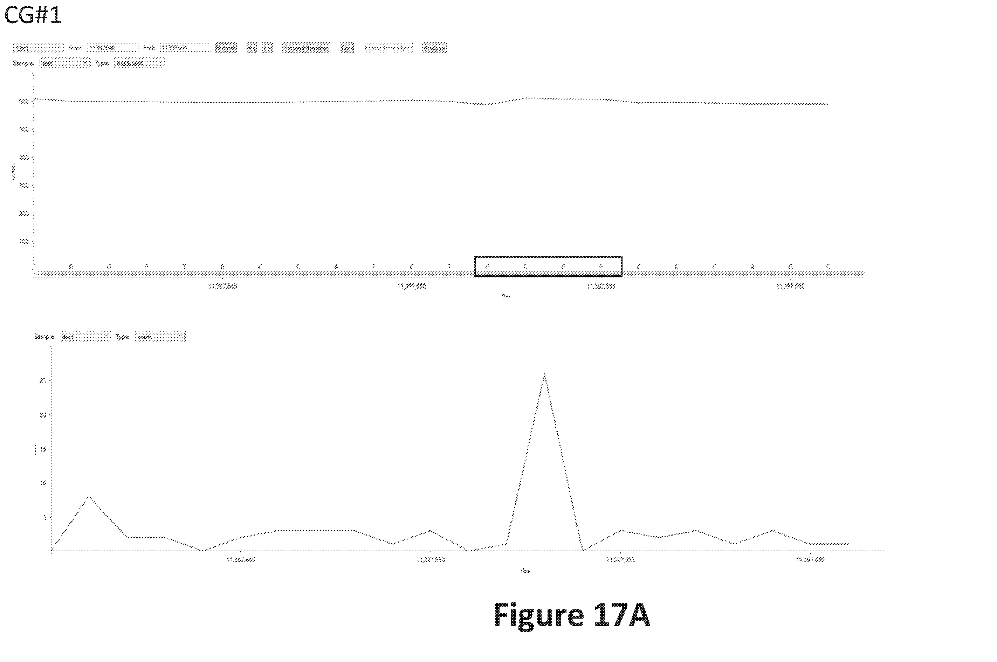

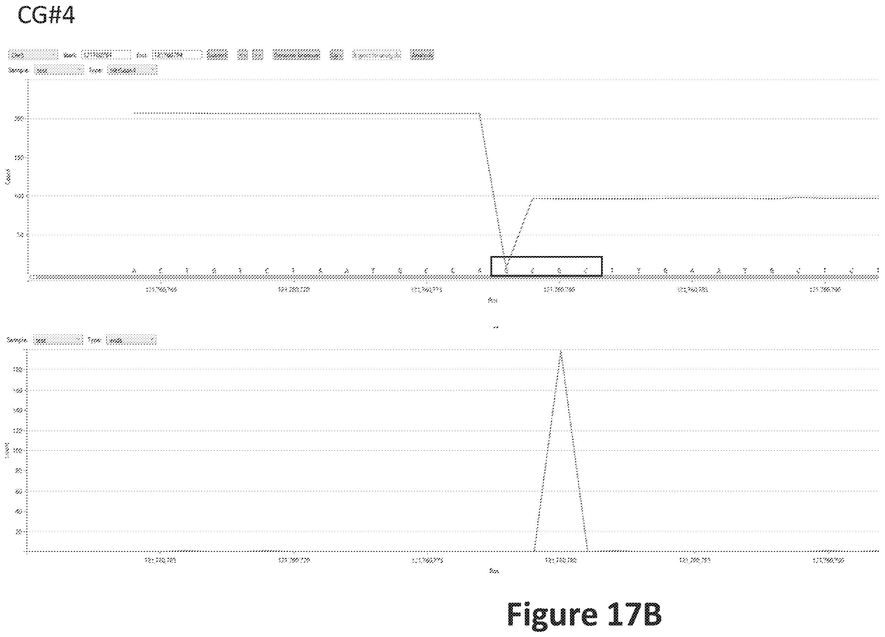

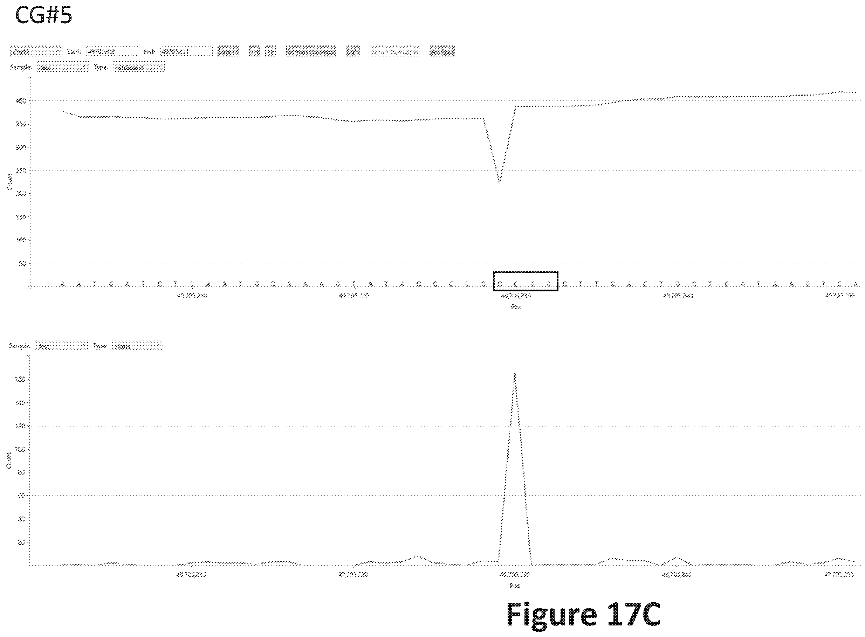

Exemplary raw data for CG#1 (highly methylated), CG#4 (highly unmethylated) and CG#5 are shown in **Figures 17A-17C.** The upper panel of each figure shows read counts per 4-bp loci, for determining a read count of the restriction locus. The restriction loci are indicated by rectangles. The bottom panel of each figure shows read counts that start or end at each base in the reference genome, for determining a read count of sequence reads starting or ending at the restriction locus. The presentation of "ends" or "starts" is according to the orientation that provided the larger number of reads.

The level of methylated DNA at each restriction locus was calculated by dividing the read count of the restriction locus by an expected read count (read count of a control locus), and multiplying by 100 to obtain percentage of methylated DNA at the restriction locus.

The level of unmethylated DNA at each restriction locus was calculated by subtracting an expected number of reads starting or ending at the restriction locus, and subsequently dividing by the expected read count of the restriction locus and multiplying by 100 to obtain percentage of unmethylated DNA at the restriction locus. For each restriction locus, the number of reads starting at the restriction locus and the number of reads ending at the restriction locus were determined, and further calculations were carried out based on the larger number of reads.

A discrepancy level (%) was calculated for each restriction locus by determining the difference between the sum of methylated and unmethylated percentages calculated in this example, and an expected sum of 100%:

$$\% \text{ discrepancy} = (\% \text{ methylated} + \% \text{ unmethylated}) - 100$$

The results are summarized in **Table 4.** The restriction loci are listed in Table 4 according to the level of discrepancy in an ascending order. The level of discrepancy may be used in evaluating and selecting potential DNA methylation markers, where loci with lower levels of discrepancy may be preferred. The level of discrepancy may also be used as an indicator of proper sample processing and analysis for already-identified DNA methylation markers, where a low level of discrepancy is indicative of proper sample processing and analysis.

Table 4 - methylated and unmethylated DNA levels

| CG# | Chromosome | Location | % methylated | % unmethylated | % discrepancy |
|-----|-----------|----------|--------------|----------------|---------------|
| 1 | 1 | 11397653 | 97.99 | 2.01 | 0 |
| 2 | 17 | 17362652 | 99.76 | 0.24 | 0 |
| 3 | 17 | 71690026 | 5.10 | 94.89 | -0.01 |
| 4 | 3 | 121760779 | 3.39 | 96.60 | -0.01 |
| 5 | 12 | 49705230 | 57.36 | 42.57 | -0.07 |
| 6 | 1 | 8120128 | 99.33 | 1.57 | +0.9 |
| 7 | 2 | 39309230 | 7.66 | 93.62 | +1.28 |
| 8 | 12 | 84283776 | 97.03 | 1.64 | -1.33 |

The results demonstrate that the direct determination of unmethylation in addition to methylation using the same sequencing data provides complementary methylation information of genomic regions, enabling improved methylation profiling, a more accurate and valid assessment of potential DNA methylation markers, and subsequently a more accurate analysis of methylation differences between sample.

**Example 5 - Methylation profiling at lung cancer DNA methylation markers and diagnosis of lung cancer**

The methylation profile of a DNA sample extracted from a plasma sample is determined at six genomic regions containing restriction loci of HinP1I differentially methylated between lung cancer DNA and normal non-lung cancer DNA. The genomic regions, previously disclosed in WO 2019/142193, assigned to the Applicant of the present invention, are identified as SEQ ID NOs: 1-6 and detailed in **Table 5.**

Table 5 - lung cancer-associated genomic regions

| SEQ ID NO. | Nucleic acid sequence | Description* |
|------------|----------------------|--------------|
| 1 | AGTAGCGCCCACTGAGCGGTTTTTC AGTTGCTGCACCGTTCTTAGCGCCC AACGGAACGTTTCCCGTACGCGGAG TCCATAAGTT | Position 43030476 on Chromosome 5, intergenic region |
| 2 | CGGTCCCGCA GCGCCCGCCA CACACCCGCG CCAGAGGTCC AGCGCATGTG CAGTGAAATG GCCTAGCCC | Position 176712760 on Chromosome 2, intergenic region |

(continued)

| SEQ ID NO. | Nucleic acid sequence | Description* |
|---|---|---|
| 3 | CGGATAGCGC GGCGGGCGAC AGCCCCCCGG ATAACCCCGC CGAGGGAGGG GCGCTTGTAA AACCGAGCGG CG | Position 44151837 on Chromosome 17, intergenic region |
| 4 | TCCTCCTTGC CTTCTTTCGC CGAAAGGGGG CGCGCTCCTC CCAGGCTGCG CTGGTACCTA | Position 168907269 on Chromosome 1, *PRRX1* gene |
| 5 | AGGACCCGCT CCGCAAAGCG CCCACCCTCG AGGGAGGAAA GCCGAGCTGC GCCTCCGCGC AAGGCCAGGG AGTGTGGC | Position 158629293 on Chromosome 7, *VIPR2* gene |
| 6 | AGGCCGCGAG CGCGGCGCGA TCAGTAGCGC CCACTAACAG TTCGTTCTGC ACGGCGGAGC GCGAGACCGC GGA | Position 154860262 on Chromosome 7, intergenic region |
| * Start position. The description refers to position on hg18 genomic build | | |

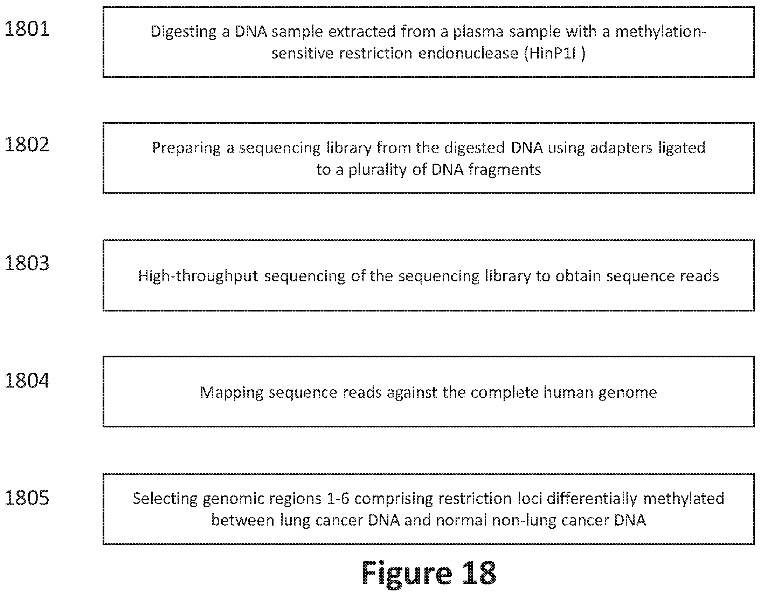

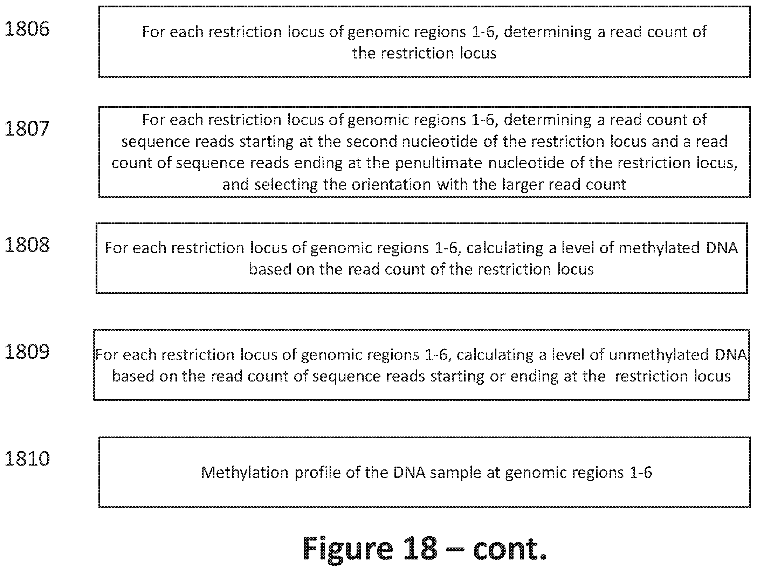

**Figure 18** is a flowchart describing an exemplary method for profiling methylation of the DNA sample according to embodiments of the present invention. The exemplary method comprises the following steps:

1801- digesting the DNA sample with the methylation-sensitive restriction endonuclease HinP1I;
1802- preparing a sequencing library from the digested DNA using adapters ligated to a plurality of DNA fragments;
1803- high-throughput sequencing of the sequencing library to obtain sequence reads;
1804- mapping sequence reads against the complete human genome;
1805- selecting genomic regions 1-6 comprising restriction loci differentially methylated between lung cancer DNA and normal non-lung cancer DNA;
1806- for each restriction locus of genomic regions 1-6, determining a read count of the restriction locus;
1807- for each restriction locus of genomic regions 1-6, determining a read count of sequence reads starting at the second nucleotide of the restriction locus and a read count of sequence reads ending at the penultimate nucleotide of the restriction locus, and selecting the orientation with the larger read count;
1808- for each restriction locus of genomic regions 1-6, calculating a level of methylated DNA based on the read count of the restriction locus;
1809- for each restriction locus of genomic regions 1-6, calculating a level of unmethylated DNA based on the read count of sequence reads starting or ending at a nucleotide within the restriction locus;

thereby obtaining a methylation profile of the DNA sample at genomic regions 1-6 (step 1910).

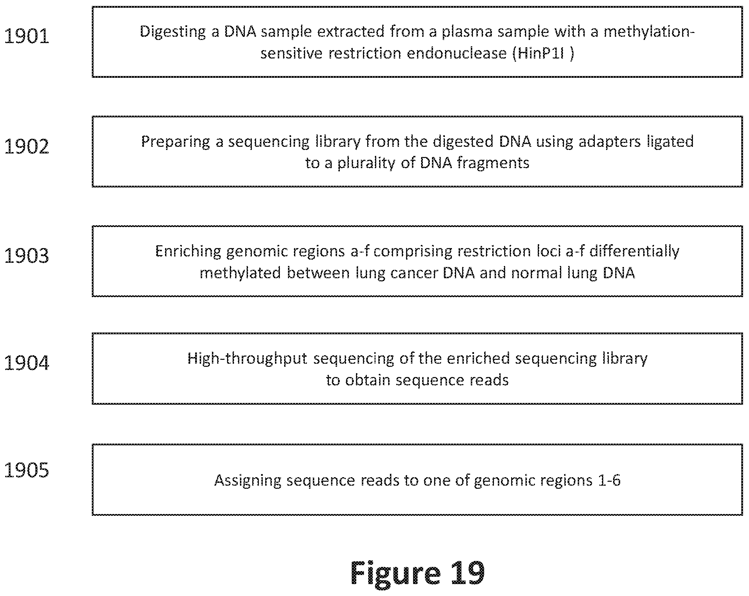

**Figure 19** is a flowchart describing an additional exemplary method for profiling methylation of the DNA sample according to embodiments of the present invention. The exemplary method comprises the following steps:

1901- digesting the DNA sample with the methylation-sensitive restriction endonuclease HinP1I;
1902- preparing a sequencing library from the digested DNA using adapters ligated to a plurality of DNA fragments;
1903- enriching genomic regions 1-6 comprising restriction loci differentially methylated between lung cancer DNA and normal non-lung cancer DNA;
1904- high-throughput sequencing of the enriched sequencing library to obtain sequence reads;
1905- assigning sequence reads to one of genomic regions 1-6;

proceeding according to steps 1906-1910 described above in order to obtain a methylation profile of the DNA sample at genomic regions 1-6.

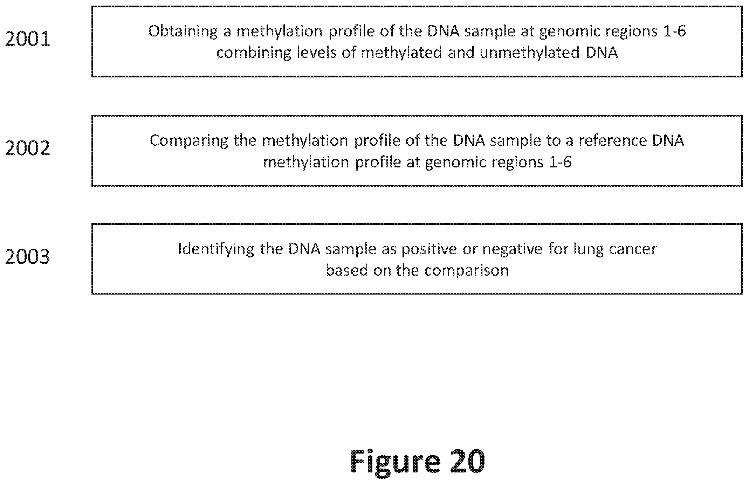

**Figure 20** is a flowchart describing an exemplary method for determining whether the DNA sample is positive or negative for lung cancer according to embodiments of the present invention. The exemplary method comprises the following steps:

2001- obtaining a methylation profile of the DNA sample at genomic regions 1-6 combining levels of methylated and unmethylated DNA, as described above;

2002- comparing the methylation profile of the DNA sample to at least one reference DNA methylation profile at genomic regions 1-6 (e.g., a lung cancer reference profile and/or a healthy non-lung cancer methylation profile); and

2003- identifying the DNA sample as positive or negative for lung cancer based on the comparison.

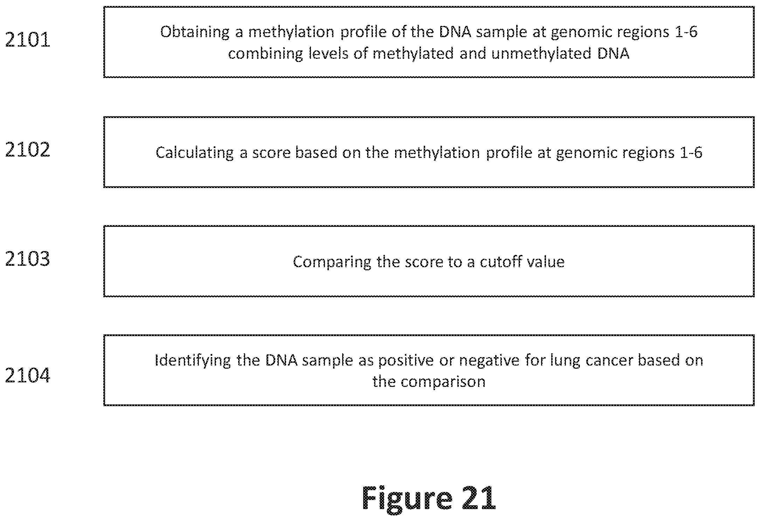

**Figure 21** is a flowchart describing an additional exemplary method for determining whether the DNA sample is positive or negative for lung cancer according to embodiments of the present invention. The exemplary method comprises the following steps:

2101- obtaining a methylation profile of the DNA sample at genomic regions 1-6 combining levels of methylated and unmethylated DNA, as described above;

2102- calculating a score based on the methylation profile at genomic regions 1-6;

2103- comparing the score to a cutoff value; and

2104- identifying the DNA sample as positive or negative for lung cancer based on the comparison.

The foregoing description of the specific embodiments will so fully reveal the general nature of the invention that others can, by applying current knowledge, readily modify and/or adapt for various applications such specific embodiments without undue experimentation and without departing from the generic concept, and therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed chemical structures and functions may take a variety of alternative forms without departing from the invention.

**EMBODIMENTS**

1. A method for profiling genetic and epigenetic characteristics of a cell-free DNA (cfDNA) sample from a subject, the method comprising:

(a) subjecting the cell-free DNA sample to digestion with at least one methylation-sensitive restriction endonuclease, to obtain restriction endonuclease-treated DNA in which methylated sites are intact and unmethylated sites are cut;

(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules;

(c) sequencing the sequencing library by a high-throughput sequencing method to provide sequencing data; and

(d) determining from the sequencing data a methylation value for at least one restriction locus and optionally at least one additional genetic or epigenetic characteristic of the cell-free DNA sample selected from DNA mutation, copy number variation and nucleosome positioning,

wherein an amount of cell-free DNA comprising 3000 haploid equivalents is sufficient for the method, wherein the cell-free DNA sample is not subjected to amplification prior to library preparation, and wherein determining the methylation value and the at least one additional genetic or epigenetic characteristic of the cell-free DNA sample is carried out based on the same sequencing data.

**2.** A method for processing a cell-free DNA sample to obtain sequencing data for genetic and epigenetic analysis, the method comprising:

(a) subjecting the cell-free DNA sample to digestion with at least one methylation-sensitive restriction endonuclease, to obtain restriction endonuclease-treated DNA in which methylated sites are intact and unmethylated sites are cut;

(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules; and

(c) sequencing the sequencing library by a high-throughput sequencing method to obtain sequencing data,

wherein an amount of cell-free DNA comprising 3000 haploid equivalents is sufficient to achieve at least one of: unique mapping rate of at least 85%, a copy number integrity characterized by Pearson correlation of at least 0.65 compared to undigested sample and nucleosome positioning integrity characterized by Pearson correlation of at least 0.55 compared to undigested sample,

and wherein genetic and epigenetic analysis is performed based on the same sequencing data.

3. The method of embodiment 1 or embodiment 2, wherein an amount of cell-free DNA comprising 6,000 haploid equivalents is sufficient for the method.

4. The method of any one of the preceding embodiments, wherein the cell-free DNA is plasma cell-free DNA, and wherein the amount of the cell-free DNA is an amount obtained from 9-10 ml of blood.

5. The method of any one of the preceding embodiments, wherein the amount of cell-free DNA is between 10-200 ng.

6. The method of any one of the preceding embodiments, wherein the amount of cell-free DNA is between 20-100 ng.

7. The method of any one of the preceding embodiments, wherein the at least one methylation-sensitive restriction endonuclease produces non-blunt ends, and the method further comprises subjecting the restriction endonuclease-treated DNA to end repair prior to the ligation of sequencing adapters, to obtain DNA molecules with blunt ends.

8. The method of any one of the preceding embodiments, wherein the high-throughput sequencing is whole genome high-throughput sequencing.

9. The method of any one of embodiments 1-8, wherein the high-throughput sequencing is target-specific high-throughput sequencing.

10. The method of any one of the preceding embodiments, wherein determining a methylation value for at least one restriction locus comprises:

(i) selecting at least one restriction locus and determining the number of sequence reads covering a predefined genomic region of at least 50 bps in length that contains said restriction locus; and
(ii) calculating a methylation value for the at least one restriction locus based on the read count determined in step (i) and a reference read count.

11. The method of embodiment 10, wherein step (i) comprises determining the number of sequence reads covering a predefined genomic region of at least 100 bps in length that contains said restriction locus.

12. The method of any one of the preceding embodiments, wherein the at least one restriction locus is a plurality of restriction loci.

13. The method of any one of the preceding embodiments, wherein the at least one methylation-sensitive restriction endonuclease is a plurality of methylation-sensitive restriction endonucleases, and wherein the digestion with the plurality of methylation-sensitive restriction endonucleases is a simultaneous digestion.

14. The method of embodiment 13, wherein the plurality of methylation-sensitive restriction endonucleases comprises HinP1I.

15. The method of embodiment 13, wherein the plurality of methylation-sensitive restriction endonucleases comprises AciI.

16. The method of embodiment 13, wherein the digestion is carried out using HinP1I and AciI.

17. The method of any one of the preceding embodiments, wherein the step of subjecting the cell-free DNA sample to digestion with at least one methylation-sensitive restriction endonuclease further comprises determining digestion efficacy, and proceeding to preparing a sequencing library if the digestion efficacy is above a predefined threshold.

18. A method for detecting cancer-related genetic and epigenetic changes in a cell-free DNA sample (cfDNA) from a subject, the method comprising: profiling methylation and optionally at least one additional genetic and epigenetic characteristics of the cfDNA sample according to any one of embodiments 1-17, to obtain a genetic and epigenetic profile of the cfDNA sample; and comparing the genetic and epigenetic profile of the cfDNA sample to one or more reference genetic and epigenetic profile selected from a cancer profile and a non-cancer profile, to detect cancer-associated genetic and epigenetic changes in the cfDNA sample.

19. The method of embodiment 18, wherein the cell-free DNA sample is from a subject suspected of having cancer or at risk of having cancer, and the method further comprises administering to the subject active cancer surveillance and follow-up testing when cancer-associated changes are detected, the cancer surveillance and follow-up testing comprises one or more of blood tests, urine tests, cytology, imaging, endoscopy and biopsy.

20. A method for assessing the presence or absence of cancer in a subject, the method comprising:

(a) subjecting a cell-free DNA (cfDNA) sample of the subject to digestion with at least one methylation-sensitive restriction endonuclease, to obtain restriction endonuclease-treated DNA in which methylated sites are intact and unmethylated sites are cut;
(b) sequencing the restriction endonuclease-treated DNA by a high-throughput sequencing method;
(c) selecting at least one multiomic genomic region, comprising a tumor hypermethylated restriction locus and a tumor mutation locus within 150 bps of each other; and
(d) determining the likelihood that the subject has cancer based on analysis of sequence reads covering the at least one multiomic region.

21. The method of embodiment 20, wherein the at least one multiomic region comprises a tumor hypermethylated restriction locus and a tumor mutation locus within 100 bps of each other.

22. The method of embodiment 20 or embodiment 21, wherein analysis of sequence reads covering the at least one multiomic region comprises:

- determining for each multiomic region at least one of:

(i) the number of methylated-mutated sequence reads covering said multiomic region, which include all nucleotides of the restriction locus and present a mutated genotype at the mutation locus;
(ii) the number of methylated-wild type sequence reads covering said multiomic region, which include all nucleotides of the restriction locus and present a wild type genotype at the mutation locus;
(iii) the number of unmethylated-mutated sequence reads covering said multiomic region, which start or end at a nucleotide within the restriction locus and present a mutated genotype at the mutation locus; and
(iv) the number of unmethylated-wild type sequence reads covering said multiomic region, which start or end at a nucleotide within the restriction locus and present a wild type genotype at the mutation locus;
and

- comparing the number of reads determined in (i)-(iv) to reference values of cancer patients and/or healthy individuals in order to assess the likelihood that the subject has cancer.

23. A method for characterizing a cell-free DNA (cfDNA) sample of a subject suspected of having cancer or at risk of having cancer, the method comprising

(a) subjecting the cell-free DNA sample to digestion with at least one methylation-sensitive restriction endonuclease, to obtain restriction endonuclease-treated DNA in which methylated sites are intact and unmethylated sites are cut;
(b) sequencing the restriction endonuclease-treated DNA by a high-throughput sequencing method;
(c) selecting at least one multiomic genomic region, comprising a tumor hypermethylated restriction locus and a tumor mutation locus within 150 bps of each other; and
(d) determining for each multiomic region at least one of:

(i) the number of methylated-mutated sequence reads covering said multiomic region, which include all nucleotides of the restriction locus and present a mutated genotype at the mutation locus;
(ii) the number of methylated-wild type sequence reads covering said multiomic region, which include all nucleotides of the restriction locus and present a wild type genotype at the mutation locus;
(iii) the number of unmethylated-mutated sequence reads covering said multiomic region, which start or end at a nucleotide within the restriction locus and present a mutated genotype at the mutation locus; and
(iv) the number of unmethylated-wild type sequence reads covering said multiomic region, which start or end at a nucleotide within the restriction locus and present a wild type genotype at the mutation locus,

thereby characterizing the cell-free DNA sample.

24. A method for profiling methylation of a DNA sample from a subject, the method comprising:

(a) subjecting the DNA sample to digestion with at least one methylation-sensitive restriction endonuclease, to obtain restriction endonuclease-treated DNA in which methylated sites are intact and unmethylated sites are cut;
(b) preparing a sequencing library from the restriction endonuclease-treated DNA, wherein preparing the sequencing library comprises ligating sequencing adapters to DNA fragments in the restriction endonuclease-treated, wherein each adapter is capable of ligation to both the digested and undigested DNA molecules;
(c) sequencing the sequencing library by a high-throughput sequencing method to obtain sequence reads;
(d) selecting at least one restriction locus and determining the number of sequence reads covering a predefined genomic region of at least 50 bps in length that contains said restriction locus; and
(e) calculating a methylation value for the at least one restriction locus based on the read count determined in step (d) and a reference read count,

thereby profiling methylation of the cell-free DNA sample.

25. The method of embodiment 24, wherein the predefined region covering the restriction locus starts at least 25 bps upstream of the cut site within the restriction locus and ends at least 25 bps downstream of the cut site within the restriction locus.

26. The method of embodiment 25, wherein step (d) comprises determining the number of sequence reads covering a predefined genomic region of at least 100 bps in length that contains said restriction locus.

27. The method of embodiment 26, wherein the predefined region covering the restriction locus starts at least 50 bps upstream of the cut site within the restriction locus and ends at least 50 bps downstream of the cut site within the restriction locus.

28. The method of any one of embodiments 24-27, wherein the at least one restriction locus is located within a CG-island.

29. The method of any one of embodiments 24-28, wherein the reference read count is a read count determined for the predefined genomic region of at least 50 bps in length that contains the restriction locus in an undigested control DNA sample, optionally corrected for sequencing depth differences.

30. The method of any one of embodiments 24-28, wherein the reference read count is a read count determined using a reference region of at least 50 bps in length containing **a** reference locus that is not cut by the restriction endonuclease.

31. The method of any one of embodiments 24-28, wherein the reference read count is an average read count determined using a plurality of reference regions of at least 50 bps in length containing reference loci that are not cut by the restriction endonuclease.

32. The method of any one of embodiments 24-31, wherein calculating a methylation value comprises normalizing the read count determined in step (d) against a median read count of the DNA sample, to obtain a normalized read count, and calculating a ratio of the normalized read count to a normalized reference read count.

33. A method for genetic and epigenetic profiling of a DNA sample, the method comprising determining a methylation value for at least one restriction locus according to any one of embodiments 24-32, and further determining from the sequencing data at least one additional genetic or epigenetic characteristic of the DNA sample selected from DNA mutation, copy number variation and nucleosome positioning.

**34.** The method of any one of the embodiments 24-33, wherein the DNA is cell-free DNA extracted from a biological fluid sample.

**35.** The method of any one of embodiments 24-33, wherein the DNA is DNA extracted from a tumor sample.

**36.** A method for identifying genomic regions differentially methylated between a first and second source of DNA, the method comprising:

profiling methylation of at least one DNA sample from the first source according to the method of any one of embodiments 24-33, to obtain a first DNA methylation profile;
profiling methylation of at least one DNA sample from the second source according to the method of any one of embodiments 24-33, to obtain a second DNA methylation profile; and
comparing the first and second DNA methylation profiles to identify genomic regions differentially methylated between the first and second sources of DNA.

**37.** The method of embodiment 36, wherein the first source of DNA is a cancer DNA and the second source of DNA is a non-cancer DNA.

**38.** The method of embodiment 36, wherein the first source of DNA is plasma cell-free DNA of a cancer patient and the second source of DNA is plasma cell-free DNA of one or more healthy individuals.

**39.** The method of embodiment 36, wherein the first and second sources of DNA are different stages of a cancer.

**Claims**

1. A method for profiling genetic and epigenetic characteristics of a cell-free DNA (cfDNA) sample from a subject, the method comprising:

(a) subjecting the cell-free DNA sample to digestion with at least one methylation sensitive restriction endonuclease, to obtain restriction endonuclease-treated DNA in which methylated sites are intact and unmethylated sites are cut;
(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules;
(c) sequencing the sequencing library by a high-throughput sequencing method to provide sequencing data; and
(d) determining from the sequencing data a methylation value for at least one restriction locus and optionally at least one additional genetic or epigenetic characteristic of the cell-free DNA sample selected from DNA mutation, copy number variation and nucleosome positioning,

wherein an amount of cell-free DNA comprising 3000 haploid equivalents is sufficient for the method, wherein the cell-free DNA sample is not subjected to amplification prior to library preparation, and wherein determining the methylation value and the at least one additional genetic or epigenetic characteristic of the cell-free DNA sample is carried out based on the same sequencing data.

2. A method for processing a cell-free DNA sample to obtain sequencing data for genetic and epigenetic analysis, the method comprising:

(a) subjecting the cell-free DNA sample to digestion with at least one methylation sensitive restriction endonuclease, to obtain restriction endonuclease-treated DNA in which methylated sites are intact and unmethylated sites are cut;
(b) preparing a sequencing library from the restriction endonuclease-treated DNA while preserving the sequence information at the ends of the DNA molecules, wherein preparing the sequencing library comprises ligating sequencing adapters to DNA molecules in the restriction endonuclease-treated DNA, wherein each adapter is capable of ligation to both the digested and undigested DNA molecules; and

(c) sequencing the sequencing library by a high-throughput sequencing method to obtain sequencing data,

wherein an amount of cell-free DNA comprising 3000 haploid equivalents is sufficient to achieve at least one of: unique mapping rate of at least 85%, a copy number integrity **characterized by** Pearson correlation of at least 0.65 compared to undigested sample and nucleosome positioning integrity **characterized by** Pearson correlation of at least 0.55 compared to undigested sample, and wherein genetic and epigenetic analysis is performed based on the same sequencing data.

3. The method of claim 1 or claim 2, wherein:

i) an amount of cell-free DNA comprising 6,000 haploid equivalents is sufficient for the method; and/or
ii) the cell-free DNA is plasma cell-free DNA, and wherein the amount of the cell-free DNA is an amount obtained from 9-10 ml of blood; and/or
iii) the amount of cell-free DNA is between 10-200 ng; and/or
iv) the amount of cell-free DNA is between 20-100 ng.

4. The method of any one of claims 1-3, wherein:

i) the at least one methylation sensitive restriction endonuclease produces non-blunt ends, and the method further comprises subjecting the restriction endonuclease-treated DNA to end repair prior to the ligation of sequencing adapters, to obtain DNA molecules with blunt ends; and/or
ii) the high-throughput sequencing is:

a) whole genome high-throughput sequencing; and/or
b) target-specific high-throughput sequencing.

5. The method of any one of claims 1-4, wherein determining a methylation value for at least one restriction locus comprises:

(i) selecting at least one restriction locus and determining the number of sequence reads covering a predefined genomic region of at least 50 bps in length that contains said restriction locus; and
(ii) calculating a methylation value for the at least one restriction locus based on the read count determined in step (i) and a reference read count;

optionally wherein step (i) comprises determining the number of sequence reads covering a predefined genomic region of at least 100 bps in length that contains said restriction locus.

6. The method of any one of claims 1-5, wherein:

i) the at least one restriction locus is a plurality of restriction loci; and/or
ii) the at least one methylation sensitive restriction endonuclease is a plurality of methylation-sensitive restriction endonucleases, and wherein the digestion with the plurality of methylation-sensitive restriction endonucleases is a simultaneous digestion; optionally wherein:

a) the plurality of methylation-sensitive restriction endonucleases comprises HinP1I; or
b) the plurality of methylation-sensitive restriction endonucleases comprises AciI; or
c) the digestion is carried out using HinP1I and AciI.

7. The method of any one of claims 1-6, wherein the step of subjecting the cell-free DNA sample to digestion with at least one methylation-sensitive restriction endonuclease further comprises determining digestion efficacy, and proceeding to preparing a sequencing library if the digestion efficacy is above a predefined threshold.

8. A method for detecting cancer-related genetic and epigenetic changes in a cell-free DNA sample (cfDNA) from a subject, the method comprising: profiling methylation and optionally at least one additional genetic and epigenetic characteristics of the cfDNA sample according to any one of claims 1-7, to obtain a genetic and epigenetic profile of the cfDNA sample; and comparing the genetic and epigenetic profile of the cfDNA sample to one or more reference genetic and epigenetic profile selected from a cancer profile and a non-cancer profile, to detect cancer-associated genetic and epigenetic changes in the cfDNA sample.

9. A method for characterizing a cell-free DNA (cfDNA) sample of a subject suspected of having cancer or at risk of having cancer, the method comprising

(a) subjecting the cell-free DNA sample to digestion with at least one methylation sensitive restriction endonuclease, to obtain restriction endonuclease-treated DNA in which methylated sites are intact and unmethylated sites are cut;
(b) sequencing the restriction endonuclease-treated DNA by a high-throughput sequencing method;
(c) selecting at least one multiomic genomic region, comprising a tumor hypermethylated restriction locus and a tumor mutation locus within 150 bps of each other; and
(d) determining for each multiomic region at least one of:

(i) the number of methylated-mutated sequence reads covering said multiomic region, which include all nucleotides of the restriction locus and present a mutated genotype at the mutation locus;
(ii) the number of methylated-wild type sequence reads covering said multiomic region, which include all nucleotides of the restriction locus and present a wild type genotype at the mutation locus;
(iii) the number of unmethylated-mutated sequence reads covenng said multiomic region, which start or end at a nucleotide within the restriction locus and present a mutated genotype at the mutation locus; and
(iv) the number of unmethylated-wild type sequence reads covering said multiomic region, which start or end at a nucleotide within the restriction locus and present a wild type genotype at the mutation locus, thereby characterizing the cell-free DNA sample.

10. A method for profiling methylation of a DNA sample from a subject, the method comprising:

(a) subjecting the DNA sample to digestion with at least one methylation-sensitive restriction endonuclease, to obtain restriction endonuclease-treated DNA in which methylated sites are intact and unmethylated sites are cut;
(b) preparing a sequencing library from the restriction endonuclease-treated DNA, wherein preparing the sequencing library comprises ligating sequencing adapters to DNA fragments in the restriction endonuclease-treated, wherein each adapter is capable of ligation to both the digested and undigested DNA molecules;
(c) sequencing the sequencing library by a high-throughput sequencing method to obtain sequence reads;
(d) selecting at least one restriction locus and determining the number of sequence reads covering a predefined genomic region of at least 50 bps in length that contains said restriction locus; and
(e) calculating a methylation value for the at least one restriction locus based on the read count determined in step (d) and a reference read count, thereby profiling methylation of the cell-free DNA sample; optionally
wherein the predefined region covering the restriction locus starts at least 25 bps upstream of the cut site within the restriction locus and ends at least 25 bps downstream of the cut site within the restriction locus; further optionally
wherein step (d) comprises determining the number of sequence reads covering a predefined genomic region of at least 100 bps in length that contains said restriction locus; yet further optionally
wherein the predefined region covering the restriction locus starts at least 50 bps upstream of the cut site within the restriction locus and ends at least 50 bps downstream of the cut site within the restriction locus.

11. The method of claim 10, wherein:

i) the at least one restriction locus is located within a CG-island;
and/or
ii) a) the reference read count is a read count determined for the predefined genomic region of at least 50 bps in length that contains the restriction locus in an undigested control DNA sample, optionally corrected for sequencing depth differences; or

b) the reference read count is a read count determined using a reference region of at least 50 bps in length containing a reference locus that is not cut by the restriction endonuclease; or
c) the reference read count is an average read count determined using a plurality of reference regions of at least 50 bps in length containing reference loci that are not cut by the restriction endonuclease.

12. The method of claim 10 or claim 11, wherein calculating a methylation value comprises normalizing the read count determined in step (d) against a median read count of the DNA sample, to obtain a normalized read count, and calculating a ratio of the normalized read count to a normalized reference read count.

13. A method for genetic and epigenetic profiling of a DNA sample, the method comprising determining a methylation value for at least one restriction locus according to any one of claims 10-12, and further determining from the sequencing data at least one additional genetic or epigenetic characteristic of the DNA sample selected from DNA mutation, copy number variation and nucleosome positioning.

14. The method of any one of the claims 10-13, wherein:

      i) the DNA is cell-free DNA extracted from a biological fluid sample; or
      ii) wherein the DNA is DNA extracted from a tumor sample.

15. A method for identifying genomic regions differentially methylated between a first and second source of DNA, the method comprising: profiling methylation of at least one DNA sample from the first source according to the method of any one of claims 10-13, to obtain a first DNA methylation profile; profiling methylation of at least one DNA sample from the second source according to the method of any one of claims 10-13, to obtain a second DNA methylation profile; and comparing the first and second DNA methylation profiles to identify genomic regions differentially methylated between the first and second sources of DNA; optionally wherein:

      i) the first source of DNA is a cancer DNA and the
      second source of DNA is a non-cancer DNA; or
      ii) the first source of DNA is plasma cell-free DNA of a cancer patient and the second source of DNA is plasma cell-free DNA of one or more healthy individuals; or
      iii) the first and second sources of DNA are different stages of a cancer.

Figure 1A

Figure 1B

Figure 2A

Figure 2B

Figure 3A

51

BMD LNG166 -
Correlation of hits in methylation-sensitive digested samples

Pearson correlation = 0.693

Hits in undigested control

Hits in methylation-sensitive digested samples

BMD LNG166 -
Correlation of hits in bisulfite converted samples

Pearson correlation = 0.161

Hits in undigested control

Hits in bisulfite converted samples

Figure 3B

EP 4 775 687 A2

BMD LNG165 -
Correlation of "hits span 100" in methylation-sensitive digested samples

Pearson correlation = 0.647

"hits span 100" in undigested control

"hits span 100" in methylation-sensitive digested samples

BMD LNG165 -
Correlation of "hits span 100" in bisulfite converted samples

Pearson correlation = 0.19

"hits span 100" in undigested control

"hits span 100" in bisulfite converted samples

**Figure 4A**

Figure 4B

BMD LNG165

Figure 5A

Figure 5B

Figure 6

Figure 7A

Figure 7B

treatment naïve NSCLC patients

Normal tissue

Tumor tissue

Buffy coat

Plasma – 3ml

DNA extraction

DNA extraction

DNA extraction

DNA extraction

Sonication & tapestation

Sonication & tapestation

Sonication & tapestation

Double digestion | Undigested (from subset)

Double digestion

Double digestion

Double digestion

Purification

Purification

Purification (elution in DDW)

Purification (elution in DDW)

Figure 8A

EP 4 775 687 A2

Figure 8B

# BMD LNG165

## Tumor hypermethylated loci

**Clinical data:**

* Male
* 48 years old
* Currently smoking
* 26 pack years
* Adenocarcinoma
* Stage IA
* Tumor size: 17x20 mm

**Methylation data in tumor vs. normal:**

* Average tumor methylation: 66.9%
* Average normal tissue methylation: 71.9%
* Total investigated loci: 5,395,447
* Number of hypermethylated loci: 1,587,356 (29.4% (0.070 of low background compound loci are hypermethylated)
* Number of hypomethylated loci: 2,017,241 (37.3%)
* Number of isomethylated loci: 1,790,850 (33.1%)

**:or-normal hypermethylated loci in plasma:**

* Number of low background loci: 10,122
* Number of low background loci detected in plasma: 5,544

# Figure 9A

EP 4 775 687 A2

BMD LNG166

## Tumor hypermethylated loci

EP 4 775 687 A2

Clinical data:

Male
51 years old
Currently smoking
35 pack years
Adenocarcinoma
Stage IB
Tumor size: 40 mm

ethylation data in tumor vs. normal:

Average tumor methylation: 63.7%
Average normal tissue methylation: 65.0%
Total investigated loci: 5,395,447
Number of hypermethylated loci: 1,499,837 (27.7%) (0.037 of low background compound loci are hypermethylated)
Number of hypomethylated loci: 1,728,589 (32.0%)
Number of isomethylated loci: 2,167,021 (40.1%)

.nor-normal hypermethylated loci in plasma:

Number of low background loci: 5,227
Number of low background loci detected in plasma: 2,902

## Figure 9B

EP 4 775 687 A2

BMD LNG165

Figure 10A

BMD LNG166

Plasma hypermethylation level

Locus #

Figure 10B

EP 4 775 687 A2

# BMD LNG165

## Tumor mutations (Point mutations, Deletions, Insertions)

Mutations in tumor:

- Total low background mutations: 50,208
- Point mutations: 49,099 (97.8%)
- Deletions: 858 (1.7%)
- Insertions: 251 (0.5%)

Mutations in plasma:

- Low background mutations detected: 1,149 (2.2%)
- Average mutation fraction in plasma: 0.06%

## Figure 11A

EP 4 775 687 A2

BMD LNG166

Tumor mutations   (Point mutations, Deletions, Insertions)

Mutations in tumor:

- Total low background mutations: 104,942
- Point mutations: 102,125 (97.3%)
- Deletions: 2,128 (2.0%)
- Insertions: 689 (0.7%)

Mutations in plasma:

- Low background mutations detected: 30,086 (28.6%)
- Average mutation fraction in plasma: 0.17%

**Figure 11B**

EP 4 775 687 A2

A multiomic region in BMD LNG165: (chr. 7 pos. 150220856-150220921)

Reference genome:     ...GGAGCGGCATCAGTGCGGACTCCGGCACCCCACCCACCCAGCAGGGGTTAAGGAGGGACTGGTGC...

Tumor genome:         ...GGAG**CG**GCATCAGTGCGGACTCCGGCACCCCACCCACCCAGCAGGGGTTAAGGAGGGACTGG**C**GC...
                                ↑                                                                    ↑
                          hypermethylation                                             Mutation (T->C)

EP 4 775 687 A2

# Figure 12

Position of hypermethylated locus     Position of mutation

M ─────────────── P       Concordant methylated
M ─────────────── WT      Discordant methylated
U ─────────────── WT      Concordant unmethylated     } Multiomic alignments
U ─────────────── P       discordant unmethylated

# Figure 13

EP 4 775 687 A2

# Methylation-sensitive HinP1I site before and after digestion and end repair

Fragments that were unmethylated at the restriction site will be split into a fragment ending at the third nucleotide (G) and a fragment starting at the second nucleotide (C)

## Figure 14

# Cell free DNA molecules spanning a HinP1I restriction site

Molecule 1 ⎯⎯⎯⎯⎯⎯⎯⎯⎯⎯⎯ GĊGC ⎯⎯
Molecule 2 ⎯⎯⎯⎯⎯⎯⎯⎯⎯⎯ GĊGC ⎯⎯⎯⎯⎯⎯
Molecule 3 ⎯⎯⎯⎯⎯⎯⎯⎯⎯⎯ GCGC ⎯⎯⎯⎯⎯
Molecule 4 ⎯⎯⎯⎯ GCGC ⎯⎯⎯⎯⎯⎯⎯⎯⎯
Molecule 5 ⎯⎯⎯⎯ GCGC ⎯⎯⎯⎯⎯⎯⎯

Before digestion –
5 molecules spanning the full site; 2/5 are methylated

Digestion and library prep (including end repair)

Molecule 1 ⎯⎯⎯⎯⎯⎯⎯⎯⎯ GĊGC ⎯⎯
Molecule 2 ⎯⎯⎯⎯⎯⎯⎯⎯ GĊGC ⎯⎯⎯⎯⎯⎯

Molecule 3L ⎯⎯⎯⎯⎯⎯⎯⎯⎯⎯⎯ GCG    CGC⎯⎯ Molecule 3R

Molecule 4L ⎯⎯GCG    CGC⎯⎯⎯⎯⎯⎯⎯⎯ Molecule 4R

Molecule 5L ⎯⎯⎯⎯⎯⎯ GCG    CGC⎯⎯⎯⎯⎯ Molecule 5R

Fragments spanning intact site

# Figure 15

Fragments starting/ending at restriction site

EP 4 775 687 A2

# Example locus in cell free DNA of control plasma

Copy number of intact reads spanning 4-bp

Figure 16A

Copy number of reads starting at nucleotide

Figure 16B

Copy number of reads ending at nucleotide

Figure 16C

EP 4 775 687 A2

CG#1

Figure 17A

Figure 17B

Figure 17C

1801 Digesting a DNA sample extracted from a plasma sample with a methylation-sensitive restriction endonuclease (HinP1I )

1802 Preparing a sequencing library from the digested DNA using adapters ligated to a plurality of DNA fragments

1803 High-throughput sequencing of the sequencing library to obtain sequence reads

1804 Mapping sequence reads against the complete human genome

1805 Selecting genomic regions 1-6 comprising restriction loci differentially methylated between lung cancer DNA and normal non-lung cancer DNA

## Figure 18

EP 4 775 687 A2

1806

For each restriction locus of genomic regions 1-6, determining a read count of the restriction locus

1807

For each restriction locus of genomic regions 1-6, determining a read count of sequence reads starting at the second nucleotide of the restriction locus and a read count of sequence reads ending at the penultimate nucleotide of the restriction locus, and selecting the orientation with the larger read count

1808

For each restriction locus of genomic regions 1-6, calculating a level of methylated DNA based on the read count of the restriction locus

1809

For each restriction locus of genomic regions 1-6, calculating a level of unmethylated DNA based on the read count of sequence reads starting or ending at the restriction locus

1810

Methylation profile of the DNA sample at genomic regions 1-6

# Figure 18 – cont.

| 1901 | Digesting a DNA sample extracted from a plasma sample with a methylation-sensitive restriction endonuclease (HinP1I ) |
|---|---|
| 1902 | Preparing a sequencing library from the digested DNA using adapters ligated to a plurality of DNA fragments |
| 1903 | Enriching genomic regions a-f comprising restriction loci a-f differentially methylated between lung cancer DNA and normal lung DNA |
| 1904 | High-throughput sequencing of the enriched sequencing library to obtain sequence reads |
| 1905 | Assigning sequence reads to one of genomic regions 1-6 |

# Figure 19

EP 4 775 687 A2

2001 — Obtaining a methylation profile of the DNA sample at genomic regions 1-6 combining levels of methylated and unmethylated DNA

2002 — Comparing the methylation profile of the DNA sample to a reference DNA methylation profile at genomic regions 1-6

2003 — Identifying the DNA sample as positive or negative for lung cancer based on the comparison

**Figure 20**

| 2101 | Obtaining a methylation profile of the DNA sample at genomic regions 1-6 combining levels of methylated and unmethylated DNA |
|---|---|

| 2102 | Calculating a score based on the methylation profile at genomic regions 1-6 |
|---|---|

| 2103 | Comparing the score to a cutoff value |
|---|---|

| 2104 | Identifying the DNA sample as positive or negative for lung cancer based on the comparison |
|---|---|

# Figure 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10392666 B
- WO 2016061624 A
- WO 2018195211 A
- WO 2011070441 A
- WO 2017006317 A
- WO 2019142193 A
- WO 2020188561 A

**Non-patent literature cited in the description**

- **BALL et al.** *Nat Biotechnol*, 2009, vol. 27 (4), 361-368
- **BRUNNER et al.** *Genome Res.*, 2009, vol. 19 (6), 1044-1056
- **JELINEK et al.** *Epigenetics*, 2012, vol. 7 (12), 1368-1378
- **MARSH ; PASQUALONE.** *Front Physiol*, 2014, vol. 5, 173
- **MARSH et al.** *Front Genet*, 2016, vol. 7, 191
- **VISWANATHAN et al.** *Nucleic Acids Research*, 2019, vol. 47 (19), e122
- **PEREIRA et al.** *PLoS ONE*, 2020, vol. 15 (6), e0233800
- **TANAKA et al.** *Analytical Biochemistry*, 2020, vol. 609, 113977